# EUROPEAN PATENT APPLICATION

(11) **EP 4 095 128 A1**
(43) Date of publication of application: **30.11.2022**
(21) Application number: 21305676.5
(22) Date of filing: 25.05.2021
(51) Int. Cl.: C07D 215/42, C07D 215/48, C07D 401/04, C07D 401/12, C07D 401/14, C07F 7/08, C07F 11/00, A61K 31/47, A61P 35/00

(54) **TETRAHYDROQUINOLINE (THQ) COUMPOUNDS**

(71) Applicant: Centre national de la recherche scientifique, 75016 Paris (FR); Commissariat à l'énergie atomique et aux énergies alternatives, 75015 Paris (FR); Centre Hospitalier Universitaire Grenoble Alpes, 38700 La Tronche (FR); Institut national de la santé et de la recherche médicale (INSERM), 75013 Paris (FR); Université Grenoble Alpes, 38400 Saint-Martin-d'Hères (FR)
(72) Inventor: MACEK JILKOVA, Zuzana, 38000 GRENOBLE (FR); GOVIN, Jérôme, 38320 EYBENS (FR); EMADALI, Anouk, 38000 GRENOBLE (FR); PETOSA, Carlo, 38000 GRENOBLE (FR); WONG, Yung-Sing, 38400 SAINT MARTIN D'HERES (FR)
(74) Representative: Lavoix

(57) **Abstract**

The present invention relates to new tetrahydroquinoline (THQ) compounds and new methods for synthetizing such compounds.

The THQs are inhibitors of BET proteins (Bromodomain and Extra Terminal Domain) such as BRD4 and are useful in therapeutic treatment of cancer or inflammation.

The present invention relates in particular to compound having the following structure : 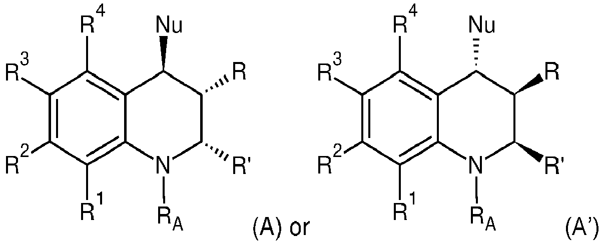 wherein R, R', R_{A}, Nu, R1, R2, R3, and R4 are each independently an atom or a chemical group of atoms

## Description

The present invention relates to new tetrahydroquinoline (THQ) compounds and new methods for synthetizing such compounds.

The present invention relates to the applications thereof, in particular in therapeutic treatment.

### Technological background

THQ synthesis is usually performed using the multicomponent Povarov reaction in cycloaddition [4+2] or sequential Mannich/Friedel-Craft reactions.

In addition, the implementation of chiral catalysts such as Jacobsen's urea, phosphoric acid described by Zhu and Masson and, more recently, the silylated prolinol/POCl₃/oxidation sequential reactions reported by Chan have been effective in obtaining 2,3,4-substituted THQs covering different configurations enantioselectively.

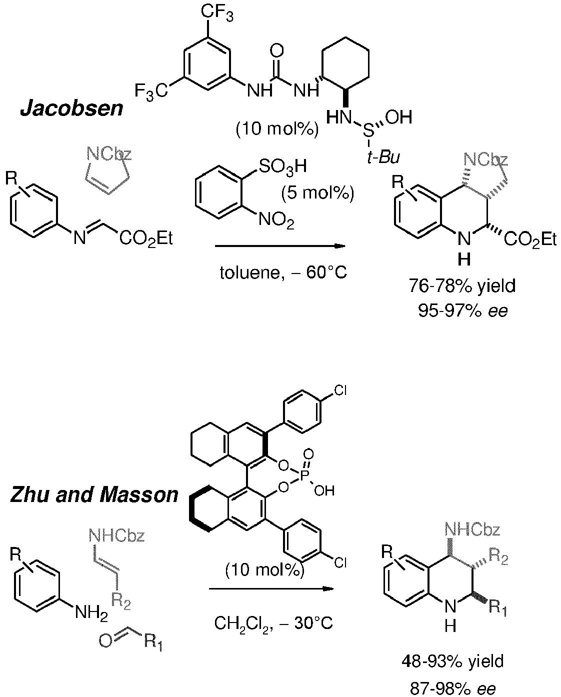

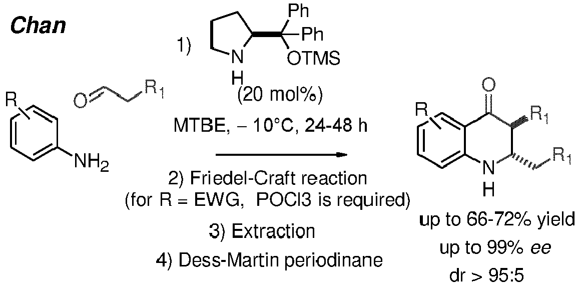

However, access to the cis-2,3 and trans-3,4 configuration of enantioselectively 2,3,4-substituted THQs has not yet been obtained.

RVX208 (*quinazoline* structure below) is the first molecule having a BD2 selectivity of BRD4 described in the litterature and has been able to suppress the inflammatory response specifically for interleukin-6 (IL-6).

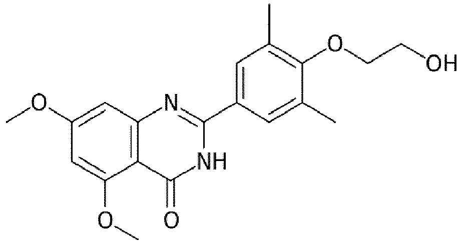

However, concerning THQ derivatives, PCT applications WO 201638120 A1 and WO 2014140076 A1 to Glaxo Smith Klime (GSK) describe a class of THQ being BET inhibitors.

However, THQ derivatives described by GSK are 2,3,4 substituted in configurations *trans-2,3* and *trans-3,4,* because of the limitation of the existing synthesis routes which are not able to enantioselectively provide 2,3,4-THQ in the *cis*-2,3 and *trans-*3,4 configuration.

### Goals of the invention

The present invention aims to solve the technical problem of providing a THQ substituted at least in 2,3,4 positions in another diastereomeric configuration and enantiomerically purethan the *trans-*2,3 and *trans-*3,4 configuration.

The present invention aims to solve the technical problem of providing a THQ substituted at least in 2,3,4 positions in the *cis*-2,3 and *trans-*3,4 configuration.

The present invention aims to solve the technical problem of providing a process for preparing such THQ derivatives.

The present invention aims to solve the technical problem of providing a process preparing an enantiomeric mixture wherein one enantiomer of the THQ substituted at least in 2,3,4 positions in the *cis*-2,3 and *trans-*3,4 configuration predominates, and preferably, one enantiomer predominates to the extent of about 90% or greater, and preferably about 98% or greater (% in mol/mol of the enantiomer to the mixture of enantiomers).

Preferably, the present invention aims to solve the technical problem of providing a process easily implemented.

Preferably, the present invention aims to solve the technical problem of providing a process which could be implemented at the industrial scale.

### Description of the invention

The present invention provides on one hand an innovative method of preparation of compounds according to the present invention, having a specific 2,3,4-THQ in the *cis*-2,3 and *trans-3,4* configuration.

The present invention relates to compounds having the following structure : 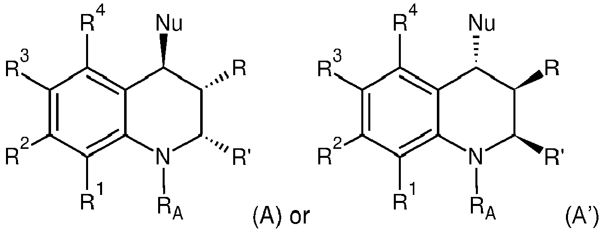 wherein R, R', R_{A}, Nu, R1, R2, R3, and R4 are each independently an atom or a chemical group of atoms.

In the present invention, the term compound relates to the compounds according to the recited structure or a salt thereof.

Advantageously, the compound of the invention is an enantiomer substantially free of other enantiomers or an enantiomeric mixture wherein one enantiomer of the compound according to the invention predominates. One enantiomer predominates to the extent of about 90% or greater, and preferably about 98% or greater (% in mol/mol of the enantiomer to the mixture of enantiomers).

In particular, the method according to the present invention allows the very specific configuration of the 2,3,4-THQ in the *cis*-2,3 and *trans-*3,4 configuration. In the prior art, such configuration is not obtainable in particular selectively and in enantiomerically pure form, and even less in a one pot reaction.

Also, the method according to the present invention provides in a one pot reaction a 2,3,4-THQ in the *trans-2,3* and *trans-3,4* configuration. In the prior art, such configuration is not obtainable in a one pot reaction. Accordingly the present invention is very advantageous over the prior art.

*The cis-2,3* andtrans-3,4 configuration of THQs is found to be essential for inhibitory activity on bromodomains and anti-inflammatory cellular effects. When the molecules are of *trans-2,3* and *trans-3,4* configuration, no activity is observed on the inhibition of bromodomains (table XX), nor on cellular anti-inflammatory assays.

The present invention relates to compounds having the following structure: 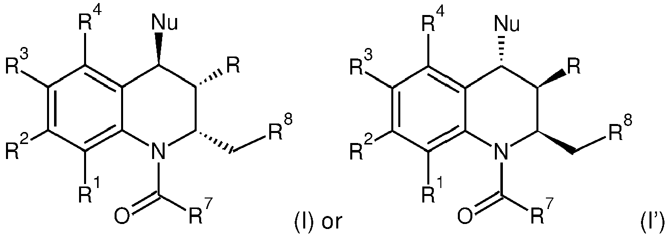 or 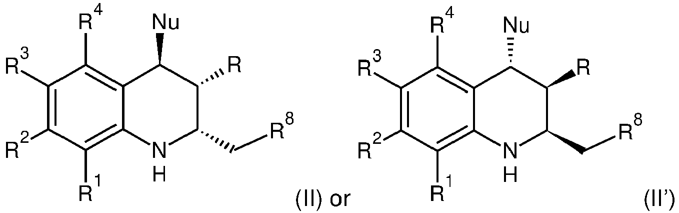
wherein R represents an alkyl group,
wherein R1, R2, R3, R4, R7 and R8 are each independently an atom or a chemical group of atoms,
wherein -Nu represents a group of atoms, preferably selected from the group consisting of -N₃, -OR₅, -SR₅, -SeR₅ and 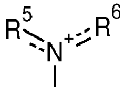 wherein -N is linked to the heterocycle and wherein the covalent bonds between N and R5, and N and R6 represent single or double bonds with R5 or R6, and wherein R5 and R6 represent each independently an atom or a chemical group of atoms and may form together a cycle or heterocycle, optionally substituted, or -Nu together with R form an heterocyle, for example thereby forming a hexahydropyrroloquinoline.

In particular, the compounds of the invention are enantiomers of structure A, A', I, I' II or II'.

Also, the present invention provides on the other hand compounds of structure A, A', I, I' II or II' according to the invention and structures falling within these definitions, because such compounds are good candidates for therapeutic treatments, for example for treating a disease involving an inflammatory disorder or a cancer. This is why the present invention focuses on these specific compounds.

In one preferred embodiment, R represents an alkyl group, typically a C1-C6 alkyl, for example a methyl, ethyl or propyl. In an embodiment, R is CH₃ (methyl).

In one preferred embodiment, R1, R2 and R4 are hydrogen atoms.

In one preferred embodiment, R' represents an alkyl group or-(CH₂)-R8 or -C(O)Rc, wherein Rc is a group of atoms, typically an alkyl, typically a C1-C6 alkyl, for example a methyl, ethyl or propyl, wherein R8 is as defined in the present invention.

In an embodiment R8 is a group of atoms, typically an alkyl, typically a C1-C6 alkyl, for example a methyl, ethyl or propyl. In an embodiment, R8 is CH₃ (methyl).

In one preferred embodiment, R_{A} is H, an alkyl, -C(O)R7, -S(O)₂R7, -C(O)OR7,

In one preferred embodiment, R_{A} is H.

In one preferred embodiment, R_{A} is -C(O)R7.

In one preferred embodiment, R5 and R6 are each independently selected from the group consisting of H, C(O)OR⁹, an aryl or heteroaryl ring optionally substituted, wherein R9 is an alkyl group.

In one preferred embodiment, R5 and R6 form together an heteroaryl of the following structure: 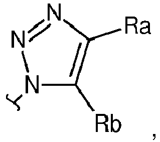 wherein Ra and Rb are each independently an atom or a chemical group of atoms, preferably selected from the group consisting of H, a halogen atom (Br, Cl, F), an alkyl group optionally comprising one or more heteroatoms (O, N, S), an optionally substituted aryl or heteroaryl, for example an optionally substituted phenyl ring, and wherein Ra and Rb may form together a cycle or heterocycle, optionally substituted.

In one preferred embodiment, the compound according to the invention is of the following structure:

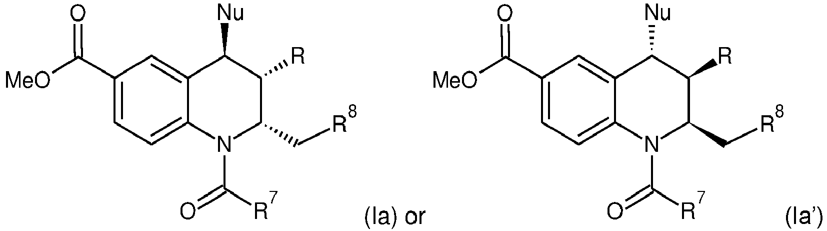

In one preferred embodiment, the compound according to the invention is 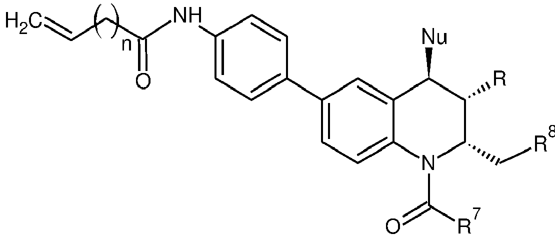 or

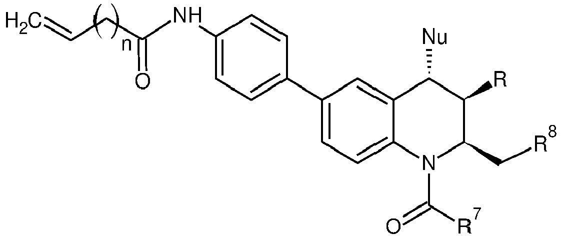

In one preferred embodiment, Nu is N₃. Advantageously, in such an embodiment wherein Nu is N₃, enable reactions of the click chemistry type to provide different compounds. Click Chemistry is a term describing reactions that are high yielding, wide in scope, creating only byproducts that can be removed without chromatography, and being stereospecific, simple to perform, and can be conducted in easily removable or benign solvents. Click chemistry is based on Azide-Alkyne Cycloaddition.

An example of click chemistry without the use of copper catalyst based compounds is: 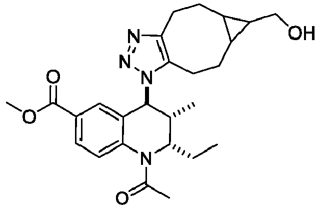 or the corresponding *cis*-2,3 and *trans-*3,4 enantiomer thereof.

In one preferred embodiment, Nu is

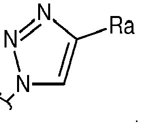

Such compound may be for example obtained by reacting a compound of structure I' or II' with

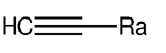

In one embodiment, a compound according to the invention has the following structure: 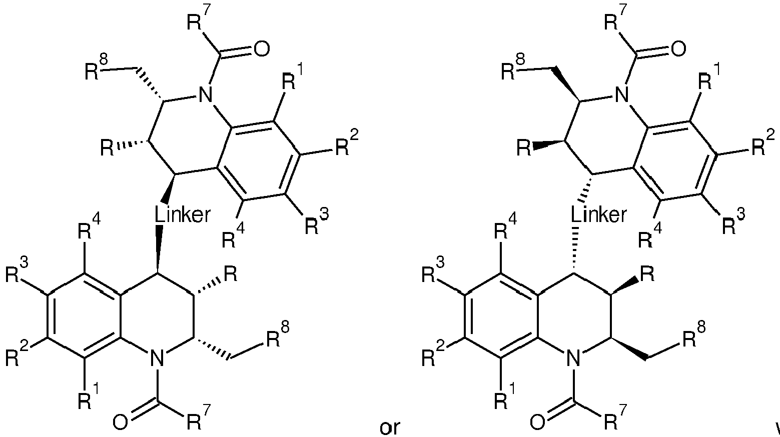 wherein « linker » represents a group of atoms; or 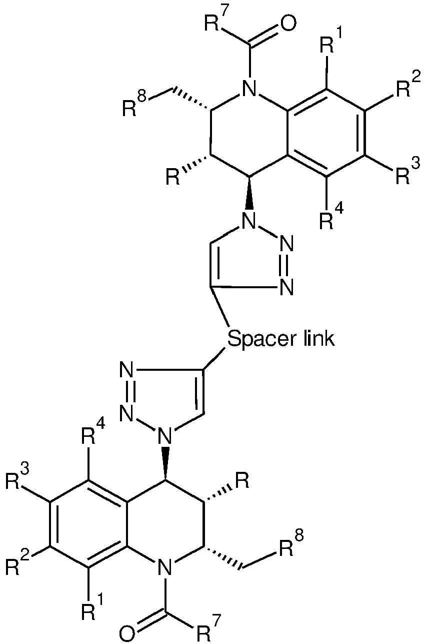 or a corresponding *cis*-2,3 and *trans-*3,4 enantiomer thereof: 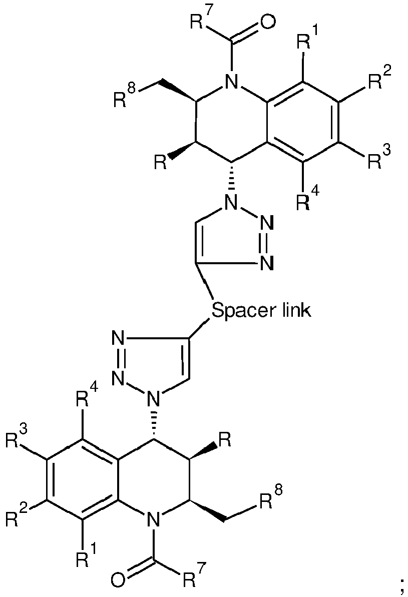 or 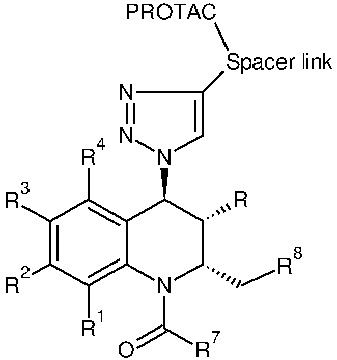 or the corresponding *cis*-2,3 and *trans-*3,4 enantiomer thereof: 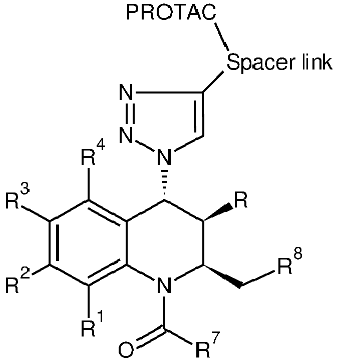 wherein the spacer link is a covalent link comprising several atoms

In one preferred embodiment, a compound according to the present invention has the following structure: 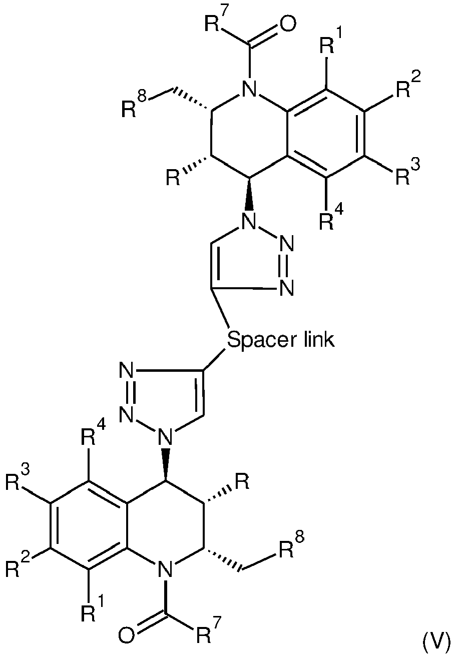 or a corresponding *cis*-2,3 and *trans-*3,4 enantiomer thereof: 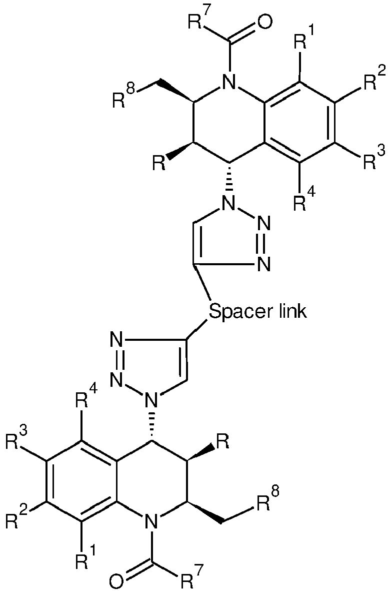 wherein the spacer link is a covalent link comprising several atoms. A skilled person knows many spacer links. The spacer link is functional to bind to or inhibit two targets, one target linking to one of the groups on each side of the spacer link. Such targets are for example BD1 and BD2.

An example of spacer link is -(CH₂O)n-, wherein n is an integer, for example ranging from 2 to 10, typically n is 2, 3, 4, 5 or 6..

An example of spacer link is -CH₂-(OC₂H₄)n-OCH₂-, wherein n is an integer, for example ranging from 1 to 10, typically n is 2, 3, 4, 5 or 6.

Also, in an embodiment, the compound of the present invention has a PROTAC function. Recently, PROTAC (Proteolysis-Targeting Chimeras) degraders have emerged as promising therapeutic strategy through utilization of the cells own protein destruction machinery to selectively degrade essential tumor drivers. A number of BET protein degraders (BBD) have been identified. In an embodiment, the compound of the present invention comprises one or more bromodomain and extra terminal domain (BET) proteolysis targeting chimera (PROTAC) (BET-PROTAC). In an embodiment a compound of the present invention has the following structure: 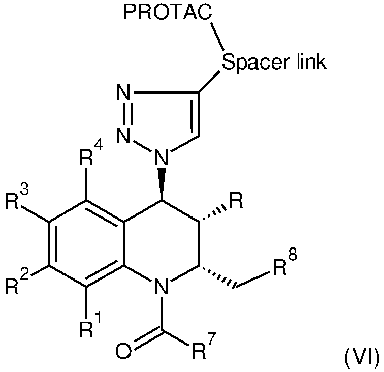 or the corresponding *cis*-2,3 and *trans-*3,4 enantiomer thereof: 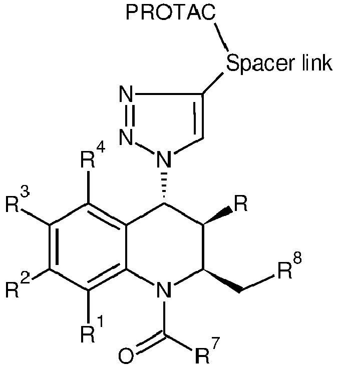 wherein the spacer link is a covalent link comprising several atoms, and PROTAC is a chemical group functioning as a PROTAC. A proteolysis targeting chimera (PROTAC) is a heterobifunctional small molecule composed of two active domains and a linker capable of removing specific unwanted proteins (the spacer link in the present invention). Rather than acting as a conventional enzyme inhibitor, a PROTAC works by inducing selective intracellular proteolysis. PROTACs consist of two covalently linked protein-binding molecules: one capable of engaging an E3 ubiquitin ligase, and another that binds to a target protein meant for degradation.

A skilled person knows many spacer links and PROTAC.

An example of spacer link is -(CH₂O)n-, wherein n is an integer, for example ranging from 2 to 10, typically n is 2, 3, 4, 5 or 6.

An example of spacer link is -CH₂-(OC₂H₄)n-OCH₂-, wherein n is an integer, for example ranging from 1 to 10, typically n is 2, 3, 4, 5 or 6.

An example of such compounds are: 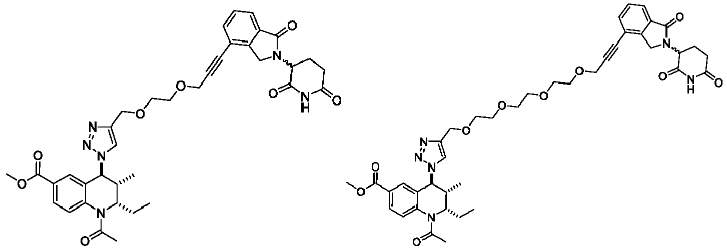 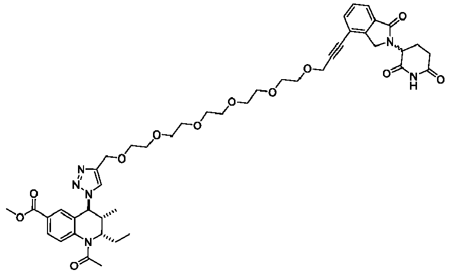 or the corresponding *cis*-2,3 and *trans-*3,4 enantiomers thereof.

In another embodiment, compounds of the invention are of the following structure 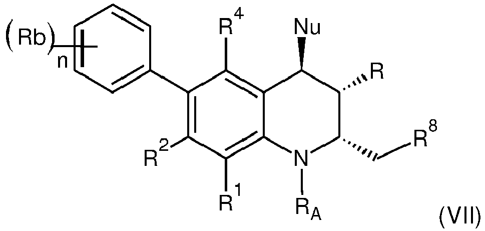 or the corresponding *cis*-2,3 and *trans-3,4* enantiomer thereof: 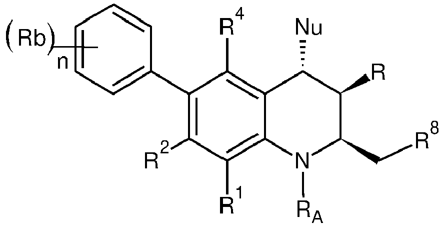 wherein Rb, independently at each occurrence, has the same meaning as R1, R2, R3, R4 and n represents the number of occurrence of Rb on the aromatic group.

In one embodiment of structure (VII), n is 1 and Rb is -NC(O)-(CH₂)ₘ(C(O)NHOH, wherein m is an integer, for example ranging from 1 to 20, typically m is 2, 3, 4, 5, 6, 7, 8, 9 or 10. An example of such compound is: 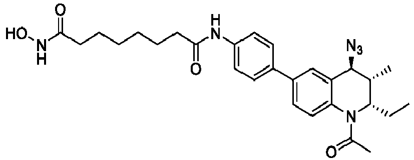 or the corresponding *cis*-2,3 and *trans-*3,4 enantiomer thereof.

In one embodiment, R is an alkyl, preferably in C1-C5, for example -C(CH₂)=CH₂, an alkoxy, for example an allyloxy or 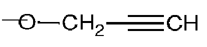 (prop-2-yn-1-yloxy), an aryl, preferably a phenyl group, and for example an alkyl-phenyl substituted on the phenyl group by a silane group, for example -CH₂₋phenyl-O-Si-trialkyl.

In one preferred embodiment, in the compound according to the invention, in particular of structure (I') R and R8 are identical.

In one embodiment, R3 is H, OCH₃, O-alkyl, preferably O-C₁-C₅alkyl, an ester, for example -C(O)O-alkyl, preferably C(O)OMe.

In an embodiment, R7 is an alkyl, typically a C1 to C5 alkyl.

In one embodiment, R7 is -CH₃ or -CH₂CH₃. Preferably, R7 is -CH₃.

In one embodiment, Nu is -S-aryl, for example -S-phenyl, -Se-aryl, for example-Se-phenyl,-NH-aryl, for example -NH-phenyl, OH, NH₂, -S-alkyl-SH.

In one embodiment, Nu is an isopropoxycarbonyl-amino group.

In one embodiment, Nu is an optionally substituted pyrimidin-2-yl-amino group.

In one embodiment, Nu is an optionally substituted 1H-1,2,3-triazol-1-yl group.

In one embodiment, Nu is an optionally substituted (phenyl)-1H-1,2,3-triazol-1-yl group.

In one embodiment, Nu is an optionally substituted 4-cyclohexyl-1H-1,2,3-triazol-1-yl group.

In one embodiment, wherein Nu is a substituted 1H-1,2,3-triazol-1-yl group said triazole is substituted by a group comprising a polyethylene glycol group. Example of such groups are: 2-(prop-2-yn-1-yloxy)ethoxy)methyl, oxybis(ethane-2,1-diyl))bis(oxy))bis(methylene), 2,5,8,11-tetraoxatetradec-13-yn-1-yl, 2,5,8,11,14-pentaoxaheptadec-16-yn-1-yl, 2,5,8,11,14,17-hexaoxaicos-19-yn-1-yl.

In one embodiment, -Nu and R form together a 5-member heterocycle, typically forming with the THQ ring a hexahydropyrroloquinoline. Such 5-member heterocycle can be substituted, for example by one or more alkyl groups and/or aryl groups, or -C(O)-alkyl, for example -C(O)-C₂H₅. Examples of substituted hexahydropyrroloquinoline are given in M. B. Haarr, M. O Sydnes, Molecules, 2021, 26, 341.

In one embodiment, Nu is a link forming a dimeric THQ.

Dimeric THQ are for example of the following structure: 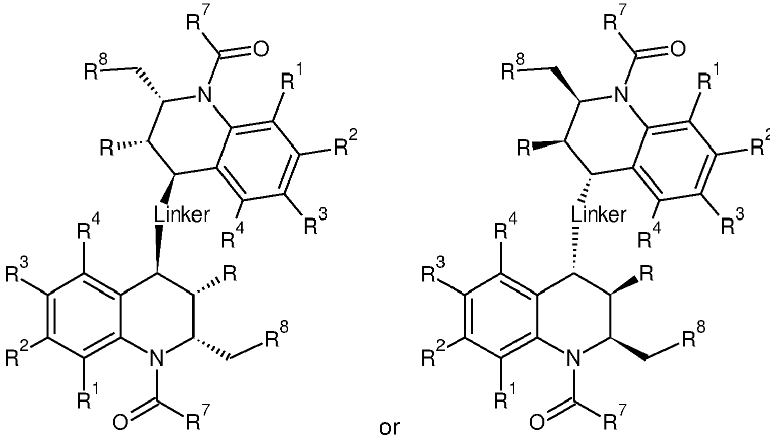 wherein « linker » represents a group of atoms, preferably as defined for Nu groups, wherein at least one atom (preferably terminal atom) is further linked to the resulting part of the dimer.

In one embodiment, a compound according to the present invention is selected from the group consisting of: 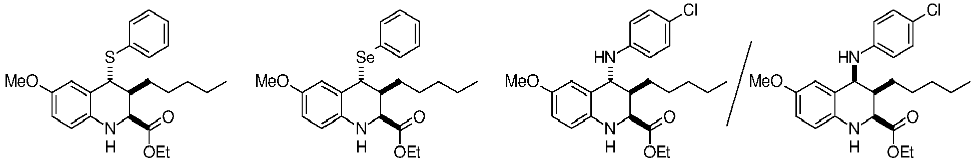 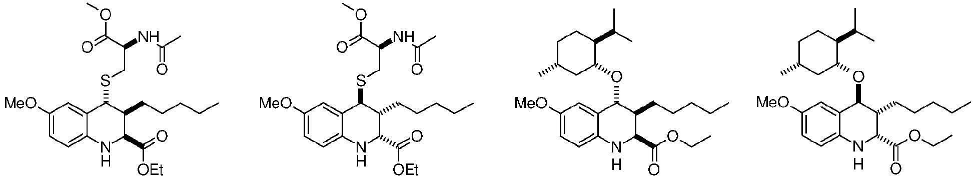 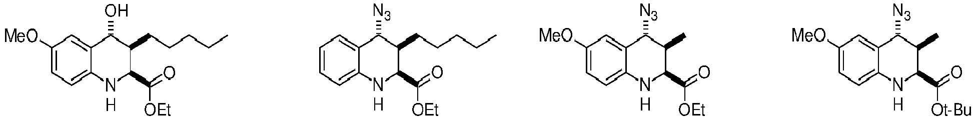 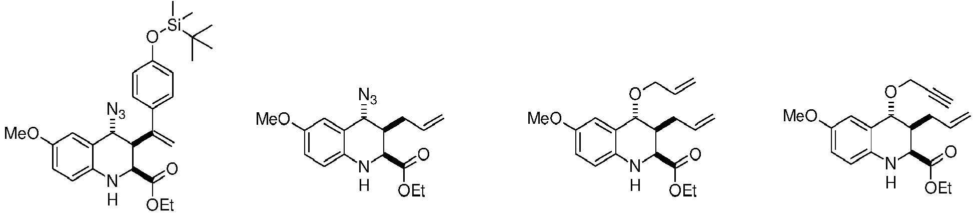 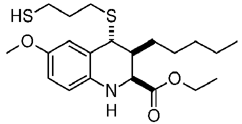 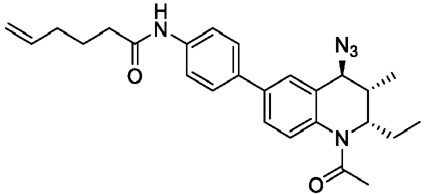 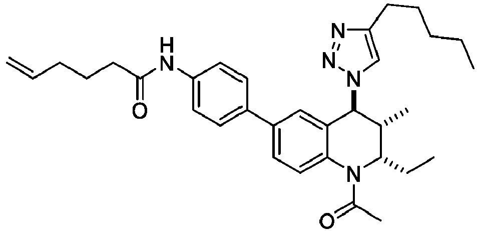 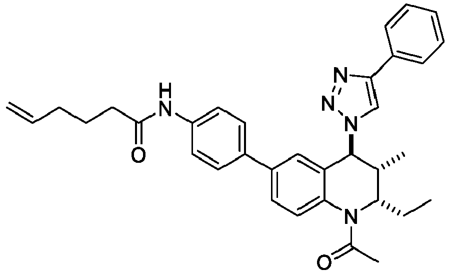 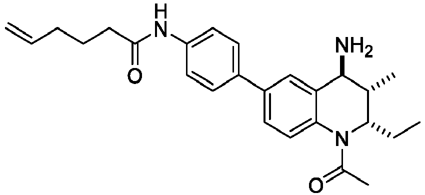 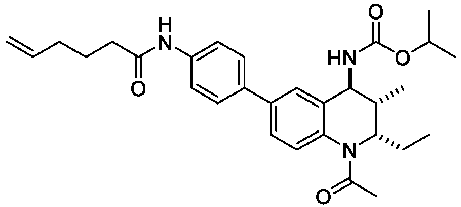 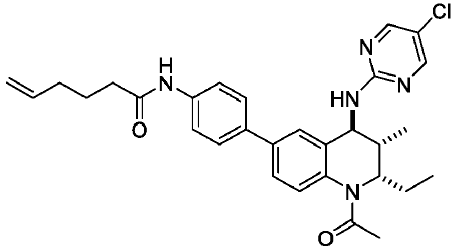 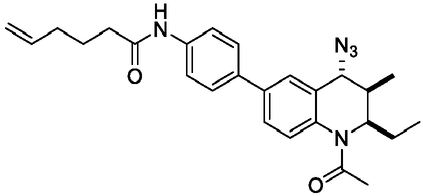 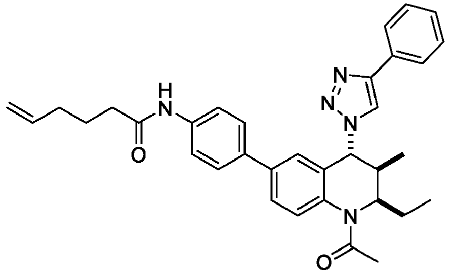 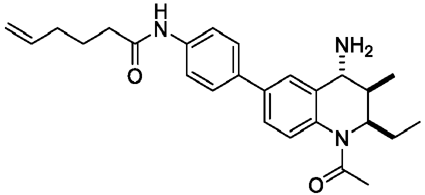 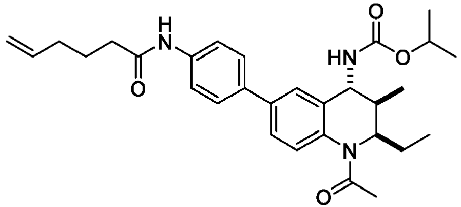 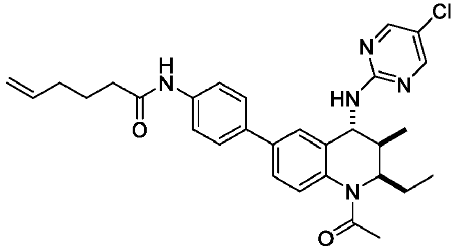 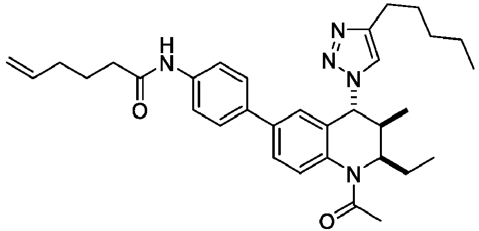 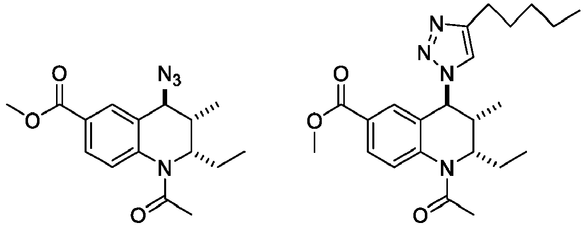 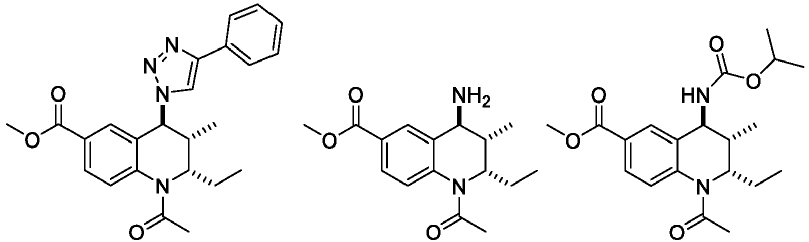 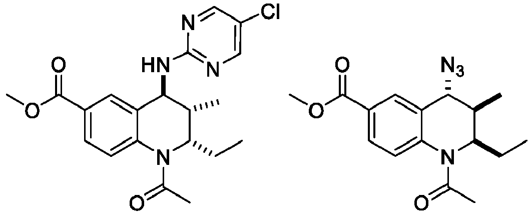 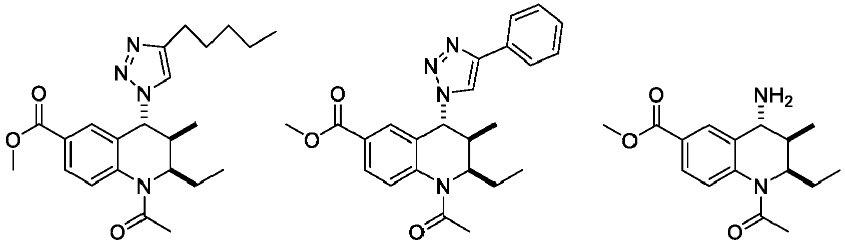 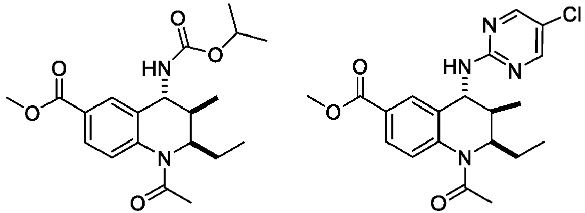 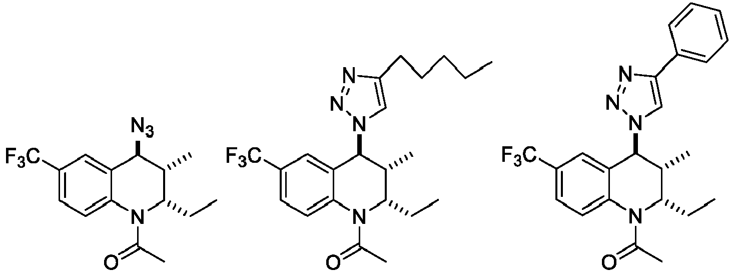 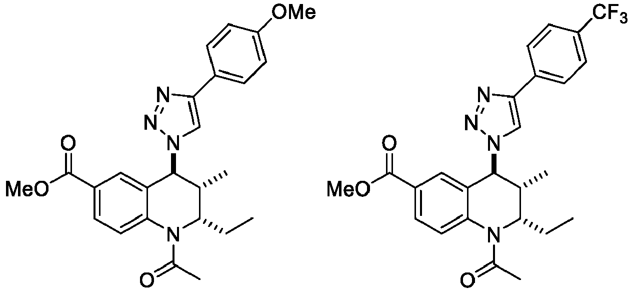 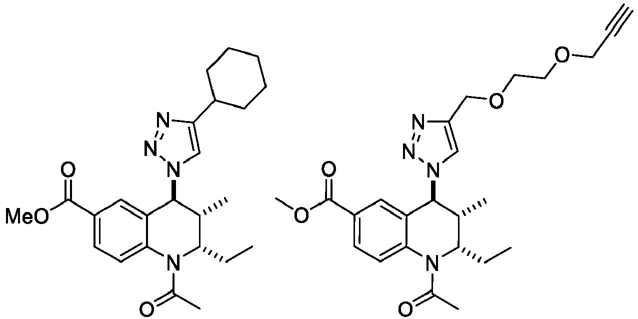 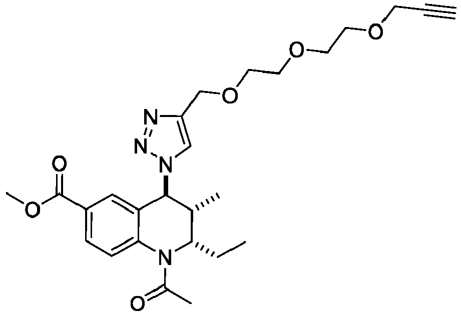 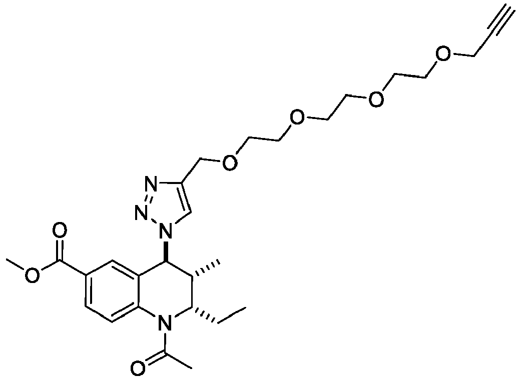 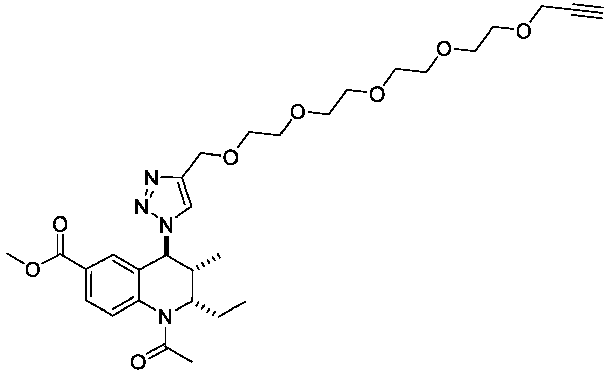 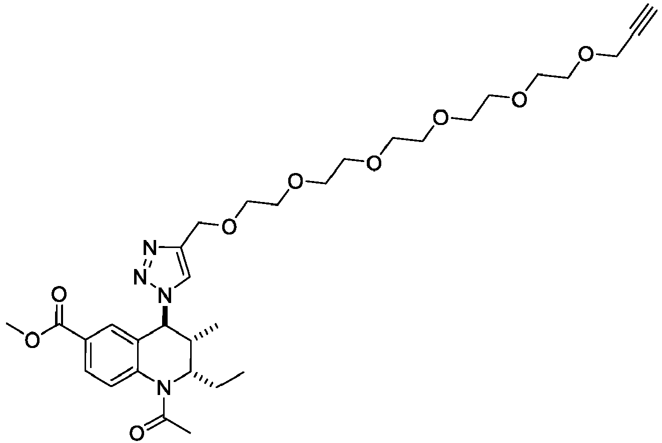 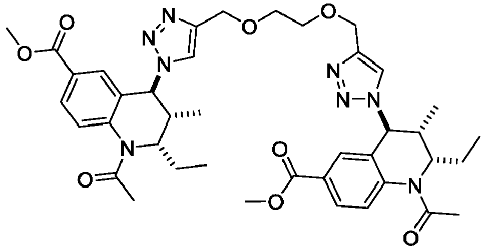 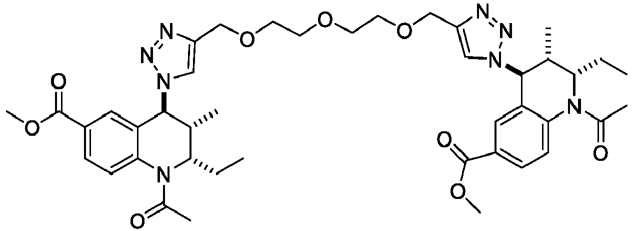 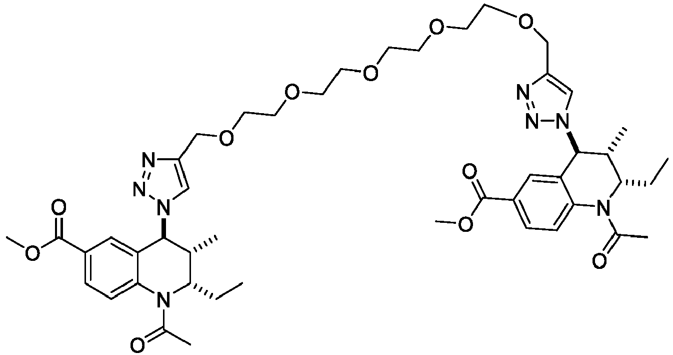 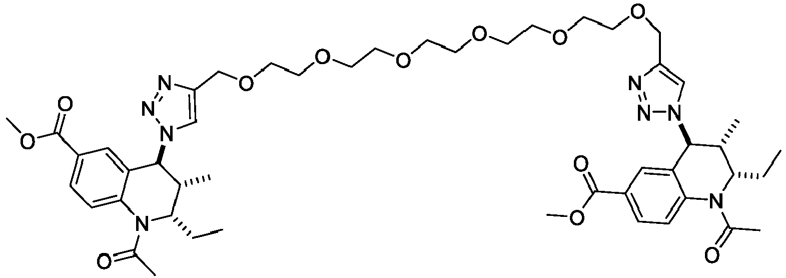 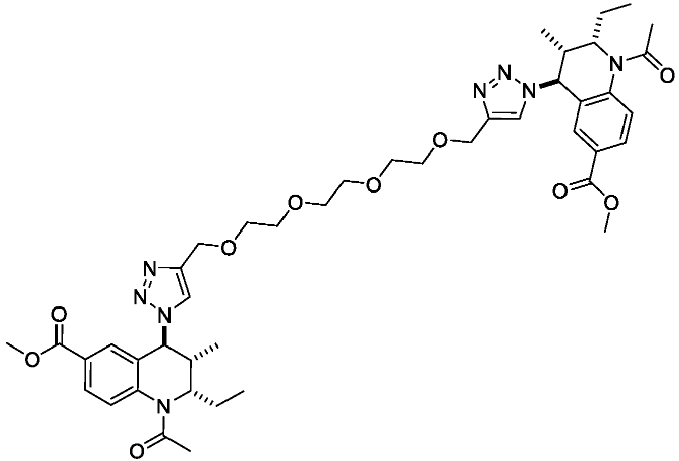 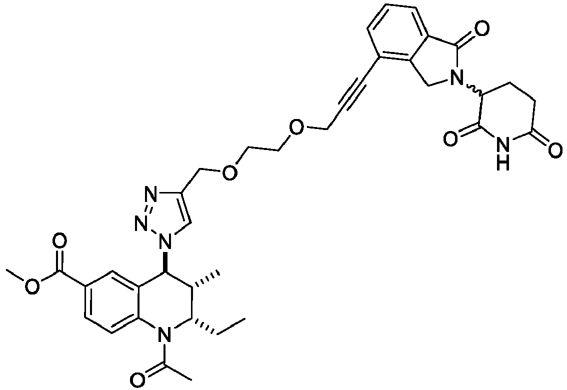 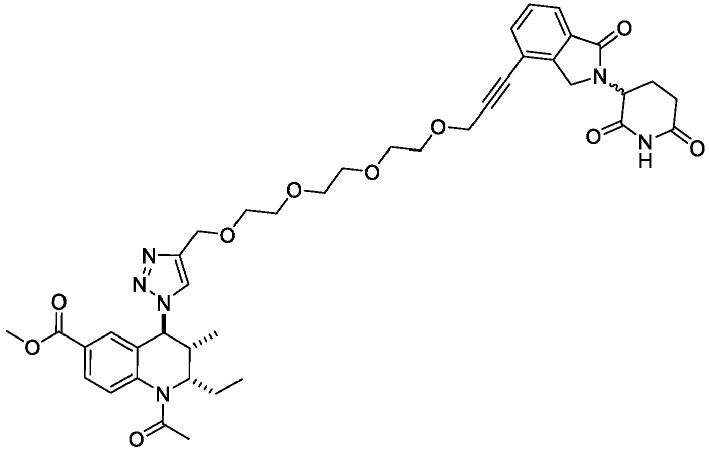 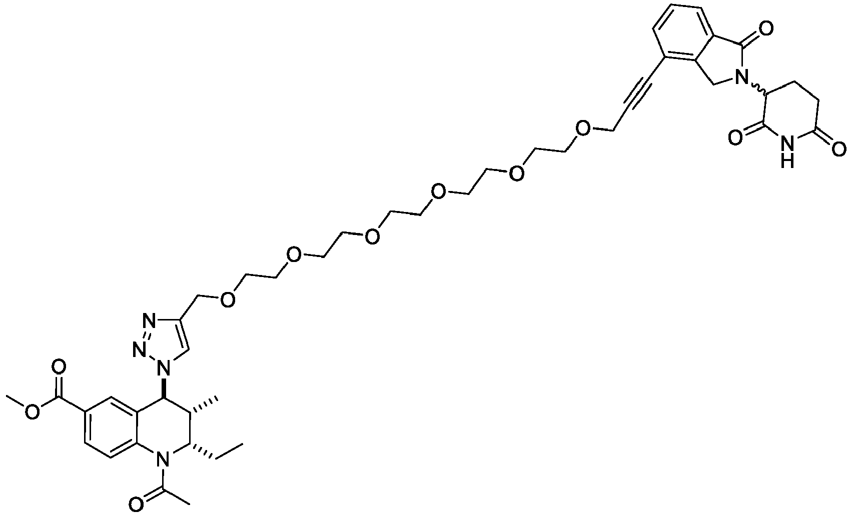 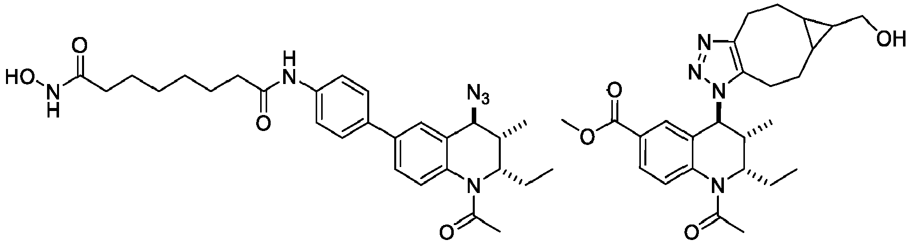 or the corresponding *cis*-2,3 and *trans-*3,4 enantiomer thereof.

In one embodiment, the compound is 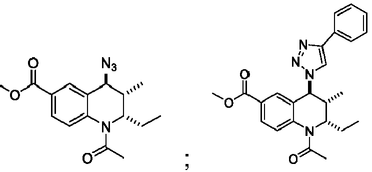 or 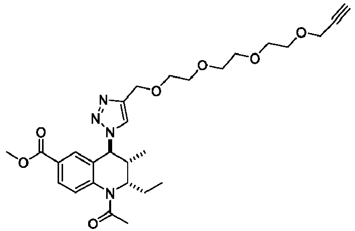 or the corresponding *cis*-2,3 and *trans-*3,4 enantiomer thereof.

In one embodiment, a compound of the present invention is a pure enantiomer. "Pure" refers to a compound comprising less than 5%, preferably less than 4%, preferably less than 3%, preferably less than 2%, preferably less than 1%, preferably less than 0.5%, preferably less than 0.1%, even preferably less than 0.01%, of the other enantiomers.

The present invention relates to a method for providing tetrahydroquinoline (THQ) compounds as described according to the present invention.

More particularly, the present invention relates to a method for selective production of tetrahydroquinoline, said method comprising reacting reactive compounds in the presence of (i) L-Proline and/or D-Proline and/or (S) silyl prolinol and/or (R) silyl prolinol and (ii) a Lewis acid to provide a tetrahydroquinoline compound.

The present invention relates in particular to a new route to prepare asymmetric THQ according to an enantioselective way.

Preferably, the present invention is a one-pot method to provide tetrahydroquinoline compounds, in particular a compound of structure I or II.

In one preferred embodiment, the method according to the invention is implementing reactive compounds, which are easily commercially available.

Advantageously, a Lewis acid providing the aza-o-orthoquinone methide thereby allowing the possibility of grafting the Nu nucleophile.

In one preferred embodiment, the method according to the invention is implementing cheap catalysts, such as proline and BF₃.OEt₂. To access to another configuration (*trans-*2,3 and *trans-*3,4 configuration), silyl prolinol can be used

Advantageously, the present invention provides THQ substituted in positions 2,3,4 in the configuration *cis*-2,3 and *trans-*3,4 (with proline), and also *trans-*2,3 and *trans-*3,4 (with silyl prolinol) in a one pot reaction.

In one embodiment, the method according to the invention comprises the reaction with L-proline in association with a Lewis acid.

In one embodiment, the method according to the invention comprises the reaction with D-proline in association with a Lewis acid.

In one embodiment, the method according to the invention comprises the reaction with (S)-silyl prolinol in association with a Lewis acid.

In one embodiment, the method according to the invention comprises the reaction with (R)-silyl prolinol in association with a Lewis acid.

Typically, the method according to the present invention involves a Mannich/Friedel Craft reaction, preferably according to the Chan route. The classical Chan route implements a chiral silyl prolinol for the first Mannich reaction and then POCl₃ for the Friedel Craft reaction. However, such classical route is unable to form the reactive intermediate azo-ortho-quinone methide provided by the present invention. In particular, the method of the present invention implements advantageously a Barbas-Mannich reaction in the presence of proline as catalyst starting from an imine, an aniline derivative and an aldehyde to yield a Mannich product in the cis-2,3 configuration.

A preferred aniline derivative has the following structure: 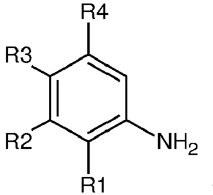 wherein R1, R2, R3 and R4 have the definition as mentioned in the present description.

Preferably, a Proline catalyst is implemented in this reaction to provides said Mannich product in the cis-2,3 configuration.

Preferably, in the presence of said Lewis acid based catalyst the very reactive aza-orthoquinone methide can be formed and react by an external nucleophile attack. Advantageously, according to the present invention the synthesis (the full sequence of reactions) is performed in one-pot, preferably by trapping (or scavenging) the aza-orthoquinone methide formed *in situ* by a nucleophilic compound.

Examples of Lewis acid based catalyst are the following compounds:
1) Lewis acid :
   - Lanthanide trifluoromethanesulfonates (ex : Yb(OTf)₃)
   - Transition metals (ex : Y(OTf)₃)
   - Post-transition metal (ex : Zn(OTf)₃)
2) Organic Lewis acid :
   - Carbocation (trityl), for example described by Naidu, Veluru Ramesh, Shengjun Ni, et Johan Franzén. «The Carbocation: A Forgotten Lewis Acid Catalyst ». ChemCatChem 7, n° 13 (3 juillet 2015): 1896-1905 (https://doi.org/10.1002/cctc.201500225).

In one embodiment, said Lewis acid based catalyst is BF₃.OEt₂.

In one embodiment, the method according to the invention comprises the following sequence in one-pot: 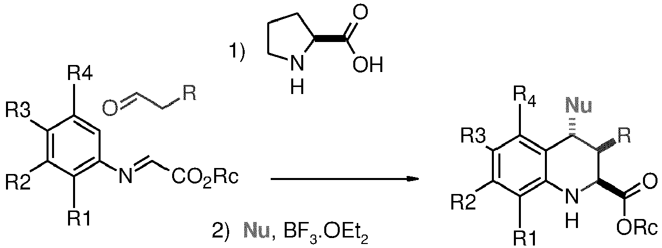 wherein Rc is a group of atoms, typically an alkyl group, for example an ethyl group.

The corresponding *cis*-2,3 and *trans-*3,4 enantiomer thereof may be obtained with the same reaction except reacting with D-proline instead of L-proline.

In one embodiment, R1, R2 and R4 are hydrogen atoms.

In one embodiment, the method according to the invention comprises the following sequence in one-pot, wherein equivalents, temperature and solvents are given for illustrative purpose:

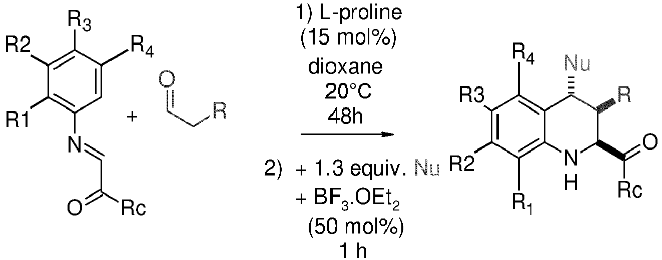

The corresponding *cis*-2,3 and *trans-3,4* enantiomer thereof may be obtained with the same reaction except reacting with D-proline instead of L-proline.

In one embodiment, the method according to the invention comprises the following sequence in one-pot, wherein equivalents, temperature and solvents are given for illustrative purpose:

### New multicomponent "one pot" general reaction

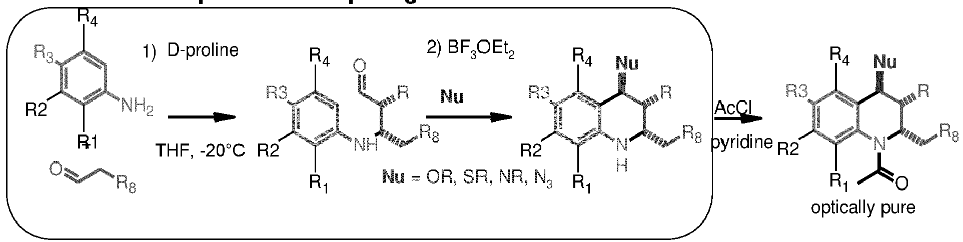

The corresponding *cis*-2,3 and *trans-*3,4 enantiomer thereof may be obtained with the same reaction except reacting with D-proline instead of L-proline.

### New multicomponent "one pot" general reaction

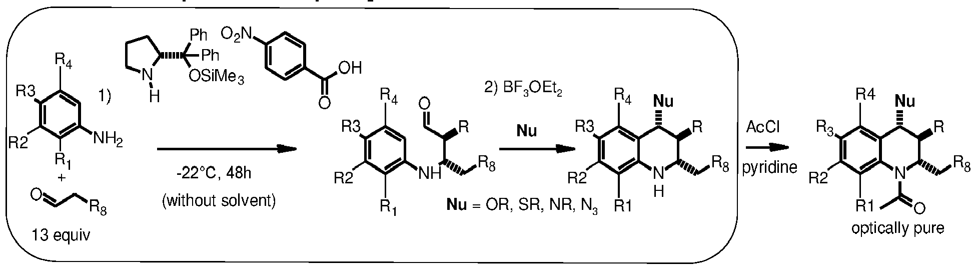

The corresponding *cis*-2,3 and *trans-*3,4 enantiomer thereof may be obtained with the same reaction except reacting with D-proline instead of L-proline.

In one embodiment, the method according to the invention comprises the following sequence in one-pot, wherein equivalents, temperature and solvents are given for illustrative purpose:

### one pot general reaction

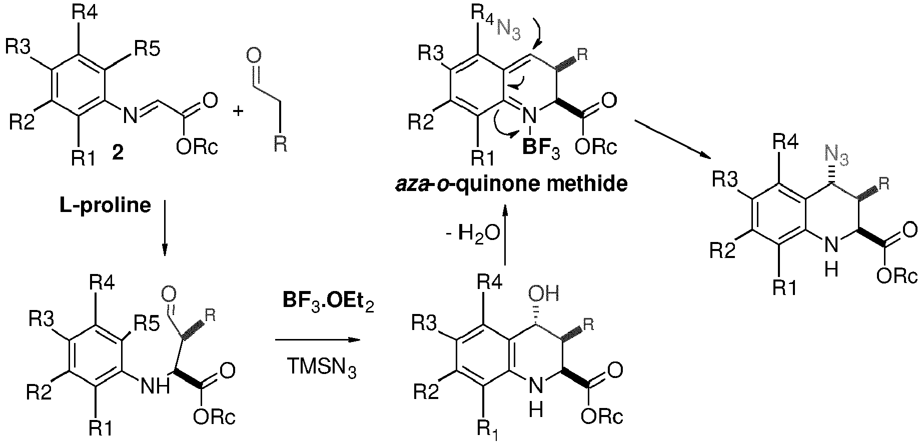

wherein Rc is a group of atoms, typically an alkyl group, for example an ethyl group.

### one pot example of reaction

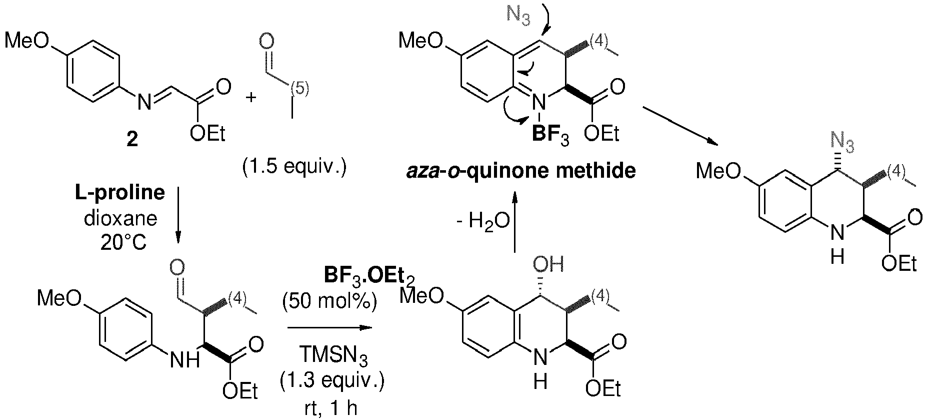

wherein (4) and (5) represent respectively the alkyl chain length (4 or 5 carbon atoms).

In one embodiment, said Lewis acid is BF₃OEt₂.

In the present invention, the term carbon atom chain refers to a chain comprising in the present invention the term "alkyl group" refers to a linear, branched or cyclic alkyl group, optionally substituted and/or optionally containing one or more heteroatoms in the alkyl chain. Preferably the "alkyl group" is a linear or branched, substituted or unsubstituted, C1-C10 alkyl group, such as C1-C4 or C1-C6, in particular a methyl, ethyl group, propyl group, preferably methyl. A cyclic alkyl group is a cycloalkyl group. The term "cycloalkyl group" means a saturated or partially unsaturated, monocyclic, fused bicyclic or bridged polycyclic ring assembly containing the number of ring atoms indicated. This includes substituted or unsubstituted cylcoalkyl groups. For example, cycloalkyl group may be C3-C10 alkyl group, such as C5 or C6, in particular a cyclopropyl, cyclobutyl, cyclopentyl, cyclohexyl, cycloheptyl or cyclooctyl group etc. Cycloalkyl groups include heterocycle, by replacing one or more carbon atoms of the cycloalkyl group by one or more nitrogen, oxygen, or sulphur atoms an alkyl group containing one or more oxygen (heteroatom) is a cycloalkyl group. Examples of suitable alkoxy groups include methoxy, ethoxy, n-propoxy, l-propoxy, n-butoxy, s-butoxy, t-butoxy.

The term "aryl group" means a substituted or unsubstituted monocylic or fused bicycic aromatic ring assembly containing six to ten ring carbon atoms. For example, aryl may be phenyl or naphthyl, preferably phenyl. The term "heteroaryl group" is as defined for aryl group where one or more of the ring members is a heteroatom. For example heteroaryl groups includes pyridyl, indolyl, indazolyl, quinoxalinyl, quinolinyl, benzofuranyl, benzopyranyl, benzothiopyranyl, benzo[I,3]dioxole, imidazolyl, benzo-imidazolyl, pyrimidinyl, furanyl, oxazolyl, isoxazolyl, triazolyl, tetrazolyl, pyrazolyl, thienyl, etc. Aryl and heteroaryl also include akylaryl and alkylheteroaryl groups, i.e. an aryl or heteroaryl group linked by an alkyl part to the rest of the molecule. Also, Aryl and heteroaryl include aralkyl and heteroaralkyl groups, i.e. aryl and heteroaryl substituted by one or more alkyl groups.

Advantageously, the present invention provides a very simple method to provide the compounds of the invention.

Advantageously, the method according to the present invention is reproducible and do not needs to implement hazardous reacting conditions.

Advantageously, the method according to the present invention is ecofriendly, i.e. economical in the number of synthesis steps (less time, less purification, less solvents consumed), use of a natural catalyst, i.e. proline, catalytic synthesis (thereby economical in the quantity of reagents).

BET proteins (Bromodomain and ExtraTerminal Domain) are regulators of epigenetic mechanisms involved in gene expression. The BET proteins (BRD2, BRD3, BRD4 and BRDT) have two bromodomains (BD), namely BD1 and BD2, which recognize acetylated lysins in histones. These C-terminal regions of histones are involved in the recruitment of molecular complexes essential for the activation and regulation of transcription. Inhibitors that prevent bromodomains from interacting with these regions are recent targets of choice in therapeutics and referred to as BET inhibitors (iBETs). The compounds of the present invention, in particular compounds of formula (I) or (II), are BET inhibitors.

Advantageously, a compound of the invention, in particular a compound of formula (I) or (II) is for use as a selective BET inhibitor.

Advantageously, the compounds of the invention, in particular compounds of formula (I) or (II), have shown efficacy in inhibiting BRD.

In one embodiment, the compounds of the present invention, in particular compounds of formula (I) or (II), are BRD4 inhibitors.

Preferred compounds according to the invention, in particular compounds of formula (I) or (II), present a selectivity for BD2 toward BD1.

Advantageously, the compounds of the invention, in particular compounds of formula (I) or (II), present a very low cell toxicity.

Advantageously, the compounds of the present invention, in particular compounds of formula (I) or (II), are anti-inflammatory compounds.

Advantageously, the compounds of the invention, in particular compounds of formula (I) or (II), are effective in inhibiting the expression of IL-6 and TNF-α.

Advantageously, the compounds of the present invention, in particular compounds of formula (I) or (II), are good candidates for anti-cancer compounds.

In one embodiment, the compounds of the present invention are used in the treatment of an inflammation, preferably involving expression of IL-6 and/or TNF-α, or BET-dependent cancers: hematological malignancies (leukemia, lymphoma, multiple myeloma), solid tumors (prostate cancer, breast, lung,...) or inflammation-induced cancers (for example hepatocellular carcinoma).

According to WO 2016/038120, Bromodomain inhibitors are believed to be useful in the treatment of a variety of diseases or conditions related to systemic or tissue inflammation, inflammatory responses to infection or hypoxia, cellular activation and proliferation, lipid metabolism, fibrosis and in the prevention and treatment of viral infections.

Bromodomain inhibitors may be useful in the treatment of a wide variety of acute or chronic autoimmune and/or inflammatory conditions such as rheumatoid arthritis, osteoarthritis, acute gout, psoriasis, systemic lupus erythematosus, multiple sclerosis, inflammatory bowel disease (Crohn's disease and Ulcerative colitis), asthma, chronic obstructive airways disease, pneumonitis, myocarditis, pericarditis, myositis, eczema, dermatitis (including atopic dermatitis), alopecia, vitiligo, bullous skin diseases, nephritis, vasculitis, hypercholesterolemia, atherosclerosis, Alzheimer's disease, depression, Sjogren's syndrome, sialoadenitis, central retinal vein occlusion, branched retinal vein occlusion, Irvine-Gass syndrome (post cataract and post-surgical), retinitis pigmentosa, pars planitis, birdshot retinochoroidopathy, epiretinal membrane, cystic macular edema, parafoveal telengiectasis, tractional maculopathies, vitreomacular traction syndromes, retinal detachment, neuroretinitis, idiopathic macular edema, retinitis, dry eye (keratoconjunctivitis Sicca), vernal keratoconjunctivitis, atopic keratoconjunctivitis, uveitis (such as anterior uveitis, pan uveitis, posterior uveitis, uveitis-associated macular edema), scleritis, diabetic retinopathy, diabetic macula edema, age-related macular dystrophy, hepatitis, pancreatitis, primary biliary cirrhosis, sclerosing cholangitis, Addison's disease, hypophysitis, thyroiditis, type I diabetes, type II diabetes, giant cell arteritis, nephritis including lupus nephritis, vasculitis with organ involvement such as glomerulonephritis, vasculitis including giant cell arteritis, Wegener's granulomatosis, Polyarteritis nodosa, Behcet's disease, Kawasaki disease, Takayasu's Arteritis, pyoderma gangrenosum, vasculitis with organ involvement and acute rejection of transplanted organs.

Bromodomain inhibitors may be useful in the treatment of diseases or conditions which involve inflammatory responses to infections with bacteria, viruses, fungi, parasites or their toxins, such as sepsis, acute sepsis, sepsis syndrome, septic shock, endotoxaemia, systemic inflammatory response syndrome (SIRS), multi-organ dysfunction syndrome, toxic shock syndrome, acute lung injury, ARDS (adult respiratory distress syndrome), acute renal failure, fulminant hepatitis, burns, acute pancreatitis, post-surgical syndromes, sarcoidosis, Herxheimer reactions, encephalitis, myelitis, meningitis, malaria and SIRS associated with viral infections such as influenza, herpes zoster, herpes simplex and coronavirus. In one embodiment the disease or condition which involves an inflammatory response to an infection with bacteria, a virus, fungi, a parasite or their toxins is acute sepsis.

Bromodomain inhibitors may be useful in the treatment of viral infections.

Bromodomain inhibitors may be useful in the treatment of cancer, including hematological (such as leukaemia, lymphoma and multiple myeloma), epithelial including lung, breast and colon carcinomas, midline carcinomas, mesenchymal, hepatic, renal and neurological tumours.

In one embodiment the disease or condition for which a bromodomain inhibitor is indicated is selected from diseases associated with systemic inflammatory response syndrome, such as sepsis, burns, pancreatitis, major trauma, haemorrhage and ischaemia. In this embodiment the bromodomain inhibitor would be administered at the point of diagnosis to reduce the incidence of SIRS, the onset of shock, multi-organ dysfunction syndrome, which includes the onset of acute lung injury, ARDS, acute renal, hepatic, cardiac or gastro-intestinal injury and mortality. In another embodiment the bromodomain inhibitor would be administered prior to surgical or other procedures associated with a high risk of sepsis, haemorrhage, extensive tissue damage, SIRS or MODS (multiple organ dysfunction syndrome). In a particular embodiment the disease or condition for which a bromodomain inhibitor is indicated is sepsis, sepsis syndrome, septic shock and endotoxaemia. In another embodiment, the bromodomain inhibitor is indicated for the treatment of acute or chronic pancreatitis. In another embodiment the bromodomain is indicated for the treatment of burns.

As used herein the reference to the "treatment" of a particular disease or condition includes the prevention or prophylaxis of such a disease or condition.

The term "diseases or conditions for which a bromodomain inhibitor is indicated", is intended to include each of or all of the above diseases or conditions. While it is possible that for use in therapy, a compound of the present invention as well as pharmaceutically acceptable salts thereof may be administered as the raw chemical, it is common to present the active ingredient as a pharmaceutical composition.

In one embodiment, a compound of the present invention or a composition of the present invention is for use in a method for treating an inflammation or a cancer, preferably a cancer involving BET over-expression.

The present invention also relates to a composition, in particular a pharmaceutical composition, comprising a compound as described according to the present invention.

In a further aspect, the present invention relates to a pharmaceutical composition comprising a compound of the present invention or a pharmaceutically acceptable salt thereof and one or more pharmaceutically acceptable carriers, diluents or excipients.

The composition of the present invention may conveniently be presented for use in the form of a pharmaceutical composition and thus pharmaceutical compositions comprising a combination as defined above together with a pharmaceutically acceptable diluent or carrier represent a further aspect of the invention.

The present invention also relates to a method for preparing a medicament or pharmaceutical composition comprising one or more compounds as defined in the present invention.

The present invention also relates to the use of one or more compounds as defined in the present invention in a method for preparing a medicament or pharmaceutical composition.

The present invention also relates to a method of therapeutic treatment of a human, said method comprising the administration of one or more compounds as defined in the present invention to said human in an effective amount, preferably in a medicament or pharmaceutical composition.

In the present invention, the terms "according to the invention" or "of the present invention" or the like refer to all and any embodiments, preferred and advantageous features alone or in any possible combination.

Other aims, characteristics and advantages of the invention will appear clearly to the person skilled in the art upon reading the explanatory description which makes reference to the Examples which are given simply as an illustration and which in no way limit the scope of the invention.

The Examples make up an integral part of the present invention, and any characteristic which appears novel with respect to any prior state of the art from the description taken in its entirety, including the Examples, makes up an integral part of the invention in its function and in its generality.

Thus, every example has a general scope.

Furthermore, in the Examples, all percentages are given by weight, unless indicated otherwise, temperature is expressed in degrees Celsius unless indicated otherwise, and the pressure is atmospheric pressure, unless indicated otherwise.

On the figures
Figure 1 shows a graphic representing the evaluation of cytotoxicity after iBET/LPS treatment (on J774 mouse macrophages).

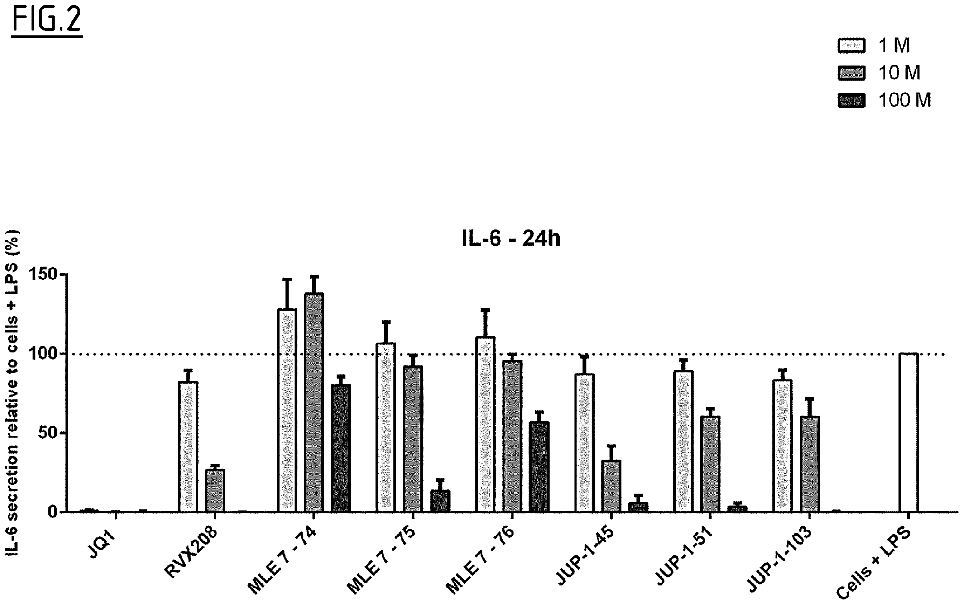

Figure 2 shows a graphic representing the evaluation of IL-6 cytokine expression after iBET/LPS treatment at 24h of different compounds.

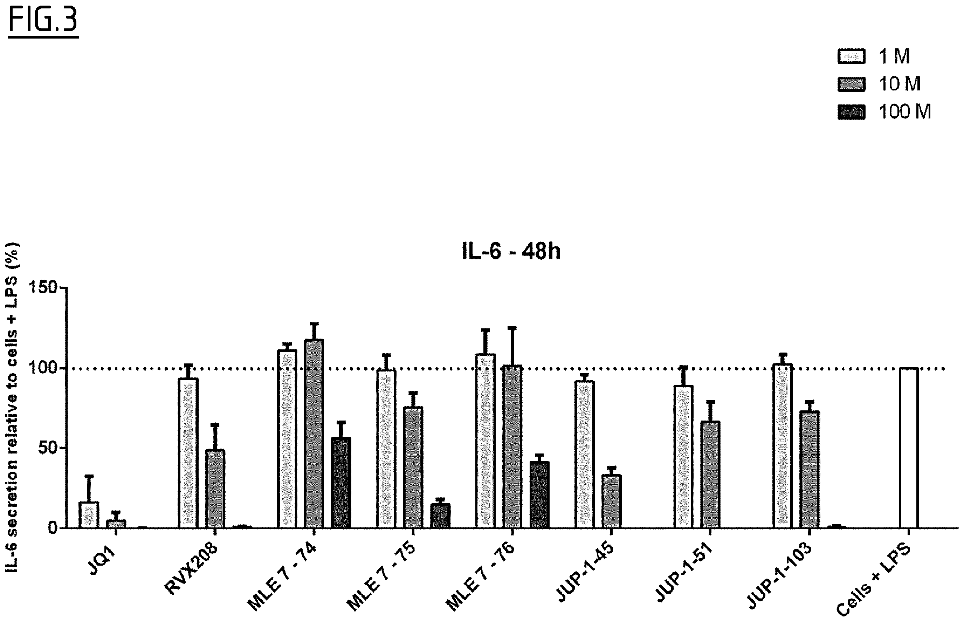

Figure 3 shows a graphic representing the evaluation of IL-6 cytokine expression after iBET/LPS treatment at 48h of different compounds.

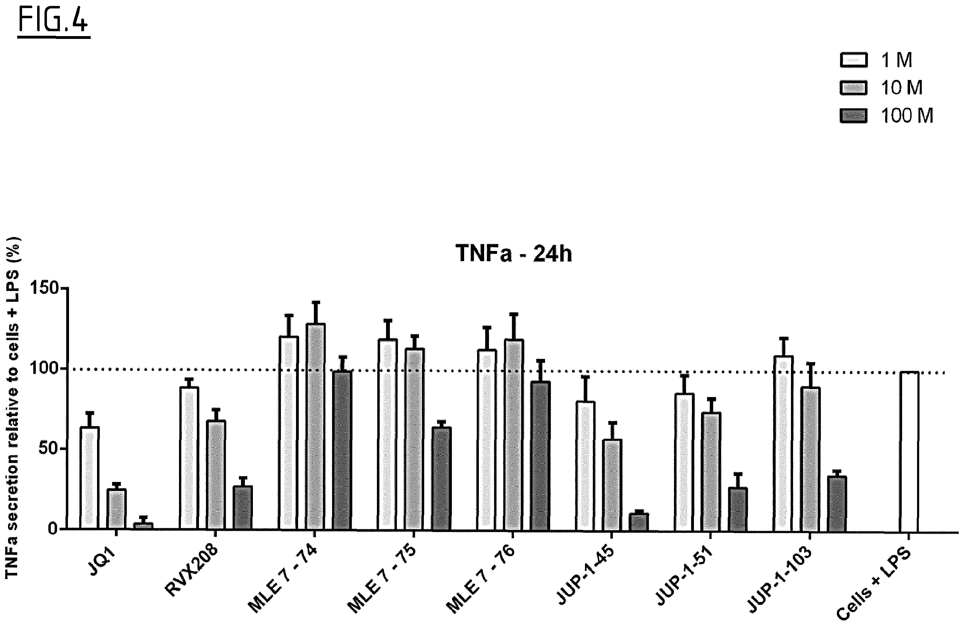

Figure 4 shows a graphic representing the evaluation of TNF-α cytokine expression after iBET/LPS treatment at 24h of different compounds.
Figure 5 shows a graphic representing the evaluation of TNF-α cytokine expression after iBET/LPS treatment at 48h of different compounds.

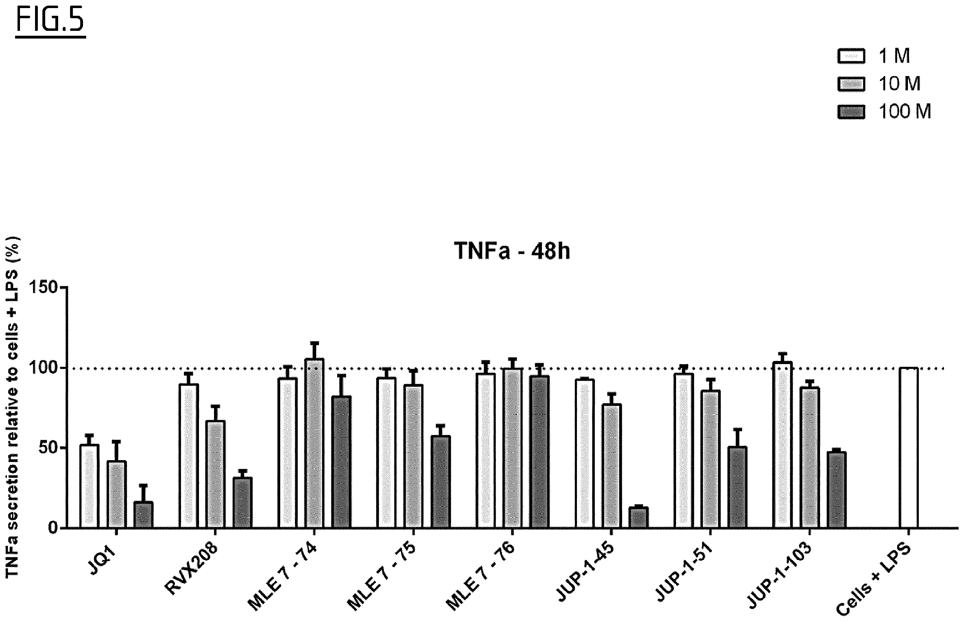

Figure 5 shows a graphic representing the HTRF signal as a function of different inhibitors concentration (MLE-6-161, MLE-6-162 are compounds of the invention whereas MLE-7-74, MLE-7-75 , MLE-7-76 and JQ1 are compound of the prior art).

### Examples

### (2S,3S,4R)-Ethyl-4-azido-6-methoxy-3-pentyl-1,2,3,4-tetrahydroquinoline-2-carboxylate (7)

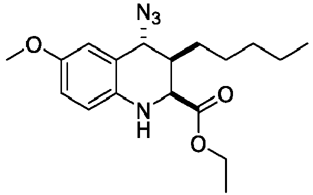

### Method A

To a solution of ethyl (*E*)-2-((4-methoxyphenyl)imino)acetate **2** (300.0 mg, 1.45 mmol) and freshly distilled heptanal (248.0 mg, 2.17 mmol) in anhydrous dioxane (10 mL) was added L-proline (26.4 mg, 0.21 mmol) at rt. The reaction mixture was stirred for 48 h at the same temperature. After the reaction was complete, TMSN₃ (216.0 mg, 1.88 mmol) and BF₃.Et₂O (90 µL, 0.73 mmol) were added and the mixture was stirred at rt for 1 h. The reaction was quenched with NaOH (2 N) until neutralization, extracted with EtOAc, dried by MgSO₄, filtered off and concentrated by vacuum. After flash chromatography (silica gel, 5% EtOAc/cyclohexane), the product **7** was isolated as an oil (333.0 mg, 66.4%); R*_{f}* = 0.27 (10% EtOAc/cyclohexane); [α]_{D}²⁰ = +126.6 (c = 1.03, CHCl₃); IR vₘₐₓ (thin film, CH₂Cl₂) 3393, 2954, 2927, 2091, 1734, 1622, 1501, 1463, 1224 cm⁻¹; UV : 208, 245, 325; ¹H NMR (400 MHz, CDCl₃) *δ* ppm 0.84 (t, *J* = 6.9 Hz, 3H), 0.94-1.29 (m, 8H), 1.32 (t, *J* = 7.1 Hz, 3H), 2 .24 (m, 1H), 3.76 (s, 3H), 4.19 (m, 1H), 4.26 (m, 1H), 4.29 (m, 1H), 4.40 (d, *J* = 2.4 Hz, 1H), 6.64 (d, *J* = 8.8 Hz, 1H), 6.68 (d, *J* = 2.8 Hz, 1H), 6.81 (dd, *J* = 8.8, 2.8 Hz, 1H); ¹³C NMR (100 MHz, CDCl₃) *δ* ppm 14.1 (CH₃), 14.4 (CH₃), 22.6 (CH₂), 26.4 (CH₂), 27.0 (CH₂), 31.8 (CH₂), 38.6 (CH), 53.5 (CH), 56.0 (CH₃), 61.4 (CH), 61.6 (CH₂), 115.6 (CH), 115.8 (C), 116.3 (CH), 117.1 (CH), 136.8 (C), 151.9 (C), 172.5 (C); LRMS (ESI+) *m*/*z* (%) 347 (20) [*M*+H]⁺, 304 (100); HRMS (ESI+) *m*/*z* calc. for C₁₈H₂₇N₄O₃ 347.2078, found 347.2077.

### (2S,3S,4R)-Ethyl-6-methoxy-3-pentyl-4-(phenylthio)-1,2,3,4-tetrahydroquinoline-2-carboxylate (8)

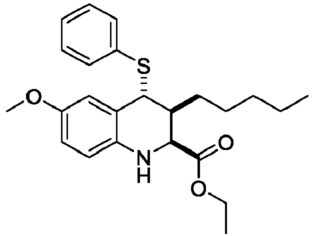

From imine **2** (300.0 mg, 1.45 mmol), freshly distilled heptanal (248.0 mg, 2.17 mmol) and thiophenol (206.0 mg, 1.88 mmol) in dioxane (10 mL), and using method **A,** the product was obtained after flash chromatography (silica gel, 5% EtOAc/ cyclohexane) to give **8** (329.0 mg, 55%); R*_{f}* = 0.17 (5% EtOAc/cyclohexane); [α]_{D}²⁰ = +110 (c = 1.08, CHCl₃); IR vₘₐₓ (thin film, CH₂Cl₂) 3393, 2900, 1731, 1504, 1463, 1215, 1150 cm⁻¹; UV: 206, 223, 245, 328; ¹H NMR (400 MHz, CDCl₃) *δ* ppm 0.76 (*J* = 7.3 Hz, 3H), 0.93-1.21 (m, 8H), 1.28 (t, *J* = 7.1Hz, 3H), 2.30 (m, 1H), 3.69 (s, 3H), 4.17-4.28 (m, 2H), 4.28 (d, *J* = 1.8 Hz, 1H), 4.68 (d, *J* = 2.8 Hz, 1H), 6.57 (d, *J* = 8.6 Hz, 1H), 6.70 (dd, *J* = 8.6, 2.8 Hz, 1H), 6.73 (d, *J* = 2.8 Hz, 1H), 7.28-7.38 (m, 3H), 7.53 (dd, J= 8.1, 1.4 Hz, 2H); ¹³C NMR (100 MHz, CDCl₃) *δ* ppm 14.1 (CH₃), 14.3 (CH₃), 22.6 (CH₂), 26.5 (CH₂), 27.3 (CH₂), 31.4 (CH₂), 37.8 (CH), 50.5 (CH), 53.5 (CH), 55.9 (CH₃), 61.3 (CH₂), 115.9 (CH), 116.2 (2xCH), 118.0 (C), 127.9 (CH), 129.2 (2xCH), 133.4 (2xCH), 134.9 (C), 137.1 (C), 152.0 (C), 173.1 (C); LRMS (ESI+) *m*/*z* (%) 414 (20) [*M*+H]⁺, 304 (100); HRMS (ESI+) *m*/*z* calc. for C₂₄H₃₂NO₃S 414.2097, found 414.2093.

### (2S,3S,4R)-Ethyl-6-methoxy-3-pentyl-4-(phenylselanyl)-1,2,3,4-tetrahydroquinoline-2-carboxylate (9)

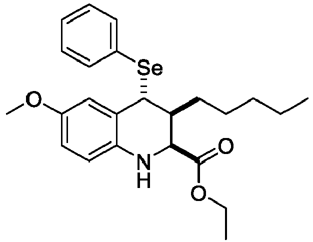

From imine **2** (300.0 mg, 1.45 mmol), freshly distilled heptanal (248.0 mg, 2.17 mmol) and benzeneselenol (296.0 mg, 1.88 mmol), and using method **A,** the product was obtained after flash chromatography (silica gel, 5% EtOAc/cyclohexane) to give **9** (220.0 mg, 33%); R*_{f}* = 0.23 (5% EtOAc/cyclohexane); [α]_{D}²⁰ = +135 (c = 1.01, CHCl₃); IR vₘₐₓ (thin film, CH₂Cl₂) 3398, 2926, 1733, 1505, 1467, 1213, 1036 cm⁻¹; UV: 206, 226, 335; ¹H NMR (400 MHz, CDCl₃) *δ* ppm 0.75 (*J* = 7.3 Hz, 3H), 0.86-1.18 (m, 8H), 1.29 (t, *J =* 7.1Hz, 3H), 2.30 (m, 1H), 3.70 (s, 3H), 4.17-4.30 (m, 2H), 4.52 (d, *J* = 1.6 Hz, 1H), 4.79 (d, *J* = 2.8 Hz, 1H), 6.54 (d, *J* = 8.7 Hz, 1H), 6.67 (dd, *J* = 8.7, 2.9 Hz, 1H), 6.72 (d, *J* = 2.8 Hz, 1H), 7.29-7.37 (m, 3H), 7.65 (m, 2H); ¹³C NMR (100 MHz, CDCl₃) *δ* ppm 14.1 (CH₃), 14.4 (CH₃), 22.6 (CH₂), 26.5 (CH₂), 27.4 (CH₂), 31.3 (CH₂), 38.3 (CH), 46.2 (CH), 54.2 (CH), 55.9 (CH₃), 61.4 (CH₂), 115.6 (CH), 155.9 (CH), 116.2 (CH), 118.9 (C), 128.3 (CH), 129.3 (2xCH), 130.3 (C), 135.8 (2xCH), 136.9 (C), 152.0 (C), 173.2 (C); LRMS (ESI+) *m*/*z* (%) 462 (15) [*M*+H]⁺, 304 (100); HRMS (ESI+) *m*/*z* calc. for C₂₄H₃₂NO₃Se 462.1543, found 462.1542.

### (2S,3R,4R)-Ethyl-4-((4-chlorophenyl)amino)-6-methoxy-3-pentyl-1,2,3,4-tetrahydroquinoline-2-carboxylate (10a)

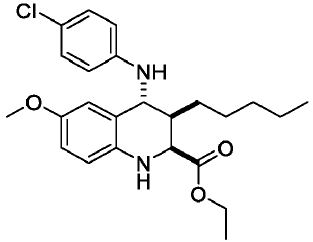

From imine **2** (300.0 mg, 1.45 mmol), freshly distilled heptanal (248.0 mg, 2.17 mmol) and 4-chloroaniline (221.0 mg, 1.74 mmol), and using method **A,** the product was obtained after flash chromatography (silica gel neutralized by Et₃N, 5% EtOAc/cyclohexane) to give **10a** (315.0 mg, 50.5%) and **10b** (31.0 mg, 5%);
**10a :** R*_{f}* = 0.23 (10% EtOAc/cyclohexane); [α]_{D}²⁰ = +80.2 (c = 1.03, CHCl₃); IR vₘₐₓ (thin film, CH₂Cl₂) 3393, 2954, 2924, 1725, 1595, 1501, 1460, 1212, 1150 cm⁻¹ ; UV : 206, 258, 315; ¹H NMR (400 MHz, CDCl₃) *δ* ppm 0.88 (t, *J* = 7.0 Hz, 3H), 1.09 (m, 1H), 1.15-1.35 (m, 7H), 1 .27 (t, *J* = 7.1 Hz, 3H), 2.41 (m, 1H), 3.68 (s, 3H), 4.10 (bs, 1H), 4.14-4.31 (m, 2H), 4.36 (bs, 1H), 6.57-6.61 (m, 3H), 6.70 (d, *J* = 2.8 Hz, 1H), 6.75 (dd, *J* = 8.8, 2.8 Hz, 1H), 7.16 (d, *J* = 8.8 Hz, 2H); ¹³C NMR (100 MHz, CDCl₃) *δ* ppm 14.2 (CH₃), 14.4 (CH₃), 22.7 (CH₂), 26.9 (CH₂), 27.3 (CH₂), 32.2 (CH₂), 37.9 (CH), 53.7 (CH), 54 .4(CH), 55.9 (CH₃), 61.5 (CH₂), 113.8 (2xCH), 115.6 (CH), 116.3 (CH), 116.6 (CH), 119.6 (C), 122.1 (C), 129.4 (2xCH), 136.8 (C), 145.1 (C), 152.3 (C), 173.3 (C); HRMS (ESI-) *m*/*z* calc. for C₂₄H₃₆N₂O₃Cl 429.1950, found 429.1949.

### (2S,3R,4S)-Ethyl-4-((4-chlorophenyl)amino)-6-methoxy-3-pentyl-1,2,3,4-tetrahydroquinoline-2-carboxylate (10b)

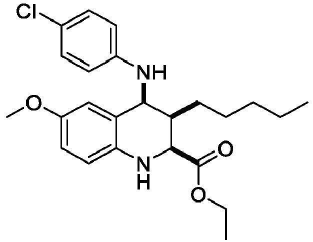

**10b:** R*_{f}* = 0.20 (10% EtOAc/cyclohexane); [α]_{D}²⁰ = +1.02 (c = 0.89, CHCl₃); IR vₘₐₓ (thin film, CH₂Cl₂) 3390, 2954, 2927, 2853, 2087, 1725, 1595, 1507, 1203, 1033 cm⁻¹; UV : 206, 258, 315; ¹H NMR (400 MHz, CDCl₃) *δ* ppm 0.86 (t, *J* = 7.1 Hz, 3H), 0.91 (t, *J* = 6.8 Hz, 3H), 1.24-1.56 (m, 8H), 2.80 (m, 1H), 3.71 (s, 3H), 3.76 (m, 1H), 3.95 (m, 1H), 4.09 (d, *J* = 4.2 Hz, 1H), 4.19 (bs, 1H), 6.45 (d, *J* = 8.8 Hz, 2H), 6.63 (d, *J* = 2.8 Hz, 1H), 6.64 (d, *J* = 8.8 Hz, 1H), 6.78 (dd, *J* = 8.8, 2.8 Hz, 1H), 7.13 (d, *J =* 8.8 Hz, 2H); ¹³C NMR (100 MHz, CDCl₃) *δ* ppm 13.9 (CH₃), 14.3 (CH₃), 22.8 (CH₂), 27.4 (CH₂), 31.2 (CH₂), 32.1 (CH₂), 36.7 (CH), 54.0 (CH), 54.5 (CH), 56.0 (CH₃), 61.3 (CH₂), 113.3 (2xCH), 115.5 (CH), 116.4 (CH), 116.6 (CH), 119.8 (C), 121.8 (C), 129.3 (2xCH), 136.6 (C), 145.1 (C), 152.5 (C), 175.6 (C); HRMS (ESI-) *m*/*z* calc. for C₂₄H₃₀N₂O₃Cl 429.1950, found 429.1945.

### (2S,3S,4R)-ethyl 4-{[(R)-2-acetamido-3-methoxy-3-oxopropyl]thio}-6-methoxy-3-pentyl-1,2,3,4-tetrahydroquinoline-2-carboxylate (11)

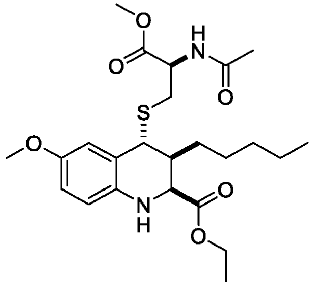

From imine **2** (300.0 mg, 1.45 mmol), freshly distilled heptanal (248.0 mg, 2.17 mmol) and methyl acetyl-*L*-cysteinate (281.0 mg, 1.59 mmol), and using method **A,** the product was obtained after flash chromatography (silica gel, 40% EtOAc/cyclohexane) to give **11** (320.6 mg, 46%); R*_{f}* = 0.29 (50% EtOAc/cyclohexane); [α]_{D}²⁰ = -11.6 (c = 1.19, CHCl₃); IR vₘₐₓ (thin film, CH₂Cl₂) 3385, 2929, 1730, 1656, 1505, 1370, 1216, 1153 cm⁻¹; UV: 205, 246, 330; ¹H NMR (400 MHz, CDCl₃) *δ* ppm 0.85 (t, *J* = 7.0 Hz, 3H), 1.08 (m, 1H), 1.14-1.29 (m, 7H), 1.34 (t, *J* = 7.1 Hz, 3H), 2.07 (s, 3H), 2.31 (m, 1H), 2.94 (dd, *J* = 13.7, 6.3 Hz, 1H), 3.23 (dd, *J* = 13.7, 4.5 Hz, 1H), 3.75 (s, 3H), 3.79 (s, 3H), 3.93 (d, *J* = 1.6 Hz, 1H), 4.22-4.37 (m, 2H), 4.50 (m, 1H), 4.94 (ddd, *J* = 7.6, 6.3, 4.5 Hz, 1H), 6.54 (d, *J* = 8.7 Hz, 1H), 6.69 (dd, *J* = 8.7, 2.8 Hz, 1H), 6.73 (d, *J* = 2.8 Hz, 1H); ¹³C NMR (100 MHz, CDCl₃) *δ* ppm 14.2 (CH₃), 14.5 (CH₃), 22.7 (CH₂), 23.4 (CH₃), 27.1 (CH₂), 27.7 (CH₂), 31.9 (CH₂), 33.8 (CH₂), 39.0 (CH), 46.2 (CH), 52.1 (CH₃), 53.0 (CH), 53.7 (CH), 55.9 (CH₃), 61.6 (CH₂), 115.9 (CH), 116.0 (CH), 116.1 (CH), 118.2 (C), 136.7 (C), 152.0 (C), 170.1 (C), 171.4 (C), 173.1 (C); LRMS (ESI+) *m*/*z* (%) 481 (1) [*M*+H]⁺, 302 (100); HRMS (ESI+) *m*/*z* calc. for C₂₄H₃₇N₂O₆S 481.2367, found 481.2357.

### (2R,3R,4S)-ethyl 4-{[(R)-2-acetamido-3-methoxy-3-oxopropyl]thio}-6-methoxy-3-pentyl-1,2,3,4-tetrahydroquinoline-2-carboxylate (12)

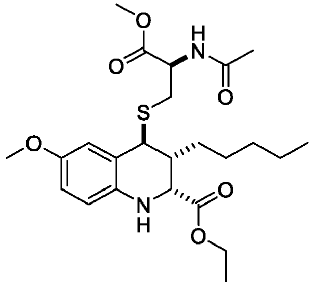

From **2** (300.0 mg, 1.45 mmol), freshly distilled heptanal (248.0 mg, 2.17 mmol) and methyl acetyl-*L*-cysteinate (281.0 mg, 1.59 mmol), and using the method **A** and D-proline as catalyst, the product was obtained after flash chromatography (silica gel, 40% EtOAc/cyclohexane) to give **12** (346.4 mg, 50%); R*_{f}* = 0.27 (50% EtOAc/cyclohexane); [α]_{D}²⁰ = +123.8 (c = 1.00, CHCl₃); IR vₘₐₓ (thin film, CH₂Cl₂) 3303, 2954, 2927, 2863, 1739, 1655, 1503, 1467, 1290, 1230 cm⁻¹; UV: 206, 247, 333; ¹H NMR (400 MHz, CDCl₃) *δ* ppm 0.85 (t, *J* = 7.0 Hz, 3H), 1.06 (m, 1H), 1.13-1.30 (m, 7H), 1.33 (t, *J* = 7.1 Hz, 3H), 2.05 (s, 3H), 2.30 (m, 1H), 3.08 (dd, *J* = 13.9, 4.9 Hz, 1H), 3.14 (dd, *J* = 13.9, 4.7 Hz, 1H), 3.75 (s, 3H), 3.80 (s, 3H), 3.85 (d, *J* = 1.7 Hz, 1H), 4.31-4.36 (m, 2H), 4.51 (d, *J* = 2.9 Hz, 1H), 4.95 (dt, *J* = 7.8, 4.8 Hz, 1H), 6.54 (dd, J = 7.8, 1.2 Hz, 1H), 6.67-6.70 (m, 2H); ¹³C NMR (100 MHz, CDCl₃) *δ* ppm 14.1 (CH₃), 14.5 (CH₃), 22.6 (CH₂), 23.3 (CH₃), 27.1 (CH₂), 27.7 (CH₂), 31.9 (CH₂), 34.1 (CH₂), 39.7 (CH), 47.0 (CH), 52.2 (CH₃), 52.8 (CH), 53.6 (CH), 55.9 (CH₃), 61.6 (CH₂), 115.8 (CH), 116.0 (CH), 116.1 (CH), 118.2 (C), 136.7 (C), 152.0 (C), 170.0 (C), 171.4 (C), 173.0 (C); HRMS (ESI+) *m*/*z* calc. for C₂₄H₃₇N₂O₆S 481.2367, found 481.2361.

### Ethyl-(2S,3S,4R)-4-(((1R,2S,5R)-2-isopropyl-5-methylcyclohexyl)oxy)-6-methoxy-3-pentyl-1,2,3,4-tetrahydroquinoline-2-carboxylate (13)

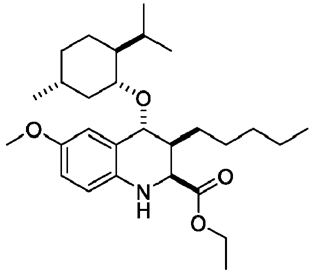

From imine **2** (300.0 mg, 1.45 mmol), freshly distilled heptanal (248.0 mg, 2.17 mmol) and (-)-menthol (295.0 mg, 1.88 mmol), and using method **A,** the product was obtained after flash chromatography (silica gel, 5% EtOAc/cyclohexane) to give **13** (217.0 mg, 32.6%); R*_{f}* = 0.29 (5% EtOAc/cyclohexane); [α]_{D}²⁰ = +24.6 (c = 1.58, CHCl₃); IR vₘₐₓ (thin film, CH₂Cl₂) 3404, 2920, 1733, 1503, 1460, 1366, 1213, 1154, 1040 cm⁻¹; UV :226, 246; ¹H NMR (400 MHz, CDCl₃) *δ* ppm 0.82-0.91 (m, 9H), 0.98 (d, *J* = 6.6 Hz, 3H), 1.02-1.34 (m, 13H), 1.32 (t, *J* = 7.1 Hz, 3H), 1.42 (m, 1H), 1.63-1.72 (m, 2H), 2.26-2.35 (m, 3H), 3.31 (td, *J* = 10.4, 4.1 Hz, 1H), 4.17 (d, *J* = 1.9 Hz, 1H), 4.23 (m, 1H), 4.23-4.30 (m, 2H), 6.60 (m, 1H), 6.71-6.75 (m, 2H); ¹³C NMR (100 MHz, CDCl₃) *δ* ppm 14.2 (CH₃), 14.4 (CH₃), 16.0 (CH₃), 21.6 (CH₃), 22.6 (CH₃, CH₂), 23.1 (CH₂), 25.2 (CH), 25.8 (CH₂), 27.4 (CH₂), 31.9 (CH), 32.0 (CH₂), 34.7 (CH₂), 38.6 (CH), 41.7 (CH₂), 48.2 (CH), 53.7 (CH), 56.0 (CH₃), 61.3 (CH₂), 73.5 (CH), 76.9 (CH), 115.9 (CH), 116.2 (CH), 116.8 (CH), 120.2 (C), 137.3 (C), 152.3 (C), 173.5 (C); HRMS (ESI+) *m*/*z* calc. for C₂₈H₄₆NO₄ 460.3421, found 460.3419.

### Ethyl-(2R,3R,4S)-4-(((1R,2S,5R)-2-isopropyl-5-methylcyclohexyl)oxy)-6-methoxy-3-pentyl-1,2,3,4-tetrahydroquinoline-2-carboxylate (14)

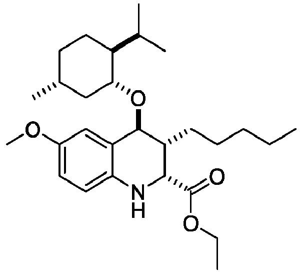

From imine **2** (300.0 mg, 1.45 mmol), freshly distilled heptanal (248.0 mg, 2.17 mmol) and (-)-menthol (295 mg, 1.88 mmol), and using method **A** and D-proline as catalyst, the product was obtained after flash chromatography (silica gel, 5% EtOAc/cyclohexane) to give **14** (232.5 mg, 35%); R*_{f}* = 0.4 (5% EtOAc/cyclohexane); [α]_{D}²⁰ = -108.6 (c = 0.89, CHCl₃); IR vₘₐₓ (thin film, CH₂Cl₂) 3402, 2930, 2089, 1505, 1463, 1250 cm⁻¹; UV: 225, 246; ¹H NMR (400 MHz, CDCl₃) *δ* ppm 0.42 (d, *J* = 6.9 Hz, 3H), 0.80 (d, *J* = 7.1 Hz, 3H), 0.84 (t, *J* = 7.0 Hz, 3H), 0.96 (d, *J* = 6.6 Hz, 3H), 1.01-1.32 (m, 13H), 1.33 (t, *J* = 7.1 Hz, 3H), 1.42 (m, 1H), 1.54-1.68 (m, 2H), 2.10 (m, 1H), 2.25 (m, 1H), 2.33 (m, 1H), 3.32 (td, *J* = 10.5, 4.0 Hz, 1H), 3.74 (s, 3H), 4.20-4.32 (m, 2H), 4.29 (m, 1H), 4.41 (m, 1H), 6.58 (d, *J* = 8.7 Hz, 1H), 6.65 (d, *J* = 2.8 Hz, 1H), 6.74 (dd, *J* = 8.7, 2.8 Hz, 1H); ¹³C NMR (100 MHz, CDCl₃) *δ* ppm 14.2 (CH₃), 14.5 (CH₃), 15.5 (CH₃), 21.3 (CH₃), 22.6 (CH), 22.7 (CH₃), 23.2 (CH₂), 25.4 (CH), 26.1 (CH₂), 27.3 (CH₂), 31.9 (CH), 32.0 (CH₂), 34.8 (CH), 38.8 (CH), 41.6 (CH₂), 48.9 (CH), 53.4 (CH), 56.1 (CH₃), 61.3 (CH₂), 73.8 (CH), 75.5 (CH), 115.7 (CH), 115.9 (CH), 117.0 (CH), 118.8 (C), 137.4 (C), 151.3 (C), 173.9 (C); HRMS (ESI+) *m*/*z* calc. for C₂₈H₄₆NO₄ 460.3421, found 460.3426.

### (2S,3S,4R)-Ethyl 4-hydroxy-6-methoxy-3-pentyl-1,2,3,4-tetrahydroquinoline-2-carboxylate (15)

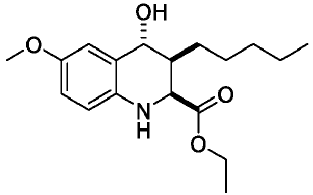

From imine **2** (300.0 mg, 1.45 mmol), freshly distilled heptanal (248.0 mg, 2.17 mmol) and water (34.0 mg, 1.88 mmol), and using method **A,** the product was obtained after flash chromatography (silica gel, 10% to 15% EtOAc/cyclohexane) to give **15** (125.6 mg, 27%); R*_{f}* = 0.33 (20% EtOAc/cyclohexane); [α]_{D}²⁰ = +33.3 (c = 1.00, CHCl₃); IR vₘₐₓ (thin film, CH₂Cl₂) 3395, 2956, 2923, 2851, 1730, 1503, 1461, 1284, 1227, 1153, 1027 cm^{- 1}; UV: 208, 246, 322; ¹H NMR (400 MHz, CDCl₃) *δ* ppm 0.84 (t, *J* = 6.9 Hz, 3H), 0.85-1.31 (m, 8H), 1.32 (t, *J* = 7.1 Hz, 3H), 2.31 (m, 1H), 4.22-4.32 (m, 2H), 4.25 (d, *J* = 3.2 Hz, 1H), 4.52 (d, *J* = 2.4 Hz, 1H), 6.62 (m, 1H), 6.76-6.79 (m, 2H); ¹³C NMR (100 MHz, CDCl₃) *δ* ppm 14.1 (CH₃), 14.4 (CH₃), 22.6 (CH₂), 26.0 (CH₂), 27.2 (CH₂), 31.9 (CH₂), 40.5 (CH), 53.0 (CH), 55.9 (CH₃), 61.4 (CH₂), 69.6 (CH), 115.5 (CH), 116.1 (CH), 116.6 (CH), 121.1 (C), 136.8 (C), 152.1 (C), 173.4 (C); LRMS (ESI+) *m*/*z* (%) 322 (60) [*M*+H]⁺, 304 (100); HRMS (ESI+) *m*/*z* calc. for C₁₈H₂₈NO₄ 322.2013, found 322.2013.

### (2S,3S,4S)-Ethyl-4-azido-3-pentyl-1,2,3,4-tetrahydroquinoline-2-carboxylate (16)

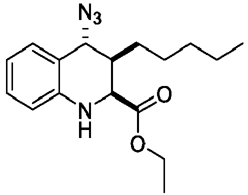

To a solution of aniline (108.0 mg, 1.16 mmol) and ethylglyoxylate (0.28 mL, 1.39 mmol, 50% in toluene) in toluene was added MgSO₄ (87.0 mg, 0.72 mmol) at rt. The reaction mixture was stirred at 110°C for 1 h. After cooling rt, the reaction was filtered off and co-evaporated with toluene two times. The mixture was diluted in dioxane (8 mL) and freshly distilled heptanal (199.0 mg, 1.74 mmol) and L-proline (20.0 mg, 0.17 mmol) were added at rt. The reaction mixture was stirred at this temperature for 48 h. After the reaction was complete TMSN₃ (213.0 mg, 1.51 mmol) and BF₃.Et₂O (54 µL, 0.58 mmol) were added at rt and the reaction was stirred at this temperature for 1h30. The reaction was quenched by NaOH (2 N), extracted with EtOAc, dried by MgSO₄, filtered off and concentrated in vacuum. The product was obtained after flash chromatography (silica gel, 5% EtOAc/cyclohexane) to give 16 (140.0 mg, 38%); R*_{f}* = 0.21 (5% EtOAc/cyclohexane); [α]_{D}²⁰ = + 151.3 (c = 1.10, CHCl₃); IR vₘₐₓ (thin film, CH₂Cl₂) 3427, 2932, 2857, 2093, 1732, 1620, 1496, 1259, 1220 cm⁻¹; UV : 225, 252; ¹H NMR (400 MHz, CDCl₃) *δ* ppm 0.84 (t, *J* = 6.9 Hz, 3H), 1.02 (m, 1H), 1.08-1.29 (m, 7H), 1.33 (t, *J* = 7.1 Hz, 1H), 2.24 (m, 1H), 4.21-4.35 (m, 3H), 4.44 (d, *J* = 2.5 Hz, 1H), 6.66 (d, *J* = 8.1 Hz, 1H), 6.71 (td, *J* = 7.4, 1.1 Hz, 1H), 7.10 (dd, *J* = 7.4, 1 .1 Hz, 1H), 7.16 (ddd, *J* = 8.1, 7.4, 1.5 Hz, 1H); ¹³C NMR (100 MHz, CDCl₃) *δ* ppm 14.1 (CH₃), 14.4 (CH₃), 22.6 (CH₂), 26.4 (CH₂), 27.0 (CH₂), 31.8 (CH₂), 38.2 (CH), 53.2 (CH), 61.2 (CH), 61.7 (CH₂), 114.8 (C), 114.9 (CH), 117.4 (CH), 130.2 (CH), 131.2 (CH), 142.7 (C), 172.5 (C); HRMS (ESI+) *m*/*z* calc. for C₁₇H₂₄O₂N₄Li 323.2054, found 323.2047.

### (2S,3S,4R)-Ethyl-4-azido-6-methoxy-3-methyl-1,2,3,4-tetrahydroquinoline-2-carboxylate (17)

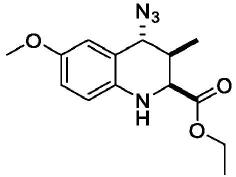

From imine **2** (450.0 mg, 2.17 mmol), recently purchased propioaldehyde (189.0 mg, 3.26 mmol) and TMSN₃ (275.0 mg, 2.82 mmol), and using method **A,** the product was obtained after flash chromatography (silica gel, 4% EtOAc/cyclohexane) to give **17** as an oil (373.0 mg, 59.5%); R*_{f}* = 0.48 (7% EtOAc/cyclohexane); [α]_{D}²⁰ = +126.1 (c = 1.00, CHCl₃); IR vₘₐₓ (thin film, CH₂Cl₂) 3384, 2933, 2090, 1731, 1501, 1463, 1251 cm⁻¹; UV : 205, 249, 326; ¹H NMR (400 MHz, CDCl₃) *δ* ppm 0.76 (d, *J* = 7.0 Hz, 3H), 1.32 (t, *J* = 7.1 Hz, 3H), 2.40 (m, 1H), 3.76 (s, 3H), 4.18 (dd, *J* = 2.9, 2.3 Hz, 1H), 4.24-4.34 (m, 3H), 6.65 (d, *J* = 8.8 Hz, 1H), 6.68 (d, *J* = 2.8 Hz, 1H), 6.81 (dd, *J* = 8.8, 2.9 Hz, 1H); ¹³C NMR (100 MHz, CDCl₃) *δ* ppm 12.0 (CH₃), 14.4 (CH₃), 33.3 (CH), 53.2 (CH), 56.0 (CH₃), 61.7 (CH₂), 63.4 (CH), 115,4 (C), 115.6 (CH), 116.4 (CH), 117.2 (CH), 136.4 (C), 151.9 (C), 172.4 (C); HRMS (ESI+) *m*/*z* calc. for C₁₄H₁₉N₄O₃ 291.1452, found 291.1447.

### (2S,3S,4R)-tert-Butyl-4-azido-6-methoxy-3-methyl-1,2,3,4-tetrahydroquinoline-2-carboxylate (18)

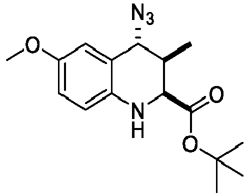

From *tert*-butyl (*E*)-2-((4-methoxyphenyl)imino)acetate¹ (341.0 mg, 1.45 mmol), recently purchased propioaldehyde (126.0 mg, 2.17 mmol) and TMSN₃ (218.0 mg, 1.88 mmol), and using method **A,** the product was obtained after flash chromatography (silica gel, 5% to 10% EtOAc/cyclohexane) to give **18** (221.0 mg, 48%); R*_{f}* = 0.37 (5% EtOAc/cyclohexane); [α]_{D}²⁰ = +145.2 (c = 1.11, CHCl₃); IR vₘₐₓ (thin film, CH₂Cl₂) 3398, 2977, 2090, 1725, 1503, 1366, 1240, 1157, 1040 cm⁻¹; UV : 247; ¹H NMR (400 MHz, CDCl₃) *δ* ppm 0.76 (d, *J* = 7.0 Hz, 3H), 1.51 (s, 9H), 2.35 (m, 1H), 3.75 (s, 3H), 4.06 (d, *J* = 3.1 Hz, 1H), 4.22 (d, *J* = 2.4 Hz, 1H), 6.63 (d, *J* = 8.8 Hz, 1H), 6.67 (d, *J* = 2.8 Hz, 1H), 6.80 (dd, *J* = 8.8, 2.8 Hz, 1H); ¹³C NMR (100 MHz, CDCl₃) *δ* ppm 11.8 (CH₃), 28.2 (3×CH₃), 33.2 (CH), 53.4 (CH), 56.0 (CH₃), 63.6 (CH), 82.3 (C), 115.3 (C), 115.6 (CH), 116.3 (CH), 117.3 (CH), 136.6 (C), 151.8 (C), 171.6 (C); LRMS (ESI+) *m*/*z* (%) 319 (10) [*M*+H]⁺, 220 (100); HRMS (ESI+) *m*/*z* calc. for C₁₆H₂₃N₄O₃ 319.1770, found 319.1764.

### (2S,3S,4R)-Ethyl-4-azido-3-(4-((tert-butyldimethylsilyl)oxy)benzyl)-6-methoxy-1,2,3,4-tetrahydroquinoline-2-carboxylate (19)

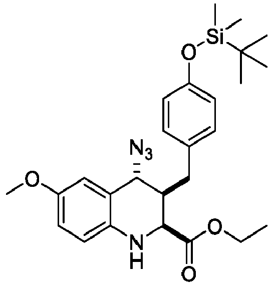

From imine **2** (300.0 mg, 1.45 mmol), 3-(4-((*tert*butyldimethylsilyl)oxy)phenyl)propanal² (574.0 mg, 2.17 mmol) and TMSN₃ (216.0 mg, 1.88 mmol), and using the method **A,** the product was obtained after flash chromatography (silica gel, 5% EtOAc/cyclohexane) to give **19** as a white solid (485.0 mg, 67.5%); R*_{f}* = 0.38 (5% EtOAc/cyclohexane); [α]_{D}²⁰ = +40.5 (c = 1.00, CHCl₃); IR vₘₐₓ (thin film, CH₂Cl₂) 3406, 2956, 2934, 2854, 2094, 1737, 1605, 1509, 1466, 1257, 1229 cm⁻¹; UV : 205, 249, 326; ¹H NMR (400 MHz, CDCl₃) *δ* ppm 0.19 (s, 6H), 0.98 (s, 9H), 1.30 (t, *J* = 7.1 Hz, 3H), 2.18 (dd, *J* = 14.0, 10.2 Hz, 1H), 2.47 (dd, *J* = 14.0, 4.8 Hz, 1H), 2.52 (m, 1H), 3.75 (s, 3H), 4.14-4.21 (m, 2H), 4.22 (d, *J* = 2.3 Hz, 1H), 4.25 (dd, *J* = 2.8, 1.9 Hz, 1H), 6.63 (d, *J* = 2.8 Hz, 1H), 6.68 (d, *J* = 8.8 Hz, 1H), 6.74 (d, *J* = 8.5 Hz, 2H), 6.84 (dd, *J* = 8.8, 2.8 Hz, 1H), 6.88 (d, *J* = 8.5 Hz, 2H); ¹³C NMR (100 MHz, CDCl₃) *δ* ppm 14.4 (CH₃), 18.3 (C), 25.8 (3×CH₃), 32.0 (CH₂), 40.3 (CH), 53.1 (CH), 55.9 (CH₃), 60.4 (CH), 61.6 (CH₂), 115.4 (C), 115.5 (CH), 116.3 (CH), 117.2 (CH), 120.1 (2xCH), 130.2 (2xCH), 131.3 (C), 136.7 (C), 151.9 (C), 154.4 (C), 172.1 (C); LRMS (ESI+) *m*/*z* (%) 497 (100) [*M*+H]⁺, 454 (35) ; HRMS (ESI+) *m*/*z* calc. for C₂₆H₃₇N₄O₄Si 497.2579, found 497.2581.

### (2S,3S,4R)-Ethyl-3-allyl-4-azido-6-methoxy-1,2,3,4-tetrahydroquinoline-2-carboxylate (20)

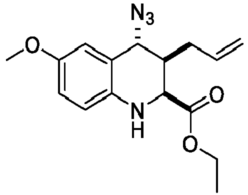

From imine **2** (300.0 mg, 1.45 mmol), recently purchased 4-pentenal (187.0 mg, 2.17 mmol) and TMSN₃ (217.0 mg, 1.88 mmol), and using method **A,** the product was obtained after flash chromatography (silica gel, 5% EtOAc/cyclohexane) to give **20** (266.0 mg, 58%); R*_{f}* = 0.29 (5% EtOAc/cyclohexane); [α]_{D}²⁰ = +188.6 (c = 1.00, CHCl₃); IR vₘₐₓ (thin film, CH₂Cl₂) 3405, 2936, 2091, 1729, 1503, 1226, 1153, 1035 cm⁻¹; UV: 226, 245; ¹H NMR (400 MHz, CDCl₃) *δ* ppm 1.31 (t, *J* = 7.1 Hz, 3H), 1.76 (m, 1H), 1.97 (m, 1H), 2.34 (m, 1H), 3.74 (s, 3H), 4.20 (m, 1H), 4.20-4.31 (m, 2H), 4.40 (d, *J* = 2.3 Hz, 1H), 4.92 (dd, *J* = 17.0, 1.4 Hz, 1H), 5.00 (d, *J* = 10.1 Hz, 1H), 5.70 (m, 1H), 6.64 (d, *J* = 8.8 Hz, 1H), 6.67 (d, *J* = 2.8 Hz, 1H), 6.81 (dd, *J* = 8.8, 2.8 Hz, 1H); ¹³C NMR (100 MHz, CDCl₃) *δ* ppm 14.3 (CH₃), 31.0 (CH₂), 38.0 (CH), 52.9 (CH), 55.9 (CH₃), 60.8 (CH), 61.5 (CH₂), 115.3 (C), 115.4 (CH), 116.2 (CH), 117.1 (CH), 117.7 (CH₂), 135.2 (CH), 137.0 (C), 151.9 (C), 172.0 (C); LRMS (ESI+) *m*/*z*(%) 317 (12) [*M*+H]⁺, 274 (100); HRMS (ESI+) *m*/*z* calc. for C₁₆H₂₁N₄O₃ 317.1608, found 317.1600.

### (2S,3S,4R)-Ethyl-3-allyl-4-(allyloxy)-6-methoxy-1,2,3,4-tetrahydroquinoline-2-carboxylate (21)

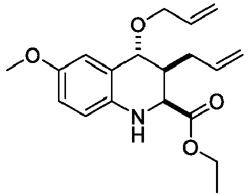

From imine 2 (300.0 mg, 1.45 mmol), recently purchased 2-pentenal (187.0 mg, 2.17 mmol) and 2-propen-1-ol (109.0 mg, 1.88 mmol), and using method **A,** the product was obtained after flash chromatography (silica gel, 5% EtOAc/cyclohexane) to give **21** (182.8 mg, 38%); R*_{f}* = 0.21 (5% EtOAc/cyclohexane); [α]_{D}²⁰ = +104.7 (c = 1.02, CHCl₃); IR vₘₐₓ (thin film, CH₂Cl₂) 3408, 2936, 1730, 1638, 1505, 1495, 1210, 1153 cm⁻¹; UV: 245; ¹H NMR (400 MHz, CDCl₃) *δ* ppm 1.32 (t, *J* = 7.1 Hz, 3H), 1.65 (m, 1H), 1.91 (m, 1H), 2.56 (m, 1H), 3.74 (s, 3H), 4.10 (m, 1H), 4.11 (m, 1H), 4.15 (d, *J* = 2.6 Hz, 1H), 4.19-4.32 (m, 2H), 4.33 (dd, *J* = 3.1, 2.1 Hz, 1H), 4.90 (ddd, *J* = 17.0, 3.2, 1.6 Hz, 1H), 4.96 (m, 1H), 5.19 (ddd, *J* = 10.4, 3.0, 1.3 Hz, 1H), 5.30 (ddd, *J* = 17.0, 1.6, 1.6 Hz, 1H), 5.72 (dddd, *J* = 17.0, 10.4, 7.8, 6.4 Hz, 1H), 5.95 (dddd, *J* = 17.0, 10.4, 5.6, 5.6 Hz, 1H), 6.61 (d, *J* = 8.7 Hz, 1H), 6.68 (d, *J =* 2.9 Hz, 1H), 6.77 (dd, *J* = 8.7, 2.9 Hz, 1H); ¹³C NMR (100 MHz, CDCl₃) *δ* ppm 14.3 (CH₃), 30.6 (CH₂), 37.4 (CH), 53.0 (CH), 56.0 (CH₃), 61.3 (CH₂), 69.0 (CH₂), 75.4 (CH), 115.8 (CH), 116.3 (CH), 116.8 (CH), 117.0 (CH), 117.1 (CH), 118.0 (C), 135.4 (CH), 135.9 (CH), 137.1 (C), 151.6 (C), 173.0 (C); LRMS (ESI+) *m*/*z* (%) 332 (24) [*M*+H]⁺, 274 (100); HRMS (ESI+) *m*/*z* calc. for C₁₉H₂₆NO₄ 332.1856, found 332.1858.

### (2S,3S,4R)-Ethyl-3-allyl-6-methoxy-4-(prop-2-yn-1-yloxy)-1,2,3,4-tetrahydroquinoline-2-carboxylate (22)

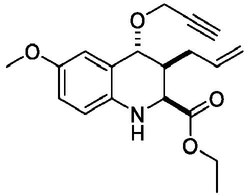

From imine **2** (300.0 mg, 1.45 mmol), recently purchased 2-pentenal (187.0 mg, 2.17 mmol) and propargyl alcohol (106.0 mg, 1.88 mmol), and using method **A,** the product was obtained after flash chromatography (silica gel, 5% EtOAc/cyclohexane) to give **22** (191.0 mg, 40%); R*_{f}* = 0.21 (5% EtOAc/cyclohexane); [α]_{D}²⁰ = +162.2 (c = 1.06, CHCl₃); IR vₘₐₓ (thin film, CH₂Cl₂) 3284, 2942, 1729, 1505, 1229, 1207, 1155 cm⁻¹; UV: 248, 324, 322; ¹H NMR (400 MHz, CDCl₃) *δ* ppm 1.32 (t, *J* = 7.1 Hz, 3H), 1.68 (m, 1H), 1.91 (m, 1H), 2.50 (t, *J* = 2.4 Hz, 1H), 2.52 (m, 1H), 3.76 (s, 3H), 4.18 (dd, *J* = 16.0, 2.4 Hz, 1H), 4.18-4.30 (m, 4H), 4.42 (d, *J* = 2.6 Hz, 1H), 4.91 (ddd, *J* = 17.0, 3.2, 1.5 Hz, 1H), 4.98 (m, 1H), 5.73 (m, 1H), 6.63 (d, *J* = 8.1 Hz, 1H), 6.77-6.81 (m, 2H); ¹³C NMR (100 MHz, CDCl₃) *δ* ppm 14.4 (CH₃), 30.7 (CH₂), 37.3 (CH), 52.9 (CH), 54.9 (CH₂), 56.1 (CH3), 61.4 (CH₂), 74.5 (CH), 74.8 (C), 80.4 (CH), 116.0 (CH), 116.7 (C), 116.8 (C), 117.2 (CH), 135.7 (CH), 137.2 (C), 151.7 (C), 173.0 (C); LRMS (ESI+) m/z(%) 329 (45), 274 (100); HRMS (ESI+) *m*/*z* calc. for C₁₉H₂₄NO₄ 330.1700, found 330.1704.

### (2S,3S,4R)-Ethyl-4-((3-mercaptopropyl)thio)-6-methoxy-3-pentyl-1,2,3,4-tetrahydroquinoline-2-carboxylate (23)

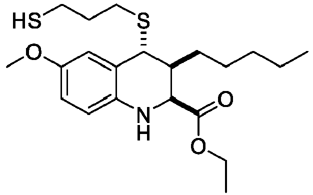

Chemical Formula: C₂₁H₃₃NO₃S₂
Molecular Weight: 411.6216

From imine **2** (300.0 mg, 1.45 mmol), freshly distilled heptanal (248.0 mg, 2.17 mmol) and 1,3 propanedithiol (235.0 mg, 2.17 mmol), and using method **A,** the product was obtained after flash chromatography (silica gel, 5% EtOAc/cyclohexane) to give **23** (208.5 mg, 35%); R*_{f}* = 0.32 (10% EtOAc/cyclohexane); [α]_{D}²⁰ = + 51.9 (c = 1.03, CHCl₃); IR vₘₐₓ (thin film, CH₂Cl₂) 3398, 2955, 2923, 2850, 1732, 1503, 1467, 1213, 1150, 1039 cm⁻¹; UV: 205, 245, 325; ¹H NMR (400 MHz, CDCl₃) *δ* ppm 0.85 (t, *J* = 6.9 Hz, 3H), 1.10 (m, 1H), 1.15-1.39 (m, 7H), 1.33 (t, *J* = 7.1 Hz, 3H), 1.96 (m, 2H), 2.34 (m, 1H), 2.64-2.85 (m, 4H), 3.74 (s, 3H), 3.89 (d, *J* = 1.7 Hz, 1H), 4.21-4.36 (m, 2H), 4.57 (d, *J* = 2.8 Hz, 1H), 6.55 (d, *J* = 8.0 Hz, 1H), 6.68 (dd, *J* = 8.0, 2.8 Hz, 1H), 6.69 (d, *J* = 2.8 Hz, 1H); ¹³C NMR (100 MHz, CDCl₃) *δ* ppm 14.1 (CH₃), 14.5 (CH₃), 22.6 (CH₂), 23.6 (CH₂), 27.7 (CH₂), 30.1 (CH₂), 31.9 (CH₂), 33.6 (CH₂), 39.0 (CH), 46.2 (CH), 53.7 (CH), 55.9 (CH₃), 61.5 (CH₂), 115.5 (CH), 116.0 (CH), 116.1 (CH), 118.9 (C), 136.7 (C), 152.0 (C), 173.2 (C); LRMS (ESI+) *m*/*z* (%) 412 (5) [*M*+H]⁺, 304 (100); HRMS (ESI+) *m*/*z* calc. for C₂₁H₃₄NO₃S₂ 412.1974, found 412.1966.

### Methyl-(2S,3R,4S)-4-azido-2-ethyl-3-methyl-1,2,3,4-tetrahydroquinoline-6-carboxylate (26)

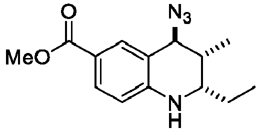

To a solution of methyl-4-aminobenzoate (1.02 g, 6.75 mmol) and propionaldehyde (1.95 g, 33.1 mmol) at -20°C, D-proline (228.5 mg, 1.985 mmol) was added at -20°C. The reaction mixture was stirred for 20 min at -20°C and kept at the same temperature for two days. Dioxane was added (50 mL) and TMSN₃ (1.3 mL, 9.92 mmol) and then BF₃.Et₂O (0.6 mL, 4.72 mmol) were added at rt and the reaction mixture was stirred at this temperature for 25 min. The reaction was quenched by NaOH (1 N), extracted with EtOAc, dried by MgSO₄, filtered off and concentrated *in vacuo.* After flash chromatography (silica gel, 10% EtOAc/ cyclohexane), the product **26** was isolated (1.52 g, 82%) as a yellow solid. The product is recrystallized in CH₂Cl₂/heptane to obtain 814 mg (44%) with an enantiomeric excess > 99%; R*_{f}* = 0.27 (10% EtOAc in cyclohexane); [α]_{D}²⁰ = - 128.3 (c = 0.93, CHCl₃); IR vₘₐₓ (thin film, CH₂Cl₂): 3427, 3053, 2969, 2093, 1704, 1613, 1436, 1265 cm⁻¹; ¹H NMR (400 MHz, CDCl₃) *δ* ppm 0.74 (d, *J* = 7.1 Hz, 3H), 1.03 (t, *J* = 7.5 Hz, 3H), 1.53-1.62 (m, 2H), 1.97 (m, 1H), 3.42 (m, 1H), 3.85 (s, 3H), 4.27 (d, *J* = 2.7 Hz, 1H), 6.52 (d, *J* = 8.5 Hz, 1H), 7.80 (dd, *J* = 8.5, 2.0 Hz, 1H), 7.83 (d, *J* = 2.0 Hz, 1H); ¹³C NMR (100 MHz, CDCl₃) *δ* ppm 10.4 (CH₃), 10.5 (CH₃), 25.3 (CH₂), 33.2 (CH), 51.1 (CH₃), 51.8 (CH), 63.8 (CH), 113.5 (CH), 114.5 (C), 118.1 (C), 131.8 (CH), 133.6 (CH), 148.1 (C), 167.2 (C); LMRS (ESI+) *m*/*z* (%) 297 (100) [*M*+Na]⁺; HRMS (ESI+) *m*/*z* calc. for C₁₄H₁₈N₄O₂Li [*M*+Li]⁺ 281.1584, found 281.1585.

### Methyl-(2S,3R,4S)-1-acetyl-4-azido-2-ethyl-3-methyl-1,2,3,4-tetrahydroquinoline-6-carboxylate (27)

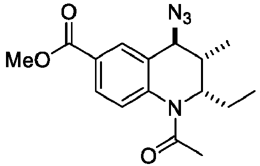

To a stirred solution of tetrahydroquinoline **26** (437.0 mg, 1.59 mmol) in dry CH₂Cl₂ under argon atmosphere was added dry pyridine (0.39 mL, 4.78 mmol). Acetyl chloride (0.23 mL, 3.18 mmol) was added dropwise at 0°C. The mixture was stirred at room temperature overnight. The same procedure was repeated (dry pyridine (0.39 mL, 4.78 mmol) and acetyl chloride (0.23 mL, 3.18 mmol) added at 0°C) and the mixture was stirred overnight at room temperature until completion. The solvent was removed and EtOAc was added. The organic layer was washed with brine, dried over MgSO₄ and the solvent was removed in vacuum. The product was obtained after flash chromatography (silica gel, 20% EtOAc/cyclohexane) to give **27** (479.9 mg, 95%); R*_{f}* = 0.29 (20% EtOAc/cyclohexane); [α]_{D}²⁰ = + 307.8 (c = 0.96, CHCl₃); IR vₘₐₓ (thin film, CH₂Cl₂): 1461, 2967, 2936, 2877, 2095, 1710, 1609, 1497, 1125 cm⁻¹; ¹H NMR (400 MHz, CDCl₃) *δ* ppm 0.85 (t, *J* = 7.2 Hz, 3H), 1.07 (m, 1H), 1.24 (d, *J* = 6.9 Hz, 3H), 1.61 (m, 1H), 2.30 (s, 3H), 2.31 (m, 1H), 3.94 (s, 3H), 4.10 (d, *J* = 10.3 Hz, 1H), 4.68 (m, 1H), 7.35 (d, *J* = 8.5 Hz, 1H), 7.97 (dd, *J* = 8.5, 2.0 Hz, 1H), 8.14 (d, *J* = 2.0 Hz, 1H); ¹³C NMR (100 MHz, CDCl₃) *δ* ppm 10.7 (CH₃), 16.8 (CH₃), 18.5 (CH₂), 23.8 (CH₃), 39.1 (CH), 52.5 (CH₃), 59.0 (CH), 63.7 (CH), 125.5 (CH), 127.3 (C), 128.2 (C), 129.8 (CH), 130.8 (CH), 141.0 (C), 166.2 (C), 170.1 (C); LRMS (ESI+) *m*/*z* (%) 317 (30) [*M*+H]⁺, 232 (100); HRMS (ESI+) *m*/*z* calc. for. C₁₆H₂₁N₄O₃ 317.1614, found 317.1608.

### Methyl-(-acetyl-2-ethyl-3-methyl-4-(4-phenyl-1H-1,2,3-triazol-1-yl)-1,2,3,4-tetrahydroquinoline-6-carboxylate (28)

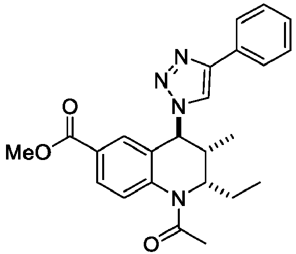

### Method B

To a solution of **6-a** (300.0 mg, 0.67 mmol) and phenylacetylene (138.0 mg, 1.35 mmol) in CH₂Cl₂ (1 mL) was added Amberlyst Cul.Et₃N^{[3]} (40.0 mg) at rt. The reaction mixture was stirred at this temperature for 24 h. After completion, the reaction was filtered off, the supported catalyst was washed with CH₂Cl₂ (2 mL), and the solvent was concentrated under vacuum. After flash chromatography (silica gel, 25% EtOAc/ cyclohexane), the product **28** was isolated (318.3 mg, 86%) as a white solid; R*_{f}* = 0.12 (30% EtOAc/cyclohexane); [α]_{D}²⁰ = +189.9 (c = 1.00, CHCl₃); IR vₘₐₓ (thin film, CH₂Cl₂): 3478, 3128, 2966, 2877, 2094, 1719, 1664, 1260 cm⁻¹; ¹H NMR (400 MHz, CDCl₃) *δ* ppm 0.95 (t, *J* = 7.2 Hz, 3H), 1.09 (d, *J* = 6.7 Hz, 2H), 1.20-1.33 (m, 2H), 1.76 (m, 1H), 2.41 (s, 3H), 2.65 (m, 1H), 3.81 (s, 3H), 5.66 (d, *J* = 11.2 Hz, 1H), 7.33 (t, *J* = 7.4 Hz, 1H), 7.41 (t, *J* = 7.4 Hz, 2H), 7.54 (bs, 1H), 7.64 (d, *J* = 1.9 Hz, 1H), 7.67 (s, 1H), 7.83 (d, *J* = 7.4 Hz, 2H), 7.99 (dd, *J* = 8.6, 1.9 Hz, 1H); ¹³C NMR (100 MHz, CDCl₃) *δ* ppm 10.8 (CH₃), 16.1 (CH₃), 18.5 (CH₂), 24.1 (CH₃), 40.6 (CH), 52.4 (CH₃), 60.6 (CH), 63.1 (CH), 117.9 (CH), 125.9 (2xCH), 126.9 (CH), 127.6 (2xC), 128.6 (CH), 129.1 (2xCH), 130.1 (CH), 130.3 (CH), 130.4 (C), 140.5 (C), 149.0 (C), 165.9 (C), 170.1 (C); LRMS (ESI+) *m*/*z* (%) 441 (100) [*M*+Na]⁺, 419 (10) [M+H]⁺; HRMS (ESI+) *m*/*z* calc. for C₂₄H₂₆N₄O₃Na 441.1903, found 441.1901.

### Methyl-(2S,3S,4R)-4-azido-2-ethyl-3-methyl-1,2,3,4-tetrahydroquinoline-6-carboxylate (29)

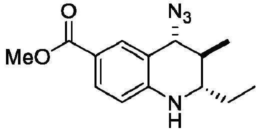

A solution of methyl-4-benzoate (100.0 mg, 0.66 mmol), (S)-(-)-α,α-diphenyl-2-pyrrolidine methanol trimethylsilyl ether (44.0 mg, 0.13 mmol) and 4-nitrophenyl acetic acid (24 mg, 0.13 mmol) in MTBE (1.5 mL) was stirred for 10 min at -10°C. Then propionaldehyde (0.65 mL, 8.61 mmol) was added and stirred for 48 h at -10°C. After the reaction was complete, TMSN₃ (0.13 mL, 0.99 mmol) and BF₃.Et₂O (180 µL, 1.39 mmol) were added at -10°C and the mixture was stirred at rt for 4 h. The reaction was quenched with NaOH (2 N) until neutralization, extracted with EtOAc, dried by MgSO₄, filtered off and concentrated by vacuum. After flash chromatography (silica gel, 5% EtOAc/cyclohexane), the product **29** was isolated (106.4 mg, 58.6%); R*_{f}*= 0.35 (10% EtOAc/cyclohexane); ¹H NMR (400 MHz, CDCl₃) δ ppm 0.98 (t, *J* = 7.5 Hz, 3H), 1.08 (d, *J* = 6.7 Hz, 3H), 1.53 (m, 1H), 1.72 (m, 1H), 1.84 (m, 1H), 3.24 (ddd, *J =* 10.3, 7.0, 3.3 Hz, 1H), 3.85 (s, 3H), 4.39 (d, *J* = 3.1 Hz, 1H), 6.52 (d, *J* = 9.1 Hz, 1H), 7.79 (d, *J =* 2.0 Hz, 1H), 7.80 (dd, *J* = 9.1, 2.0 Hz, 1H); ¹³C NMR (100 MHz, CDCl₃) *δ* ppm 8.6 (CH₃), 14.3 (CH₃), 25.7 (CH₂), 34.5 (CH), 51.8 (CH₃), 52.9 (CH), 63.7 (CH), 113.5 (CH), 116.7 (C), 117.7 (C), 131.9 (CH), 132.0 (CH), 148.3 (C), 167.2 (C); HRMS (ESI+) *m*/*z* calc. for C₁₄H₁₈N₄O₂Li [*M*+Li]⁺ 281.1584, found 281.1585.

### Methyl-(2S,3S,4R)-1-acetyl-4-azido-2-ethyl-3-methyl-1,2,3,4-tetrahydroquinoline-6-carboxylate (30)

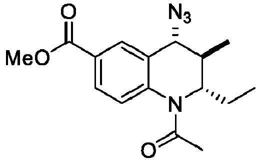

To a stirred solution of tetrahydroquinoline **29** (85.8 mg, 0.31 mmol) in dry CH₂Cl₂ (1.6 mL) under argon atmosphere was added dry pyridine (76 µL, 0.94 mmol). Acetyl chloride (45 µL, 0.63 mmol) was added dropwise at 0°C. The mixture was stirred at room temperature overnight. The same procedure was repeated (dry pyridine (76 µL, 0.94 mmol) and acetyl chloride (45 µL, 0.63 mmol) added at 0°C) and the mixture was stirred for 8 h at room temperature until completion. The solvent was removed and EtOAc was added. The organic layer was washed with brine, dried over MgSO₄ and the solvent was removed in vacuum. The product was obtained after flash chromatography (silica gel, 10% EtOAc/cyclohexane) to give **30** (65.2 mg, 65.8%); R*_{f}* = 0.20 (10% EtOAc/cyclohexane); ¹H NMR (400 MHz, CDCl₃) *δ* ppm 0.80 (t, *J =* 7.4 Hz, 3H), 1.24 (d, *J =* 6.7 Hz, 3H), 1.38 (m, 1H), 1.75 (m, 1H), 1.83 (m, 1 H), 2.16 (s, 3H), 3.95 (s, 3H), 4.31 (m, 1H), 4.48 (d, *J* = 3.3 Hz, 1H), 7.30 (d, *J =* 8.3 Hz, 1H), 7.99 (d, *J =* 2.0 Hz, 1H), 8.07 (dd, *J =* 8.3, 2.0 Hz, 1H); ¹³C NMR (100 MHz, CDCl₃) *δ* ppm 9.7 (CH₃), 17.2 (CH₃), 23.2 (CH₃), 27.2 (CH₂), 40.7 (CH), 52.6 (CH₃), 57.4 (CH), 63.8 (CH), 126.7 (CH), 127.8 (C), 129.1 (CH), 131.0 (CH), 132.2 (C), 142.9 (C), 166.2 (C), 170.2 (C); LRMS (ESI+) *m*/*z* (%) 339 (25) [*M*+Na]⁺, 317 (100) [*M*+H]⁺; HRMS (ESI+) *m*/*z* calc. for C₁₆H₂₁O₃N₄ [*M*+H]⁺ 317.1608, found 317.1606.

### Methyl-(25,35,4R)-1-acetyl-2-ethyl-3-methyl-4-(4-phenyl-1H-1,2,3-triazol-1-yl)-1,2,3,4-tetrahydroquinoline-6-carboxylate (31)

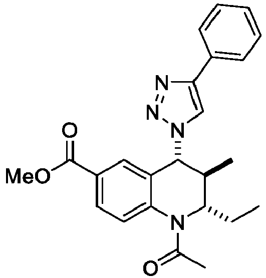

To a solution of acetyled product **30** (27.5 mg, 0.087 mmol) and phenylacetylene (18 µL, 0.174 mmol) in dry DCM (0.1 mL) was added Cul.Et₃N (9 mg) according to Method B. The mixture was stirred at RT overnight. Cul.Et₃N (6.8 mg) and phenylacetylene were added (18 µL, 0.174) again, and the reaction was stirring at RT overnight until completion. The reaction was filtered off and washed by DCM and concentrated in vacuo. After flash chromatography (silica gel, 20% EtOAc/cyclohexane), the product was isolated (16.4 mg, 45%).

R*_{f}*= 0.23 (20% EtOAc/cyclohexane);¹H NMR (400 MHz, CDCl₃) δ ppm 0.90 (t, *J =* 7.2 Hz, 3H), 0.91 (d, *J =* 7.0 Hz, 3H), 1.53 (m, 1H), 1.72 (m, 1H), 2.25 (s, 3H), 2.47 (m, 1H), 3.81 (s, 3H), 4.39 (m, 1H), 5.94 (d, *J =* 6.0 Hz, 1H), 7.25 (t, *J =* 7.4 Hz, 1H), 7.34 (t, *J =* 7.4 Hz, 2H), 7.47 (m, 1H), 7.51 (s, 1H), 7.72 (d, *J =* 7.4 Hz, 2H), 7.82 (d, *J =* 2.0 Hz, 1H), 7.99 (dd, *J =* 8.5, 2.0 Hz, 1H); LRMS (ESI+) *m*/*z* (%) 441 (35) [*M*+Na]⁺, 419 (100) [*M*+H]⁺; HRMS (ESI+) *m*/*z* calc. for C₂₄H₂₇O₃N₄ [*M*+H]⁺ 419.2078, found 419.2069.

### Methyl-(2S,3S,4R)-4-(4-(2,5,8,11-tetraoxatetradec-13-yn-1-yl)-1H-1,2,3-triazol-1-yl)-1-acetyl-2-ethyl-3-methyl-1,2,3,4-tetrahydroquinoline-6-carboxylate (32)

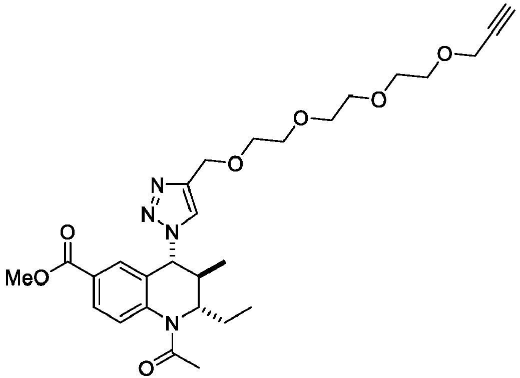

Using the same protocol as **31** and starting from **30** (20.5 mg, 0.06 mmol) and dialkyne-PEG (44 mg, 0.19 mmol), and after flash chromatography (silica gel, EtOAc 100%), the compound **32** was obtained (16.2 mg, 46%):
R*_{f}*= 0.34 (2% MeOH/CH₂Cl₂); ¹H NMR (400 MHz, CDCl₃) *δ* ppm 0.92 (d, *J =* 6.9 Hz, 3H), 0.93 (t, *J =* 7.4 Hz, 3H), 1.56 (m, 1H), 1.79 (m, 1H), 2.29 (s, 3H), 2.42 (t, *J =* 2.4 Hz, 1H), 2.45 (m, 1H), 3.61-3.72 (m, 12H), 3.88 (s, 3H), 4.19 (d, *J =* 2.4 Hz, 2H), 4.43 (m, 1H), 4.67 (s, 2H), 5.91 (d, *J* = 5.9 Hz, 1H), 7.30 (s, 1H), 7.48 (m, 1H), 7.82 (s, 1H), 8.04 (dd, *J* = 8.5, 2.0 Hz, 1H); ¹³C NMR (100 MHz, CDCl₃) δ ppm 10.2 (CH₃), 16.0 (CH₃), 23.5 (CH₃), 25.9 (CH₂), 38.0 (CH), 52.5 (CH₃), 58.6 (CH₂), 59.8 (CH), 61.0 (CH), 64.9 (CH₂), 70.0 (CH₂), 70.6 (CH₂), 70.7 (CH₂), 70.7 (CH₂), 70.8 (CH₂), 70.8 (CH₂), 74.7 (CH), 79.9 (C), 122.9 (CH), 126.4 (CH), 127.6 (2xC), 130.4 (CH), 130.7 (CH), 142.1 (C), 145.4 (C), 166.0 (C), 170.7 (C); LRMS (ESI+) *m*/*z*(%) 441 (35) [*M*+Na]⁺, 419 (100) [*M*+H]⁺; HRMS (ESI+) *m*/*z* calc. for C₂₈H₃₉O₇N₄ [*M*+H]⁺ 543.2813, found 543.2793.

### N-(4'-amino-[1,1'-biphenyl]-4-yl)hex-5-enamide (XA)

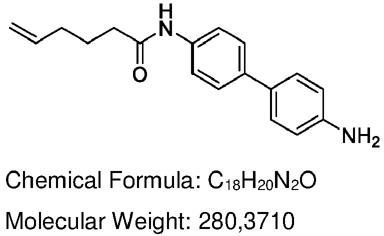

A solution of 5-hexenoic acid (1.3 mL, 7.246 mmol), DMAP (133.0 mg, 1.09 mmol), and benzidine (2.0 g, 10.87 mmol) in DCM (70 mL) was cooled at 0°C. DCC (2.24 g, 10.87 mmol) was solubilized in CH₂Cl₂ (70 mL) and this solution was added dropwise to the previous mixture. The reaction mixture was stirred overnight at rt. The mixture was filtered on and washed with HCI (1 N) then with NaHCO₃ until pH = 8. Organic layer was washed again with brine, dried over MgSO₄, filtered on and concentrated under vacuum. The product was purified by flash chromatography (30% cyclohexane in 70% mixture of 2% EtOAc in CH₂Cl₂) to yield the product **XA** (1.32 g, 65%) as a white solid. R*_{f}*= 0.20 (2% EtOAc/CH₂Cl₂); ¹H NMR (400 MHz, CDCl₃) δ ppm 1.84 (m, 2H), 2.15 (m, 2H), 2.36 (t, *J =* 7.5 Hz, 2H), 4.98-5.08 (m, 2H), 5.80 (m, 1H), 6.73 (d, *J =* 8.5 Hz, 2H), 7.37 (d, *J =* 8.5 Hz, 2H), 7.47 (d, *J =* 8.7 Hz, 2H), 7.53 (d, *J =* 8.7 Hz, 2H); ¹³C NMR (100 MHz, CDCl₃) *δ* ppm 24.8 (CH₂), 33.3 (CH₂), 37.0 (CH₂), 115.6 (2xCH), 115.7 (CH₂), 120.4 (2xCH), 126.9 (2xCH), 127.9 (2xCH), 131.1 (C), 136.5 (C), 137.4 (C), 138.0 (CH), 145.9 (C), 171.3 (C); LRMS (ESI+) *m*/*z* (%) 281 (100) [*M*+H]⁺; HRMS (ESI+): *m*/*z* calc. for C₁₈H₂₁ON₂ 281.1648, found 281.1646.

### N-(4-((2S,3R,4S)-4-azido-2-ethyl-3-methyl-1,2,3,4-tetrahydroquinolin-6-yl)phenyl)hex-5-enamide (XB)

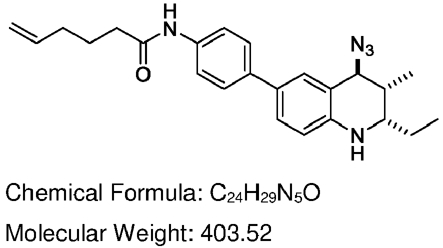

A solution of N-(4'-amino-[1,1'-biphenyl]-4-yl)hex-5-enamide **XA** (213.0 mg, 0.761 mmol) and D-proline (26.0 mg, 0.226 mmol) in CH₃CN (20 mL) was cooled at -40°C. Propionaldehyde (600.0 mg, 10.33 mmol) was added at -40°C. The reaction mixture was stirred at -40°C for 2 h then at 0°C overnight. TMSN₃ (114.0 mg, 0.989 mmol) and then BF₃.Et₂O (0.40 mL, 3.241 mmol) were added at rt. The reaction mixture is stirred at rt for 1h30. The reaction is quenched with NaOH (1 N), concentrated and dissolved in EtOAc. The layer was dried over MgSO₄, filtered on and concentrated under vacuum. The product was purified by flash chromatography (silica gel, 10% to 20% EtOAc/cyclohexane) to obtain XB (194, 1 mg, 63%); R*_{f}* = 0.37 (20% EtOAc/cyclohexane); ¹H NMR (400 MHz, CDCl₃) δ ppm 0.79 (d, *J =* 7.1 Hz, 3H), 1.02 (t, *J =* 7.4 Hz, 3H), 1.55 (m, 2H), 1.84 (m, 2H), 1.95 (m, 1H), 2.14 (m, 2H), 2.36 (t, *J =* 7.5 Hz, 2H), 3.35 (td, *J =* 7.1, 2.6 Hz, 1H), 4.28 (d, *J =* 2.6 Hz, 1H), 4.96-5.08 (m, 2H), 5.80 (ddt, *J* = 16.9, 10.2, 6.7 Hz, 1H), 6.60 (d, *J* = 8.4 Hz, 1H), 7.29 (d, *J =* 2.0 Hz, 1H), 7.34 (dd, *J =* 8.4, 2.0 Hz, 1H), 7.46 (d, *J =* 8.5 Hz, 2H), 7.53 (d, *J =* 8.5 Hz, 2H); ¹³C NMR (100 MHz, CDCl₃) *δ* ppm 10.5 (CH₃), 10.6 (CH₃), 24.8 (CH₂), 25.5 (CH₂), 33.3 (CH₂), 33.9 (CH), 37.0 (CH₂), 51.1 (CH), 64.4 (CH), 114.9 (CH), 115.6 (CH₂), 115.9 (C), 120.4 (2xCH), 126.8 (2xCH), 128.3 (CH), 129.3 (C), 129.4 (CH), 136.4 (C), 137.1 (C), 138.0 (CH), 143.6 (C), 171.4 (C); LRMS (ESI+) *m*/*z* (%) 361 (100) 404 (10) [*M*+H]⁺; HRMS (ESI+): *m*/*z* calc. for C₂₄H₃₀N₅O 404.2450, found 404.2439.

### N-(4-((2S,3R,4S)-1-acetyl-4-azido-2-ethyl-3-methyl-1,2,3,4-tetrahydroquinolin-6-yl)phel)hex-5-enamide (XC)

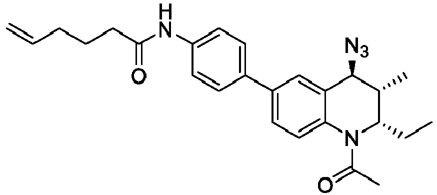

To a solution of **XB** (191.5 mg, 0.47 mmol) and pyridine (0.11 mL, 1.43 mmol) in CH₂Cl₂ (6 mL) was added acetyl chloride (36 µL, 0.50 mmol) at 0°C. The reaction mixture was stirred at 0°C to rt for 2h30. The reaction was concentrated under vacuum. After flash chromatography (silica gel, 30% EtOAc/cyclohexane), the product **XC** was isolated (117.8 mg, 84%) as a yellow solid; R*_{f}*= 0.20 (30% EtOAc/cyclohexane); [α]_{D}²⁰ = + 247.5 (c = 1.03, CHCl₃); ¹H NMR (400 MHz, CDCl₃) *δ* ppm 0.86 (t, *J =* 7.2 Hz, 3H), 1.12 (m, 1H), 1.22 (d, *J* = 6.9 Hz, 3H), 1.60 (m, 1H), 1.86 (m, 2H), 2.16 (m, 2H), 2.31 (s, 3H), 2.33 (m, 1H), 2.40 (t, *J =* 7.5 Hz, 2H), 4.11 (d, *J =* 10.0 Hz, 1H), 4.74 (m, 1H), 5.01 (m, 1H), 5.06 (m, 1H), 5.81 (ddt, *J* = 16.9, 10.0, 6.6 Hz, 1H), 7.29 (m, 1H), 7.49 (dd, *J* = 8.4, 2.1 Hz, 1H), 7.54 (d, *J =* 8.6 Hz, 2H), 7.61 (d, *J =* 2.1 Hz, 1H), 7.64 (d, *J =* 8.6 Hz, 2H); ¹³C NMR (100 MHz, CDCl₃) *δ* ppm 10.8 (CH₃), 16.9 (CH₃), 18.3 (CH₂), 23.7 (CH₃), 24.7 (CH₂), 33.3 (CH₂), 37.0 (CH₂), 39.5 (CH), 60.6 (CH), 64.1 (CH), 115.6 (CH₂), 120.4 (2xCH), 126.1 (CH), 127.0 (CH), 127.3 (CH), 127.5 (2xCH), 127.6 (C), 127.7 (C), 128.7 (C), 135.5 (C), 138.0 (CH), 138.2 (C), 170.3 (C), 171.6 (C); LMRS (ESI+) *m*/*z* (%) 359 (100) 446 (10) [*M*+H]⁺; HRMS (ESI+) *m*/*z* calc. for C₂₆H₃₁N₅O₂Na 468.2375, found 468.2383.

### N-(4-((2S,3R,4S)-1-acetyl-2-ethyl-3-methyl-4-(4-phenyl-1H-1,2,3-triazol-1-yl)-1,2,3,4-tetrahydroquinolin-6-yl)phenyl)hex-5-enamide (XD)

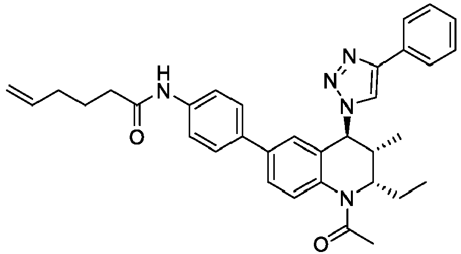

According to method **B:** To a solution of **XC** (300.0 mg, 0.67 mmol) and phenylacetylene (138.0 mg, 1.35 mmol) in CH₂Cl₂ (1 mL) was added Amberlyst Cul.Et₃N (40.0 mg) at rt. The reaction mixture was stirred at this temperature for 24 h. After completed, the reaction was filtered off, washed with CH₂Cl₂ (2 mL) and concentrated under vacuum. After flash chromatography (silica gel, 50% EtOAc/cyclohexane), the product **XD** was isolated (318.3 mg, 86%) as a white solid; R*_{f}*= 0.24 (50% EtOAc/cyclohexane); [α]_{D}²⁰ = + 294.1 (c = 0.97, CHCl₃); ¹H NMR (400 MHz, CDCl₃) *δ* ppm 0.96 (t, *J =* 7.3 Hz, 3H), 1.09 (d, *J =* 6.8 Hz, 3H), 1.31 (m, 1H), 1.77 (m, 1H), 1.82 (m, 2H), 2.10 (m, 2H), 2.35 (m, 2H), 2.41 (s, 3H), 2.64 (m, 1H), 4.96 (m, 1H), 5.00 (m, 2H), 5.69 (d, *J =* 11.3 Hz, 1H), 5.76 (m, 1H), 7.12 (s, 1H), 7.27-7.59 (m, 9H), 7.76 (m, 1H), 7.78 (d, *J=* 7.8 Hz, 2H); ¹³C NMR (100 MHZ, CDCl₃) *δ* ppm 10.8 (CH₃), 16.1 (CH₃), 18.3 (CH₂), 24.0 (CH₃), 24.7 (CH₂), 33.2 (CH₂), 37.0 (CH₂), 40.9 (CH), 60.6 (CH), 63.5 (CH), 115.6 (CH₂), 117.8 (CH), 120.3 (2xCH), 125.8 (2xCH), 127.4 (2xCH), 127.5 (C), 127.9 (CH), 128.6 (CH), 128.7 (CH), 128.9 (C), 129.0 (2xCH), 130.1 (C), 130.4 (CH), 134.9 (C), 138.0 (CH), 138.1 (C), 138.4 (C), 149.0 (C), 170.2 (C), 171.5 (C); LRMS (ESI+) *m*/*z* (%) 570 (100) 548 (10) [*M*+H]⁺; HRMS (ESI+) *m*/*z* calc. for C₃₄H₃₇N₅O₂Na 570.2845, found 570.2842.

### N-(4-((2S,3R,4S)-1-acetyl-2-ethyl-3-methyl-4-(4-pentyl-1H-1,2,3-triazol-1-yl)-1,2,3,4-tetrahydroquinolin-6-yl)phenyl)hex-5-enamide (XE)

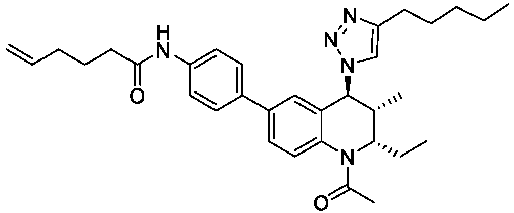

From **XC** (100.0 mg, 0.22 mmol), 1-heptyne (43.0 mg, 0.45 mmol), Amberlyst Cul.Et₃N (40.0 mg), CH₂Cl₂ (1 mL), and using the method **B,** the product was obtained after flash chromatography (silica gel 30% to 50% EtOAc/cyclohexane) to give **XE** (87.0 mg, 71.5%); R*_{f}* = 0.25 (50% EtOAc/cyclohexane); [α]_{D}²⁰ = + 234.6 (c = 0.68, CHCl₃); ¹H NMR (400 MHz, CDCl₃) *δ* ppm 0.84 (t, *J* = 6.8 Hz, 3H), 0.95 (t, *J* = 7.6 Hz, 3H), 1.05 (d, *J* = 6.8 Hz, 3H), 1.25-1.35 (m, 5H), 1.64 (m, 2H), 1.73 (m, 1H), 1.84 (m, 2H), 2.14 (m, 2H), 2.38 (m, 2H), 2.39 (s, 3H), 2.56 (m, 1H), 2.68 (m, 2H), 4.95-5.07 (m, 3H), 5.61 (d, *J* = 11.3 Hz, 1H), 5.79 (m, 1H), 7.05 (s, 1H), 7.15 (s, 1H), 7.17 (m, 1H), 7.35 (d, *J =* 8.5 Hz, 2H), 7.50 (dd, J = 8.4, 2.0 Hz, 1H), 7.56 (d, *J =* 8.5 Hz, 2H); ¹³C NMR (100 MHz, CDCl₃) *δ* ppm 10.8 (CH₃), 14.1 (CH₃), 16.1 (CH₃), 18.2 (CH₂), 22.5 (CH₂), 23.9 (CH₃), 24.7 (CH₂), 26.0 (CH₂), 29.1 (CH₂), 31.6 (CH₂), 33.3 (CH₂), 37.0 (CH₂), 40.8 (CH), 58.2 (CH), 63.2 (CH), 115.6 (CH₂), 118.7 (CH), 120.3 (2xCH), 126.3 (CH), 127.3 (CH), 127.4 (3xCH), 127.7 (C), 135.1 (C), 138.0 (CH, C), 138.1 (C), 138.3 (C), 149.9 (C), 170.2 (C), 171.5 (C); LRMS (ESI+) *m*/*z* (%) 564 (100) 542 (40) [*M*+H]⁺; HRMS (ESI+) *m*/*z* calc. for C₃₃H₄₄N₅O₂ 542.3495, found 542.3489.

### Methyl (2S,3R,4S)-1-acetyl-2-ethyl-3-methyl-4-(4-pentyl-1H-1,2,3-triazol-1-yl)-1,2,3,4-tetrahydroquinoline-6-carboxylate (XF)

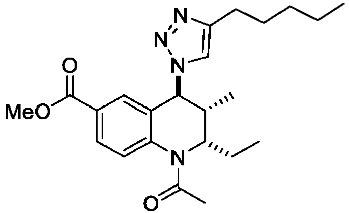

From **27** (100.0 mg, 0.32 mmol), 1-heptyne (61.0 mg, 0.63 mmol), Amberlyst Cul.Et₃N (40.0 mg), CH₂Cl₂ (1 mL), and using the method **B,** the product was obtained after flash chromatography (silica gel, 30% EtOAc/ cyclohexane) to give **XF** (98.0 mg, 75%); R*_{f}* = 0.10 (30% EtOAc/cyclohexane); [α]_{D}²⁰ = +219.7 (c = 0.76, CHCl₃); ¹H NMR (400 MHz, CDCl₃) *δ* ppm 0.88 (t, *J =* 7.4 Hz, 3H), 0.93 (t, *J =* 7.2 Hz, 3H), 1.04 (d, *J =* 6.8 Hz, 3H), 1.24 (m, 1H), 1.28-1.36 (m, 4H), 1.66 (m, 2H), 1.73 (m, 1H), 2.38 (s, 3H), 2.57 (m, 1H), 2.71 (t, *J* = 7.6 Hz, 2H), 3.83 (s, 3H), 4.69 (bs, 1H), 5.57 (d, *J* = 11.3 Hz, 1H), 7.14 (s, 1H), 7.49 (s, 1H), 7.57 (d, *J =* 2.1 Hz, 1H), 7.97 (d, *J =* 8.5, 2.1 Hz, 1H); ¹³C NMR (125 MHz, CDCl₃) *δ* ppm 10.8 (CH₃), 14.1 (CH₃), 16.1 (CH₃), 18.4 (CH₂), 22.5 (CH₂), 24.0 (CH₃), 26.0 (CH₂), 29.0 (CH₂), 31.6 (CH₂), 40.4 (CH), 52.4 (CH₃), 59.5 (CH), 62.8 (CH), 118.9 (CH), 125.8 (CH), 127.2 (C), 127.6 (C), 130.0 (CH), 130.1 (CH), 140.5 (C), 149.8 (C), 165.9 (C), 170.1 (C); LRMS (ESI+) *m*/*z* (%) 274 (100) 413 (55) [*M*+H]⁺; HRMS (ESI+) *m*/*z* calc. for C₂₃H₃₃N₄O₃ 413.2553, found 413.2551.

### N-(4-((2S,3S,4S)-1-acetyl-4-amino-2-ethyl-3-methyl-1,2,3,4-tetrahydroquinolin-6-yl)phenyl)hex-5-enamide (XG)

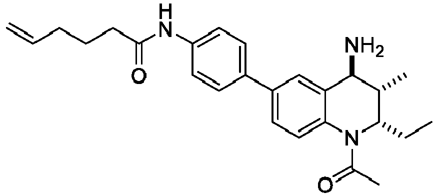

To a solution of **XC** (300.0 mg, 0.67 mmol) in methanol (6 mL) were added Et₃N (0.47 mL, 3.37 mmol) and 1,3-propanedithiol (0.34 mL, 3.37 mmol) at rt. The reaction mixture was added at this temperature for 24 h. After completion, the reaction was diluted in methanol and H₂O, acidified by HCI 1 M, washed with ether. The organic layer was basified with NaOH 1 M, extracted by EtOAc four times, dried by MgSO₄, filtered off and concentrated under vacuum. The product **XG** was obtained (240.9 mg, 85%) as a yellow solid; R*_{f}*= 0.24 (4% MeOH/CH₂Cl₂; ¹H NMR (400 MHz, CDCl₃) *δ* ppm 0.75-0.85 (t, *J =* 7.3 Hz, 3H), 1.09 (m, 1H), 1.18 (d, *J =* 6.9 Hz, 3H), 1.55 (m, 1H), 1.83 (m, 2H), 2.14 (m, 2H), 2.29 (s, 3H), 2.39 (m, 2H), 2.86 (m, 1H), 3.68 (d, *J =* 10.0 Hz, 1H), 4.78 (m, 1H), 5.00 (m, 1H), 5.04 (m, 1H), 5.81 (m, 1H), 7.21 (m, 1H), 7.40 (d, *J =* 2.0 Hz, 1H) 7.48-7.64 (m, 4H), 7.73 (m, 1H); ¹³C NMR (100 MHz, CDCl₃ ) *δ* ppm 10.7 (CH₃), 16.8 (CH₃), 18.2 (CH₂), 23.7 (CH₃), 24.8 (CH₂), 33.3 (CH₂), 36.9 (CH₂), 42.1 (CH), 53.9 (CH), 58.4 (CH), 115.5 (CH₂), 120.4 (2xCH), 125.7 (CH), 126.9 (CH), 127.3 (3×CH, C), 135.6 (C), 137.8 (C), 137.9 (2xC), 138.0 (CH), 170.3 (C), 171.9 (C); LRMS (ESI-) *m*/*z* (%) 454 (100) 418 (40) [*M*-H]⁻; HRMS (ESI-) *m*/*z* calc. for C₂₆H₃₂N₃O₂ 418.2495, found 418.2476.

### Methyl-(2S,3S,4S)-1-acetyl-4-amino-2-ethyl-3-methyl-1,2,3,4-tetrahydroquinoline-6-carboxylate (XH)

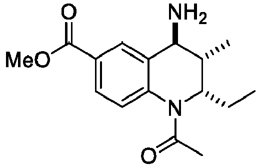

To a solution of **27** (843.2 mg, 2.66 mmol) in ethanol (40 mL) was added Pd/C (290.0 mg, 2.66 mmol). The reaction mixture was stirred at rt under H₂ for the night. The reaction was filtered off under celite, washed with ethanol and concentrated under vacuum. The product **XH** was isolated (744.5 mg, 96.2%); R*_{f}* = 0.33 (5% MeOH/CH₂Cl₂); ¹H NMR (400 MHz, CD₃OD) *δ* ppm 0.82 (t, *J* = 7.2 Hz, 3H), 1.01 (m, 1 H), 1.30 (d, *J* = 6.9 Hz, 3H), 1.68 (m, 1H), 2.31 (m, 1H), 2.34, (s, 3H), 3.93 (s, 3H), 4.31 (d, *J =* 9.5 Hz, 1H), 4.66 (m, 1H), 7.64 (d, *J =* 8.3 Hz, 1H), 8.04 (d, *J =* 8.3, 1.6 Hz, 1H), 8.27 (d, *J =* 1.6 Hz, 1H); ¹³C NMR *δ* ppm (100 MHz, CD₃OD) 10.5 (CH₃), 16.4 (CH₃), 19.2 (CH₂), 23.5 (CH₃), 39.5 (CH), 52.9 (CH₃), 54.2 (2xCH), 127.8 (CH), 128.9 (C), 131.5 (2xCH), 132.7 (C), 142.3 (C), 167.2 (C), 172.4 (C); LRMS (ESI+) *m*/*z* (%) 232 (100); HRMS (ESI+) *m*/*z* calc. for C₁₆H₂₂N₂O₃Na 313.1528, found 313.1526

### Isopropyl-((2S,3S,4S)-1-acetyl-2-ethyl-6-(4-(hex-5-enamido)phenyl)-3-methyl-1,2,3,4-tetrahydroquinolin-4-yl)carbamate (XI)

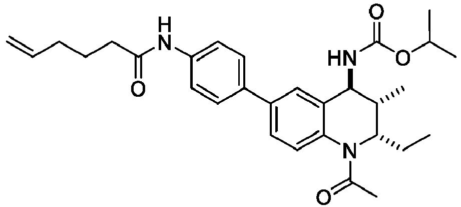

To a solution of **XG** (220.0 mg, 0.52 mmol) and DIPEA (0.27 mL, 1.57 mmol) in CH₂Cl₂ (6 mL) under N₂ at rt was added isopropyl chloroformate 2 M in toluene (0.39 mL, 0.79 mmol). The reaction mixture was stirred at this temperature for 2 h. The reaction was concentrated under vacuum. EtOAc (11 mL) and H₂O (4 mL) were added and the organic layer was washed with H₂O (3 mL), with saturated aqueous solution of NaCl (1.5 mL), dried over MgSO₄, filtered off and concentrated under vacuum. After flash chromatography (silica gel, 20% to 50% EtOAc/cyclohexane), the product **XI** (188.9 mg, 71%) was isolated as a white solid. R*_{f}*= 0.35 (50% EtOAc/cyclohexane); [α]_{D}²⁰= + 111.3 (c = 1.02, CHCl₃); ¹H NMR (400 MHz, CDCl₃) *δ* ppm 0.88 (t, *J* = 7.2 Hz, 3H), 1.15 (d, *J* = 6.8 Hz, 3H), 1.27 (d, *J* = 6.4 Hz, 6H), 1.28 (m, 1H), 1.65 (m, 1H), 1.86 (m, 2H), 2.06 (m, 1H), 2.17 (m, 2H), 2.30 (s, 3H), 2.39 (t, *J =* 7.5 Hz, 2H), 4.60 (m, 1H), 4.72 (m, 1H), 4.94- 5.11 (m, 3H), 5.82 (ddt, *J =* 16.9, 10.2, 6.7 Hz, 1H), 7.21 (m, 1H), 7.43 (dd, *J =* 8.3, 2.1 Hz, 1H), 7.51 (d, *J =* 8.5 Hz, 2H), 7.56 (m, 1H), 7.58 (d, *J =* 8.5 Hz, 2H); ¹³C NMR (100 MHz, CD₃OD) *δ* ppm 11.1 (CH₃), 16.6 (CH₃), 19.0 (CH₂), 22.6 (2×CH₃), 23.7 (CH₃), 26.2 (CH₂), 34.5 (CH₂), 37.5 (CH₂), 40.6 (CH), 54.2 (2xCH), 69.7 (CH), 115.9 (CH₂), 121.6 (2xCH), 126.7 (CH), 127.3 (C), 127.5 (CH), 128.1 (3xCH), 137.0 (C), 139.2 (CH, C), 139.6 (2xC), 159.8 (C), 172.9 (C), 174.5 (C); LRMS (ESI+) *m*/*z* (%) 528 (100) 506 (10) [*M*+H]⁺; HRMS (ESI+) *m*/*z* calc. for C₃₀H₃₉N₃O₄Na 528.2838, found 528.2839.

### Methyl-(2S,3S,4S)-1-acetyl-2-ethyl-4-((isopropoxycarbonyl)amino)-3-methyl-1,2,3,4-tetrahydroquinoline-6-carboxylate (XJ)

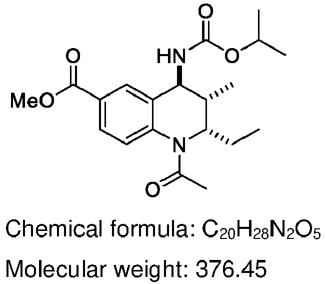

To a solution of **XH** (70.0 mg, 0.24 mmol) and DIPEA (0.12 mL, 0.70 mmol) in CH₂Cl₂ (2 mL) under N₂ at rt was added isopropyl chloroformate 2 M in toluene (0.18 mL, 0.36 mmol). The reaction mixture was stirred at this temperature for 24 h. The reaction was concentrated under vacuum. EtOAc (8 mL) and H₂O (4 mL) were added and the organic layer was washed with H₂O (3 mL), with saturated aqueous solution of NaCl (1.5 mL), dried over MgSO₄, filtered off and concentrated under vacuum. The product was obtained after flash chromatography (silica gel, 20% to 30% EtOAc/cyclohexane) to give **XJ** (70.9 mg, 78%); R*_{f}* = 0.37 (50% EtOAc/cyclohexane); ¹H NMR (400 MHz, CDCl₃) *δ* ppm 0.87 (t, *J =* 7.3 Hz, 3H), 1.13 (m, 1H), 1.14 (d, *J =* 6.9 Hz, 3H), 1.27 (d, *J* = 6.2 Hz, 3H), 1.31 (d, *J =* 6.2 Hz, 3H), 1.65 (m, 1H), 2.07 (m, 1H), 2.28 (s, 3H), 3.89 (s, 3H), 4.62 (m, 1H), 4.81 (t, *J =* 9.9 Hz, 1H), 5.0 (m, 1H), 7.34 (m, 1H), 7.89 (dd, *J* = 8.5, 2.0 Hz, 1H), 8.05 (d, *J* = 2.0 Hz, 1H); ¹³C NMR (100 MHz, CDCl₃) *δ* ppm 10.9 (CH₃), 16.4 (CH₃), 18.4 (CH₂), 22.3 (2×CH₃), 24.2 (CH₃), 40.5 (CH), 52.4 (CH₃), 52.8 (2xCH), 69.0 (CH), 125.4 (C), 127.2 (CH), 129.0 (CH), 130.6 (CH, C), 140.5 (C), 157.2 (C), 166.5 (C), 170.2 (C); LRMS (ESI+) *m*/*z* (%) 399 (100) 377 (10) [*M*+H]⁺; HRMS (ESI+) *m*/*z* calc. for C₂₀H₂₉N₂O₅ 377.2076, found 377.2069.

### N-(4-((2S,3S,4S)-1-acetyl-4-((5-chloropyrimidin-2-yl)amino)-2-ethyl-3-methyl-1,2,3,4-tetrahydroquinolin-6-yl)phenyl)hex-5-enamide (XK)

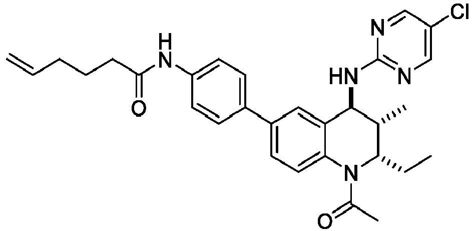

To a solution of **XG** (30.5 mg, 0.073 mmol) and 2,5-dichloropyrimidine (22.0 mg, 0.145 mmol) in DMSO (0.4 mL) was added DIPEA (28.0 mg, 0.218 mmol). The reaction mixture was stirred under microware at 160°C for 1h30. After cooling to rt EtOAc and H₂O were added. The reaction was extracted by EtOAc, the organic layer was dried by MgSO₄, filtered off and concentrated under vacuum. After flash chromatography (silica gel, 20% to 50% EtOAc/cyclohexane), the product **XK** was obtained (15.0 mg, 38.7%); R*_{f}*= 0.36 (50% EtOAc/cyclohexane); ¹H NMR (400 MHz, CDCl₃) *δ* ppm 0.91 (t, *J =* 7.2 Hz, 3H), 1.11 (d, *J* = 6.9 Hz, 3H), 1.29 (m, 1H), 1.70 (m, 1H), 1.85 (m, 2H), 2.16 (m, 2H), 2.25 (m, 1H), 2.33 (s, 3H), 2.37 (t, *J =* 7.5 Hz, 2H), 4.86 (m, 1H), 5.01 (m, 1H), 5.05 (m, 1H), 5.15 (m, 1H), 5.81 (ddt, *J =* 17.0, 10.0, 6.7 Hz, 1H), 7.29 (s, 1H), 7.39-7.44 (m, 3H), 7.48 (m, 1H), 7.54 (d, *J =* 8.2 Hz, 2H), 8.18 (s, 2H); ¹³C NMR (100 MHz, CDCl₃) *δ* ppm 10.9 (CH₃), 16.5 (CH₂), 18.4 (CH₂), 23.9 (CH₃), 24.8 (CH₂), 33.3 (CH₂), 37.1 (CH₂), 40.6 (CH), 53.4 (CH), 60.6 (CH), 115.8 (CH₂), 119.5 (C), 120.3 (2xCH), 126.1 (CH), 126.7 (C), 127.6 (4xCH), 135.1 (C), 137.5 (C), 137.8 (C) 138.0 (CH, C), 156.5 (C), 161.3 (2xCH), 170.3 (C), 171.3 (C); LRMS (ESI+) *m*/*z*(%) 532 (100) [*M*+H]⁺; HRMS (ESI+) *m*/*z* calc. for C₃₀H₃₅N₅O₂Cl 532.2479, found 532.2485.

### Methyl (2S,3S,4S)-1-acetyl-4-((5-chloropyrimidin-2-yl)amino)-2-ethyl-3-methyl-1,2,3,4-tetrahydroquinoline-6-carboxylate (XL)

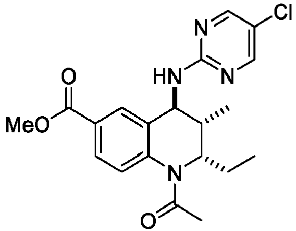

To a solution of **XH** (100.0 mg, 0.34 mmol) and 2,5-dichloropyrimidine (103.0 mg, 0.69 mmol) in DMSO (1 mL) was added DIPEA (0.17, 1.0 mmol). The reaction mixture was stirred under microware at 160°C for 1h30. After cooling to rt EtOAc and H₂O were added. The reaction was extracted by EtOAc, the organic layer was dried by MgSO₄, filtered off and concentrated under vacuum. The product was obtained after flash chromatography (silica gel, 30% to 50% EtOAc/cyclohexane) to give **XL** (13.5 mg, 10%); R*_{f}* = 0.36 (50% EtOAc/cyclohexane); ¹H NMR (400 MHz, CDCl₃) *δ* ppm 0.89 (t, *J =* 7.3 Hz, 3H), 1.10 (d, *J* = 6.9 Hz, 3H), 1.21 (m, 1H), 1.69 (m, 1H), 2.27 (m, 1H), 2.31 (s, 3H), 3.85 (s, 3H), 4.64 (m, 1H), 5.51 (d, *J =* 9.7 Hz, 1H), 7.34 (s, 1H), 7.89 (dd, *J =* 8.5, 2.1 Hz, 1H), 7.99 (d, *J =* 2.1 Hz, 1H), 8.20 (s, 2H); ¹³C NMR (100 MHz, CDCl₃) *δ* ppm 10.8 (CH₃), 16.4 (CH₃), 18.5 (CH₂), 23.9 (CH₃), 39.9 (CH), 52.3 (CH₃), 53.1 (CH), 60.5 (CH), 119.7 (CH, C), 125.4 (C), 127.1 (C), 128.8 (CH), 130.2 (CH), 140.8 (C), 156.5 (2xCH), 161.1 (C), 166.5 (C), 170.2 (C); LRMS (ESI+) *m*/*z*(%) 232 (100) 403 (45) [*M*+H]⁺; HRMS (ESI+) *m*/*z*calc. for C₂₀H₂₄N₄O₃Cl 403.1537, found 403.1534.

### Methyl (2S,3R,4S)-1-acetyl-2-ethyl-4-(4-(4-methoxyphenyl)-1H-1,2,3-triazol-1-yl)-3-methyl-1,2,3,4-tetrahydroquinoline-6-carboxylate (XM)

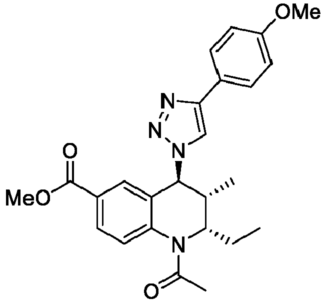

From **27** (106.0 mg, 0.33 mmol), 4-ethynylanisole (89.0 mg, 0.67 mmol), Amberlyst Cul.Et₃N (10.0 mg), CH₂Cl₂ (0.4 mL), and using the method **B,** the product was obtained after flash chromatography (silica gel, 40% EtOAc/cyclohexane) to give **XM** (118.0 mg, 78.4%); R*_{f}* = 0.25 (40% EtOAc/cyclohexane); [α]_{D}²⁰= +146.6 (c = 1.05, CHCl₃); ¹H NMR (400 MHz, CDCl₃) *δ* ppm 0.95 (t, *J* = 7.2 Hz, 3H), 1.08 (d, *J* = 6.8 Hz, 3H), 1.25 (m, 1H), 1.76 (m, 1H), 2.40 (s, 3H), 2.66 (m, 1H), 3.80 (s, 3H), 3.82 (s, 3H), 4.70 (m, 1H), 5.64 (d, *J* = 11.2 Hz, 1H), 6.93 (d, *J* = 8.8 Hz, 2H), 7.55 (m, 1H), 7.62 (s, 1H), 7.64 (s, 1H), 7.74 (d, *J* = 8.8 Hz, 2H), 7.98 (dd, *J* = 8.5, 2.0 Hz, 1H); ¹³C NMR (100 MHz, CDCl₃) *δ* ppm 10.7 (CH₃), 16.0 (CH₃), 18.4 (CH₂), 24.0 (CH₃), 40.4 (CH), 52.3 (CH₃), 55.4 (CH₃), 60.5 (CH), 62.9 (CH), 114.4 (2xCH), 117.1 (CH), 123.1 (CH), 125.8 (CH), 126.9 (C), 127.1 (2xCH), 127.5 (C), 130.0 (CH), 130.1 (CH), 140.5 (C), 148.7 (C), 159.9 (C), 165.8 (C), 170.1 (C); LRMS (ESI+) *m*/*z* (%) 449 (100) [*M*+H]⁺; HRMS (ESI+) *m*/*z* calc. for C₂₅H₂₉N₄O₄ 449.2189, found 449.2190.

### Methyl (2S,3R,4S)-1-acetyl-2-ethyl-3-methyl-4-(4-(4-(trifluoromethyl)phenyl)-1H-1,2,3-triazol-1-yl)-1,2,3,4-tetrahydroquinoline-6-carboxylate (XN)

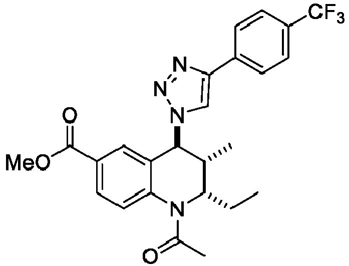

From **27** (110.0 mg, 0.35 mmol), 1-ethynyl-4-(trifluoromethyl)benzene (118.0 mg, 0.70 mmol), Amberlyst Cul.Et₃N (10.0 mg), CH₂Cl₂ (0.4 mL), and using the method **B,** the product was obtained after flash chromatography (silica gel, 30% EtOAc/cyclohexane) to give **XN** (123.0 mg, 73%); R*_{f}* = 0.15 (30% EtOAc/cyclohexane);[α]_{D}²⁰= +123.3 (c = 1.23, CHCl₃); ¹H NMR (400 MHz, CDCl₃) *δ* ppm 0.96 (t, *J* = 7.2 Hz, 3H), 1.11 (d, *J =* 6.8 Hz, 3H), 1.26 (m, 1H), 1.78 (m, 1H), 2.41 (s, 3H), 2.67 (m, 1H), 3.81 (s, 3H), 4.70 (m, 1H), 5.68 (d, *J* = 11.3 Hz, 1H), 7.51 (m, 1H), 7.64 (d, *J =* 2.0 Hz, 1H), 7.66 (d, *J =* 8.2 Hz, 2H), 7.78 (s, 1H), 7.94 (d, *J =* 8.2 Hz, 2H), 8.00 (dd, *J =* 8.6, 2.0 Hz, 1H); ¹³C NMR (100 MHz, CDCl₃) *δ* ppm 10.7 (CH₃), 16.1 (CH₃), 18.5 (CH₂), 24.0 (CH₃), 40.7 (CH), 52.4 (CH₃), 59.6 (CH), 63.3 (CH), 118.8 (CH), 124.2 (CF3, q, *J =* 272 Hz), 125.9 (2xCH, q, *J =* 4 Hz), 126.0 (2xCH), 126.7 (2xC), 127.7 (C), 130.0 (CH), 130.2 (CH), 130.3 (C, q, *J =* 32 Hz), 133.9 (C), 140.6 (C), 147.6 (C), 165.8 (C), 170.1 (C); LRMS (ESI+) *m*/*z*(%) 487 (100) [*M*+H]⁺; HRMS (ESI+) *m*/*z* calc. for C₂₅H₂₆N₄O₃F₃ 487.1957, found 487.1959.

### Methyl-(2S,3R,4S)-1-acetyl-4-(4-cyclohexyl-1H-1,2,3-triazol-1-yl)-2-ethyl-3-methyl-1,2,3,4-tetrahydroquinoline-6-carboxylate (XO)

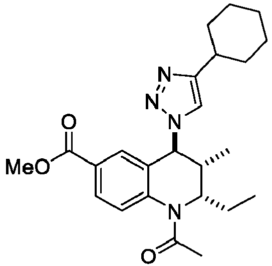

From **27** (67.5 mg, 0.21 mmol), cyclohexylacetylene (46.0 mg, 0.43 mmol), Amberlyst Cul.Et₃N (10.0 mg), CH₂Cl₂ (0.4 mL), and using the method **B,** the product was obtained after flash chromatography (silica gel, 30% EtOAc/cyclohexane) to give **XO** (67.0 mg, 74%); R*_{f}* = 0.16 (30% EtOAc/cyclohexane); [α]_{D}²⁰ = +158.6 (c = 1.20, CHCl₃); ¹H NMR (400 MHz, CDCl₃) *δ* ppm 0.93 (t, *J =* 7.2 Hz, 3H), 1.03 (d, *J =* 6.8 Hz, 3H), 1.16-1.45 (m, 5H), 1.66-1.86 (m, 5H), 2.05 (m, 2H), 2.38 (s, 3H), 2.57 (m, 1H), 2.75 (m, 1H), 3.8 (s, 3H), 4.68 (s, 1H), 5.56 (d, *J* = 11.3 Hz, 1H), 7.10 (s, 1H), 7.48 (m, 1H), 7.58 (d, *J* = 2.0 Hz, 1H), 7.97 (dd, *J =* 8.6, 2.0 Hz, 1H); ¹³C NMR (100 MHz, CDCl₃) δ ppm 10.8 (CH₃), 16.1 (CH₃), 18.5 (CH₂), 24.0 (CH₃), 26.2 (CH₂), 26.3 (CH₂), 27.1 (CH₂), 33.0 (CH₂), 33.1 (CH₂), 35.6 (CH), 40.5 (CH), 52.4 (CH₃), 59.9 (CH), 62.8 (CH), 117.5 (CH), 125.8 (CH), 127.3 (C), 127.6 (C), 130.1 (C), 130.2 (C), 140.5 (C), 155.1 (C), 166.0 (C), 170.1 (C); LRMS (ESI+) *m*/*z* (%) 425 (100) [*M*+H]⁺; HRMS (ESI+) *m*/*z* calc. for C₂₄H₃₃N₄O₃ 425.2533, found 425.2562.

### Methyl-(2S,3R,4S)-1-acetyl-2-ethyl-3-methyl-4-(4-((2-(prop-2-yn-1-yloxy)ethoxy)methyl)-1H-1,2,3-triazol-1-yl)-1,2,3,4-tetrahydroquinoline-6-carboxylate (XP)

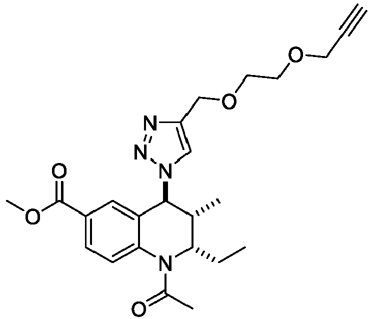

### Method C

To a stirred solution of **27** (100.0 mg, 0.32 mmol), 1,2-bis(prop-2-yn-1-yloxy)ethane (131.0 mg, 0.95 mmol) in 1:1 H₂O/t-BuOH (4 mL) was added CuSO₄.5H₂O (16.0 mg, 0.06 mmol), and sodium ascorbate (63.0 mg, 0.22 mmol) and the mixture was stirred under argon at rt for 48 h. The product was extracted with EtOAc, dried (MgSO₄) and the solvent was removed in vacuum. The product was obtained after flash chromatography (silica gel, 100% EtOAc) to give **XP** (70.2 mg, 49%); R*_{f}* = 0.44 (80% EtOAc/cyclohexane); ¹H NMR (400 MHz, CDCl₃) *δ* ppm 0.92 (t, *J =* 7.2 Hz, 3H), 1.05 (d, *J =* 6.8 Hz, 3H), 1.22 (m, 1H), 1.73 (m, 1H), 2.38 (s, 3H), 2.42 (t, *J =* 2.4 Hz, 1H), 2.59 (m, 1H), 3.68-3.75 (m, 4H), 3.84 (s, 3H), 4.17 (d, *J =* 2.4 Hz, 2H), 4.71 (m, 3H), 5.59 (d, *J =* 11.3 Hz, 1H), 7.46 (s, 1H), 7.48 (m, 1H), 7.58 (d, *J =* 2.0 Hz, 1H), 7.97 (dd, *J =* 8.6, 2.0 Hz, 1H); ¹³C NMR (100 MHz, CDCl₃) *δ* ppm 10.7 (CH₃), 16.1 (CH₃), 18.4 (CH₂), 24.0 (CH₃), 40.5 (CH), 52.4 (CH₃), 58.6 (CH₂), 59.3 (CH), 63.1 (CH), 65.1 (CH₂), 69.1 (CH₂), 70.0 (CH₂), 74.8 (CH), 79.7 (C), 120.9 (CH), 125.8 (CH), 127.0 (C), 127.6 (C), 130.1 (CH), 130.2 (CH), 140.6 (C), 146.6 (C), 165.9 (C), 170.1 (C); LRMS (ESI+) *m*/*z* (%) 455 (100) [*M*+H]⁺; HRMS (ESI+) *m*/*z* calc. for C₂₄H₃₁N₄O₅ 455.2289, found 455.2282.

### Dimethyl 4,4'-((((oxybis(ethane-2,1-diyl))bis(oxy))bis(methylene))bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XQ)

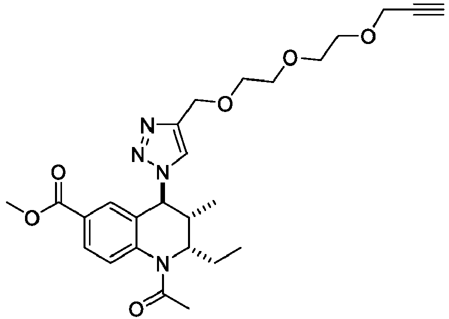

From **27** (100.0 mg, 0.32 mmol), 3-(2-(2-(prop-2-yn-1-yloxy)ethoxy)ethoxy)prop-1-yne (173.0 mg, 0.95 mmol), 1:1 H₂O/t-BuOH (4 mL), CuSO₄.5H₂O (16.0 mg, 0.06 mmol), and sodium ascorbate (63.0 mg, 0.22 mmol) and using method **C,** the product was obtained after flash chromatography (silica gel, 100% EtOAc) to give **XQ** (69.5 mg, 44%); R*_{f}* = 0.44 (4% MeOH/ CH₂Cl₂); [α]_{D}²⁰ = +97.8 (c = 0.92, CHCl₃); IR vₘₐₓ (thin film, CH₂Cl₂) 3421, 2963, 2879, 2372, 2094, 1719, 1664, 1260 cm⁻¹; ¹H NMR (400 MHz, CDCl₃) *δ* ppm 0.92 (t, *J =* 7.2 Hz, 3H), 1.04 (d, *J =* 6.8 Hz, 3H), 1.22 (m, 1H), 1.73 (m, 1H), 2.38 (s, 3H), 2.41 (t, *J =* 2.4 Hz, 1H), 2.59 (m, 1H), 3.60-3.77 (m, 8H), 3.84 (s, 3H), 4.17 (d, *J =* 2.4 Hz, 2H), 4.71 (m, 3H), 5.59 (d, *J* = 11.2 Hz, 1H), 7.44 (m, 1H), 7.46 (s, 1H), 7.57 (d, *J* = 2.0 Hz, 1H), 7.97 (d, *J =* 8.5, 2.0 Hz, 1H); ¹³C NMR (100 MHz, CDCl₃) *δ* ppm 10.7 (CH₃), 16.1 (CH₃), 18.4 (CH₂), 24.0 (CH₃), 40.4 (CH), 52.4 (CH₃), 58.5 (CH₂), 59.4 (CH), 63.1 (CH), 65.1 (CH₂), 69.2 (CH₂), 70.1 (CH₂), 70.5 (CH₂), 70.7 (CH₂), 74.7 (CH), 79.8 (C), 121.0 (CH), 125.8 (CH), 127.0 (C), 127.6 (C), 130.1 (CH), 130.2 (C), 140.6 (C), 146.6 (C), 165.9 (C), 170.1 (C); LRMS (ESI+) *m*/*z* (%) 521.2 (100) [*M*+Na]⁺, 499 (60) [*M*+H]⁺; HRMS (ESI+) *m*/*z* calc. for C₂₆H₃₅N₄O₆ 499.2551, found 499.2548.

### Methyl-(2S,3R,4S)-4-(4-(2,5,8,11-tetraoxatetradec-13-yn-1-yl)-1H-1,2,3-triazol-1-yl)-1-acetyl-2-ethyl-3-methyl-1,2,3,4-tetrahydroquinoline-6-carboxylate (XR)

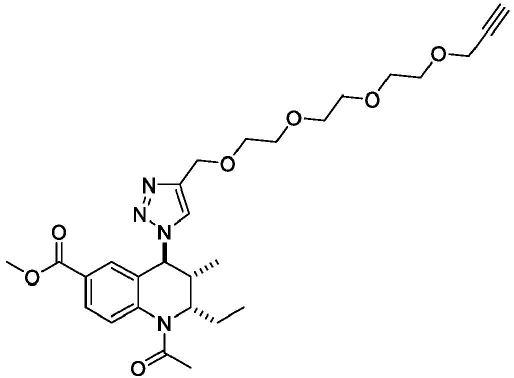

From **27** (100.0 mg, 0.32 mmol), 4,7,10,13-tetraoxahexadeca-1,15-diyne (219.0 mg, 0.95 mmol), 1:1 H₂O/t-BuOH (4 mL), CuSO₄.5H₂O (16.0 mg, 0.06 mmol), and sodium ascorbate (63.0 mg, 0.22 mmol) and using method **C,** the product was obtained after flash chromatography (silica, 100% EtOAc) to give **XR** (72.7 mg, 42%); R*_{f}* = 0.31 (4% MeOH/CH₂Cl₂); [α]_{D}²⁰ = +195.7 (c = 0.44, CHCl₃); ¹H NMR (400 MHz, CDCl₃) *δ* ppm 0.92 (t, *J =* 7.2 Hz, 3H), 1.04 (d, *J =* 6.8 Hz, 3H), 1.22 (m, 1H), 1.73 (m, 1H), 2.38 (s, 3H), 2.42 (t, *J =* 2.4 Hz, 1H), 2.60 (m, 1H), 3.63-3.72 (m, 12H), 3.84 (s, 3H), 4.18 (d, *J =* 2.4 Hz, 2H), 4.70 (m, 3H), 5.58 (d, *J* = 11.3 Hz, 1H), 7.47 (m, 2H), 7.57 (d, *J* = 2.0 Hz, 1H), 7.97 (d, *J =* 8.6, 2.0 Hz, 1H); ¹³C NMR (100 MHz, CDCl₃) *δ* ppm 10.7 (CH₃), 16.1 (CH₃), 18.4 (CH₂), 24.0 (CH₃), 40.4 (CH), 52.4 (CH₃), 58.5 (CH₂), 59.4 (CH), 63.1 (CH), 65.1 (CH₂), 69.2 (CH₂), 70.1 (CH₂), 70.5 (CH₂), 70.7 (2×CH₂), 70.8 (CH₂), 74.7 (CH), 79.8 (C), 121.0 (CH), 125.8 (CH), 127.0 (C), 127.6 (C), 130.1 (CH), 130.2 (C), 140.6 (C), 146.6 (C), 165.9 (C), 170.1 (C); LRMS (ESI+) *m*/*z*(%) 566 (100) [*M*+Na]⁺, 543 (80) [*M*+H]⁺; HRMS (ESI+) *m*/*z*calc. for C₂₈H₃₉N₄O₇ 543.2813, found 543.2808.

### Methyl-(2S,3R,4S)-4-(4-(2,5,8,11,14-pentaoxaheptadec-16-yn-1-yl)-1H-1,2,3-triazol-1-yl)-1-acetyl-2-ethyl-3-methyl-1,2,3,4-tetrahydroquinoline-6-carboxylate (XS)

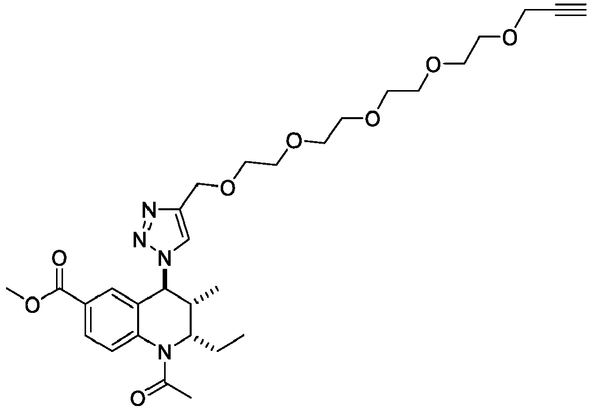

From **27** (100.0 mg, 0.32 mmol), 3,6,9,12,15-pentaoxaoctadec-1,17-diyne (256.0 mg, 0.95 mmol), 1:1 H₂O/t-BuOH (4 mL), CuSO₄.5H₂O (16.0 mg, 0.06 mmol), and sodium ascorbate (63.0 mg, 0.22 mmol) and using method **C,** the product was obtained after flash chromatography (silica, 100% EtOAc) to give **XS** (79.2 mg, 43%); R*_{f}* = 0.29 (4% MeOH/CH₂Cl₂); [α]_{D}²⁰ = +251.6 (c= 1.11, CHCl₃); ¹H NMR (400 MHz, CDCl₃) *δ* ppm 0.92 (t, *J =* 7.2 Hz, 3H), 1.05 (d, *J =* 6.8 Hz, 3H), 1.22 (m, 1H), 1.73 (m, 1H), 2.38 (s, 3H), 2.43 (t, *J =* 2.4 Hz, 1H), 2.59 (m, 1H), 3.59-3.73 (m, 16H), 3.84 (s, 3H), 4.19 (d, *J =* 2.4 Hz, 2H), 4.70 (m, 3H), 5.59 (d, *J* = 11.3 Hz, 1H), 7.46 (m, 2H), 7.57 (d, *J* = 2.0 Hz, 1H), 7.97 (d, *J =* 8.6, 2.0 Hz, 1H); ¹³C NMR (100 MHz, CDCl₃) δ ppm 10.7 (CH₃), 16.1 (CH₃), 18.4 (CH₂), 24.0 (CH₃), 40.5 (CH), 52.4 (CH₃), 58.6 (CH₂), 59.2 (CH), 63.1 (CH), 65.1 (CH₂), 69.3 (CH₂), 70.2 (CH₂), 70.6 (CH₂), 70.7 (3×CH₂), 70.8 (2×CH₂), 74.7 (CH), 79.8 (C), 120.9 (CH), 125.8 (CH), 127.0 (C), 127.6 (C), 130.1 (CH), 130.2 (CH), 140.6 (C), 146.6 (C), 165.9 (C), 170.1 (C); LRMS (ESI+) *m*/*z*(%) 609 (100) [*M*+Na]⁺, 587 (50) [*M*+H]⁺; HRMS (ESI+) *m*/*z* calc. for C₃₀H₄₃O₈N₄ 587.3075, found 587.3059.

### Methyl-(2S,3R,4S)-4-(4-(2,5,8,11,14,17-hexaoxaicos-1cos-19-yn-1-yl)-1H-1,2,3-triazol-1-yl)-1-acetyl-2-ethyl-3-methyl-1,2,3,4-tetrahydroquinoline-6-carboxylate (XT)

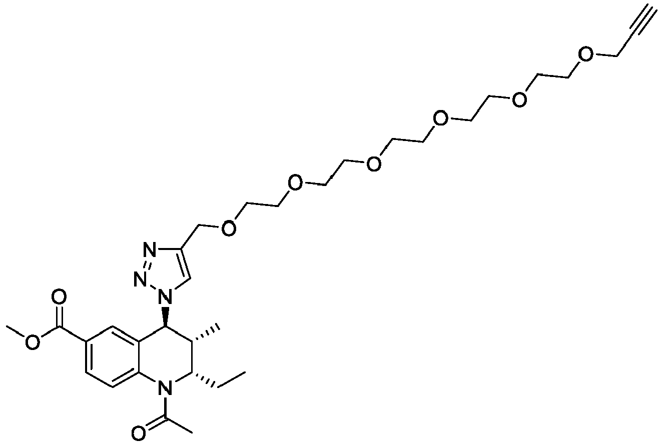

From **27** (100.0 mg, 0.32 mmol), 4,7,10,13,16,19-hexaoxadocosa-1,21-diyne (298.0 mg, 0.95 mmol), 1:1 H₂O/t-BuOH (4 mL), CuSO₄.5H₂O (16.0 mg, 0.06 mmol), and sodium ascorbate (63.0 mg, 0.22 mmol) and using method **C,** the product was obtained after flash chromatography (silica, 100% EtOAc, then 100 % CH₂Cl₂ and then gradient from 1 to 4 % MeOH in CH₂Cl₂) to give **XT** (90.0 mg, 45%); R*_{f}* = 0.29 (4% MeOH/CH₂Cl₂); ¹H NMR (400 MHz, CDCl₃) *δ* ppm 0.92 (t, *J =* 7.2 Hz, 3H), 1.04 (d, *J =* 6.8 Hz, 3H), 1.22 (m, 1H), 1.74 (m, 1H), 2.38 (s, 3H), 2.43 (t, *J =* 2.4 Hz, 1H), 2.59 (m, 1H), 3.58-3.74 (m, 20H), 3.84 (s, 3H), 4.20 (d, *J* = 2.4 Hz, 2H), 4.70 (m, 3H), 5.58 (d, *J* = 11.3 Hz, 1H), 7.46 (m, 2H), 7.57 (d, *J =* 2.0 Hz, 1H), 7.97 (d, *J =* 8.6, 2.0 Hz, 1H);¹³C NMR (100 MHz, CDCl₃) *δ* ppm 10.7 (CH₃), 16.1 (CH₃), 18.4 (CH₂), 24.0 (CH₃), 40.4 (CH), 52.4 (CH₃), 58.6 (CH₂), 59.3 (CH), 63.2 (CH), 65.0 (CH₂), 69.3 (CH₂), 70.2 (CH₂), 70.5 (3×CH₂), 70.7 (3×CH₂), 70.8 (2×CH₂), 74.7 (CH), 79.8 (C), 121.2 (CH), 125.8 (CH), 127.0 (C), 127.6 (C), 130.1 (CH), 130.3 (CH), 140.5 (C), 146.4 (C), 165.9 (C), 170.2 (C); LRMS (ESI+) *m*/*z*(%) 653 (100) [*M*+Na]⁺, 631 (55) [*M*+H]⁺; HRMS (ESI+) *m*/*z* calc. for C₃₂H₄₇O₉N₄ 631.3338, found 631.3327.

### Dimethyl-4,4'-(((ethane-1,2-diylbis(oxy))bis(methylene))bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XU)

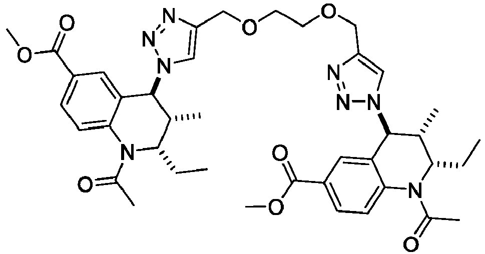

From **27** (69.6 mg, 0.22 mmol), 1,2-bis(prop-2-yn-1-yloxy)ethane (13.6 mg, 0.09 mmol), Amberlyst Cul.Et₃N (40.0 mg), CH₂Cl₂ (0.4 mL), and using the method **B** with stirring for 72 h, the product was obtained after flash chromatography (silica gel, 100% EtOAc) to give **XU** (36.8 mg, 54%); R*_{f}* = 0.37 (4% MeOH/CH₂Cl₂); [α]_{D}²⁰ = +190.9 (c = 0.95, CHCl₃); ¹H NMR (400 MHz, CDCl₃) *δ* ppm 0.92 (t, *J =* 7.2 Hz, 6H), 1.03 (d, *J =* 6.9 Hz, 6H), 1.23 (m, 2H), 1.74 (m, 2H), 2.38 (s, 6H), 2.61 (m, 2H), 3.70 (s, 4H), 3.82 (s, 6H), 4.67 (m, 6H), 5.59 (d, *J =* 11.2 Hz, 2H), 7.47 (m, 2H), 7.49 (s, 2H), 7.55 (d, *J =* 2.0 Hz, 2H), 7.96 (dd, *J =* 8.5, 2.0 Hz, 2H); ¹³C NMR (125 MHz, CDCl₃) *δ* ppm 10.8 (2xCH3), 16.1 (2xCH3), 18.4 (2xCH2), 24.1 (2×CH₃), 40.4 (2xCH), 52.4 (2×CH₃), 59.3 (2xCH), 63.1 (2xCH), 65.1 (2×CH₂), 70.1 (2×CH₂), 121.1 (2xCH), 125.8 (2xCH), 127.0 (2xC), 127.6 (2xC), 130.1 (2xCH), 130.2 (2xCH), 140.6 (2xC), 146.4 (2xC), 165.9 (2xC), 170.1 (2xC); LRMS (ESI+) *m*/*z* (%) 793 (100) [*M*+Na]⁺; HRMS (ESI+) *m*/*z*calc. for C₄₀H₅₁N₈O₈ [*M*+H]⁺ 771.3824, found 771.3824.

### Dimethyl-4,4'-((((oxybis(ethane-2,1-diyl))bis(oxy))bis(methylene))bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XV)

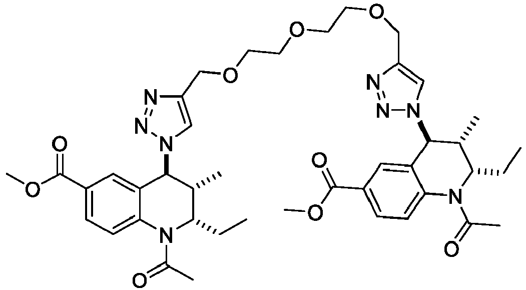

From **27** (100.0 mg, 0.32 mmol), 3-(2-(2-(prop-2-yn-1-yloxy)ethoxy)ethoxy)prop-1-yne (23.0 mg, 0.13 mmol), Amberlyst Cul.Et₃N (40.0 mg), CH₂Cl₂ (1 mL), and using the method **B** with stirring for 72 h, the product was obtained after flash chromatography (silica gel, 100% EtOAc, then 100 % CH₂Cl₂ and then gradient from 1 to 4 % MeOH in CH₂Cl₂) to give **XV** (39.3 mg, 38%); [α]_{D}²⁰ = + 189.6 (c = 0.94, CHCl₃); ¹H NMR (400 MHz, CDCl₃) *δ* ppm 0.92 (t, *J =* 7.3 Hz, 6H), 1.03 (d, *J =* 6.8 Hz, 6H), 1.23 (m, 2H), 1.73 (m, 2H), 2.37 (s, 6H), 2.61 (m, 2H), 3.59-3.72 (m, 8H), 3.83 (s, 6H), 4.67 (m, 6H), 5.59 (d, *J =* 11.3 Hz, 2H), 7.47 (m, 2H), 7.50 (s, 2H), 7.56 (d, *J =* 2.0 Hz, 2H), 7.96 (dd, *J =* 8.6, 2.0 Hz, 2H); ¹³C NMR (125 MHz, CDCl₃) *δ* ppm 10.7 (2xCH3), 16.1 (2xCH3), 18.4 (2xCH2), 24.0 (2xCH3), 40.4 (2xCH), 52.4 (2×CH₃), 59.4 (2xCH), 63.1 (2xCH), 65.1 (2×CH₂), 70.1 (2×CH₂), 70.6 (2×CH₂), 121.1 (2xCH), 125.8 (2xCH), 127.0 (2xC), 127.5 (2xC), 130.1 (2xCH), 130.2 (2xCH), 140.6 (2xC), 146.5 (2xC), 165.9 (2xC), 170.1 (2xC); HRMS (ESI+) *m*/*z* calc. for C₄₂H₅₄N₈O₉ [*M*+H]⁺ 815.4087, found 815.4087.

### Dimethy)-4,4'-((2,5,8,11-tetraoxadodecane-1,12-diyl)bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XW)

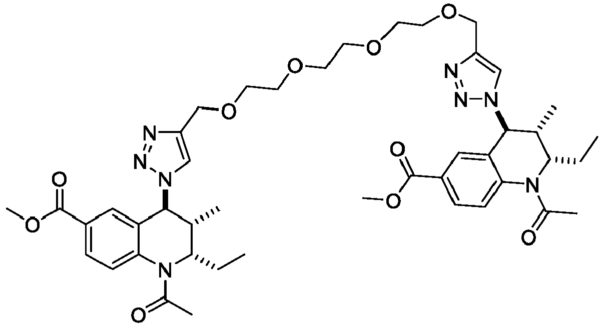

From **27** (111.0 mg, 0.35 mmol), 4,7,10,13-tetraoxahexadeca-1,15-diyne (31.6 mg, 0.14 mmol), Amberlyst Cul.Et₃N (40.0 mg), CH₂Cl₂ (1 mL), and using the method **B** with stirring for 72 h, the product was obtained after flash chromatography (silica gel, 100% EtOAc, then 100 % CH₂Cl₂ and then gradient from 1 to 4 % MeOH in CH₂Cl₂) to give **XW** (45.1 mg, 38%); R*_{f}*= 0.24 (4% MeOH/CH₂Cl₂); ¹H NMR (400 MHz, CDCl₃) *δ* ppm 0.92 (t, *J* = 7.2 Hz, 6H), 1.03 (d, *J =* 6.8 Hz, 6H), 1.22 (m, 2H), 1.73 (m, 2H), 2.37 (s, 6H), 2.60 (m, 2H), 3.61-3.70 (m, 12H), 3.83 (s, 6H), 4.67 (m, 6H), 5.58 (d, *J =* 11.3 Hz, 2H), 7.47 (m, 4H), 7.57 (d, *J =* 2.0 Hz, 2H), 7.96 (dd, *J =* 8.5, 2.0 Hz, 2H); ¹³C NMR (100 MHz, CDCl₃) *δ* ppm 10.7 (2×CH₃), 16.1 (2×CH₃), 18.4 (2×CH₂), 24.0 (2×CH₃), 40.4 (2xCH), 52.4 (2×CH₃), 59.3 (2xCH), 63.2 (2xCH), 65.0 (2×CH₂), 63.2 (2×CH₂), 70.6 (4×CH₂), 121.3 (2xCH), 125.8 (2xCH), 127.0 (2xC), 127.5 (2xC), 130.1 (2xCH), 130.2 (2xCH), 140.6 (2xC), 146.2 (2xC), 165.9 (2xC), 170.1 (2xC); LRMS (ESI+) *m*/*z* (%) 881 (100) [*M*+Na]⁺, 859 (85) [*M*+H]⁺; HRMS (ESI+) *m*/*z* calc. for C₄₄H₅₉N₈O₁₀ 859.4349, found 859.4344.

### Dimethyl-4,4'-((2,5,8,11,14-pentaoxapentadecane-1,15-diyl)bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XX)

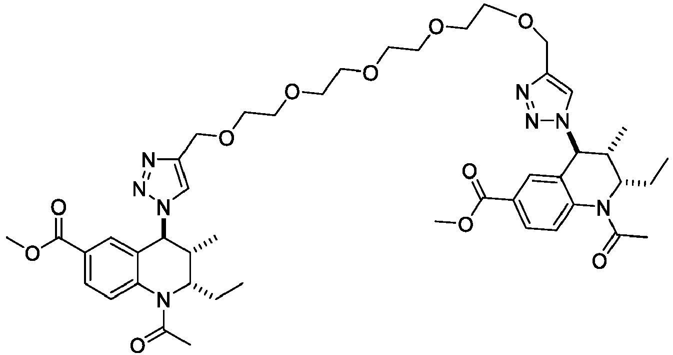

From **27** (51.6 mg, 0.19 mmol), 3,6,9,12,15-pentaoxaoctadec-1,17-diyne (20.5 mg, 0.08 mmol), Amberlyst Cul.Et₃N (33.0 mg), CH₂Cl₂ (0.4 mL), and using the method **B** with stirring for 72 h, the product was obtained after flash chromatography (silica gel, 100% EtOAc, then 100 % CH₂Cl₂ and then gradient from 1 to 4 % MeOH in CH₂Cl₂) to give a mixture of monomer and dimer. This mixture was purified by flash silica chromatography (silica gel, 6% MeOH/EtOAc) to give the desired product **XX** (42.7 mg, 62%); R*_{f}*= 0.19 (6% MeOH/EtOAc); [α]_{D}²⁰ = +130.3 (c = 0.58, CHCl₃); IR vₘₐₓ (thin film, CH₂Cl₂) 3420, 2962, 2875, 1717, 1661, 1610, 1259 cm⁻¹; ¹H NMR (400 MHz, CDCl₃) δ ppm 0.92 (t, *J* = 7.2 Hz, 6H), 1.04 (d, *J* = 6.8 Hz, 6H), 1.23 (m, 2H), 1.72 (m, 2H), 2.38 (s, 6H), 2.59 (m, 2H), 3.57-3.72 (m, 16H), 3.83 (s, 6H), 4.68 (s, 6H), 5.58 (d, *J* = 11.2 Hz, 2H), 7.46 (s, 4H), 7.57 (d, *J* = 2.0 Hz, 2H), 7.96 (dd, *J* = 8.5, 2.0 Hz, 2H); ¹³C NMR (125 MHz, CDCl₃) δ 10.8 (2×CH₃), 16.2 (2×CH₃), 18.5 (2×CH₂), 24.1 (2×CH₃), 40.5 (2xCH), 52.5 (2×CH₃), 59.3 (2xCH), 63.1 (2xCH), 65.2 (2×CH₂), 70.2 (2×CH₂), 70.8 (6×CH₂), 121.1 (2xCH), 125.8 (2xCH), 127.1 (2xC), 127.6 (2xC), 130.2 (2xCH), 130.2 (2xCH), 140.7 (2xC), 146.7 (2xC), 165.9 (2xC), 170.1 (2xC); LRMS (ESI+) *m*/*z* (%) 925 (100) [*M*+Na]⁺; HRMS (ESI+) *m*/*z* calc. for C₄₆H₆₃N₈O₁₁ [*M*+H]⁺,903.4611, found 903.4611.

### Dimethyl-4,4'-((2,5,8,11,14,17-hexaoxaoctadecane-1,18-diyl)bis(1H-1,2,3-triazole-4,1-diyl))(2S,2'S,3R,3'R,4S,4'S)-bis(1-acetyl-2-ethyl-3-methyl-1,2,3,4-tetrahydroquinoline-6-carboxylate) (XY)

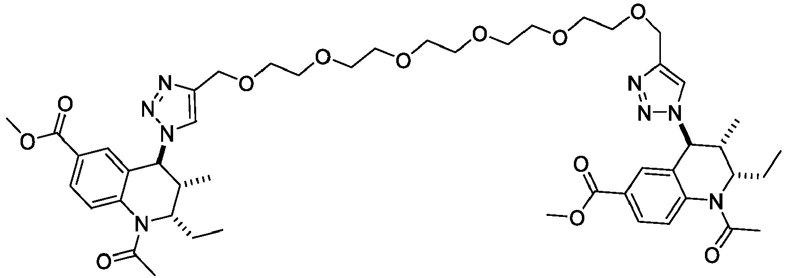

From **27** (71.9 mg, 0.23 mmol), 4,7,10,13,16,19-hexaoxadocosa-1,21-diyne (28.8 mg, 0.09 mmol), Amberlyst Cul.Et₃N (40.0 mg), CH₂Cl₂ (0.4 mL), and using the method **B** with stirring for 72 h, the product was obtained after flash chromatography (silica gel, 100% EtOAc, then 100 % CH₂Cl₂ and then gradient from 1 to 4 % MeOH in CH₂Cl₂) to give a mixture of monomer and dimer. This mixture was purified by flash silica chromatography (silica gel, 6 to 8% MeOH/EtOAc) to give the desired product **XY** (29.5 mg, 26%); R*_{f}* = 0.14 (4% MeOH/EtOAc); [α]_{D}²⁰ = +130.3 (c = 0.58, CHCl₃); ¹H NMR (400 MHz, CDCl₃) δ ppm 0.92 (t, *J* = 7.2 Hz, 6H), 1.04 (d, *J* = 6.8 Hz, 6H), 1.22 (m, 2H), 1.74 (m, 2H), 2.38 (s, 6H), 2.60 (m, 2H), 3.57-3.72 (m, 20H), 3.83 (s, 6H), 4.69 (s, 6H), 5.58 (d, *J =* 11.2 Hz, 2H), 7.47 (s, 4H), 7.57 (d, *J* = 2.0 Hz, 2H), 7.97 (dd, *J* = 8.5, 2.0 Hz, 2H); ¹³C NMR (100 MHz, CDCl₃) *δ*ppm 10.7 (2×CH₃), 16.1 (2xCH3), 18.4 (2xCH2), 24.0 (2xCH3), 40.5 (2xCH), 52.4 (2xCH3), 59.3 (2xCH), 63.1 (2xCH), 65.2 (2×CH₂), 70.2 (2×CH₂), 70.6 (2×CH₂), 70.7 (4×CH₂), 121.0 (2xCH), 125.8 (2xCH), 127.1 (2xC), 127.6 (2xC), 130.2 (2xCH), 130.2 (2xCH), 140.6 (2xC), 146.6 (2xC), 165.9 (2xC), 170.1 (2xC); LRMS (ESI+) *m*/*z* (%) 969 (100) [*M*+Na]⁺; HRMS (ESI+) *m*/*z* calc. for C₄₈H₆₇N₈O₁₂ [*M*+H]⁺ 947.4873, found 947.4873.

### Methyl-(2S,3R,4S)-1-acetyl-4-(4-(14-(2-(2,6-dioxopiperidin-3-yl)-1-oxoisoindolin-4-yl)-2,5,8,11-tetraoxatetradec-13-yn-1-yl)-1H-1,2,3-triazol-1-yl)-2-ethyl-3-methyl-1,2,3,4-tetrahydroquinoline-6-carboxylate (YF)

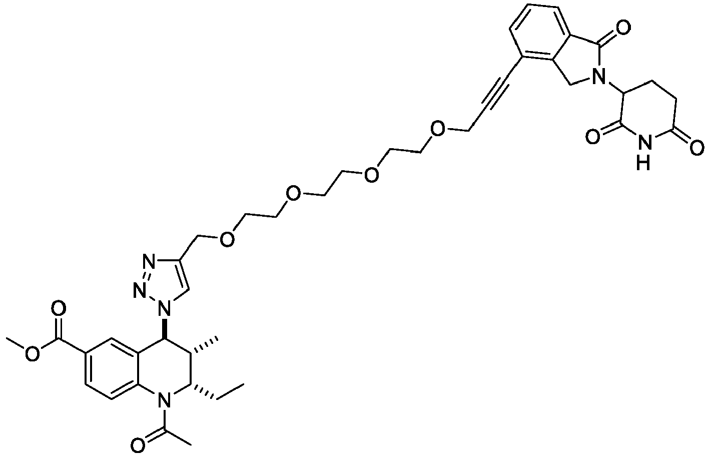

To a solution of 3-(4-iodo-1-oxoisoindolin-2-yl)piperidine-2,6-dione^{[4]} (11 mg, 0.02 mmol) and **XR** (9.5 mg, 0.257 mmol) in dry DMF (1 mL) were added Pd(PPh₃)₂Cl₂ (1.4 mg, 0.00197 mmol) and CuI (0.2 mg, 0.000985 mmol) in triethylamine (40 µL). The mixture was degazed by argon and purged many times. The reaction was stirring at 80°C for 6 h under Argon. After cooling at room temperature, the mixture was concentrated in vacuo. After flash chromatography (silica gel, 100% EtOAC, then EtOAc/MeOH (1-4%)), the product was isolated as a yellow oil (6.3 mg, 40%).

R*_{f}* = 0.30 (4% MeOH/CH₂Cl₂); ¹H NMR (400 MHz, CDCl₃) δ ppm 0.92 (t, *J* = 7.2 Hz, 3H), 1.04 (m, 3H), 1.23 (m, 1H), 1.74 (m, 1H), 2.24 (m, 1H), 2.37 (s, 3H), 2.44 (m, 1H), 2.62 (m, 1H), 2.84 (m, 1H), 2.92 (m, 1H), 3.55-3.80 (m, 12H), 3.84 (s, 3H), 4.40 (d, *J* = 16.7 Hz, 1H), 4.47 (s, 2H), 4.53 (dd, *J* = 16.7, 6.0 Hz, 1H), 4.69 (bs, 2H), 4.71 (m, 1H), 5.23 (m, 1H), 5.59 (m, 1H), 7.46 (t, *J* = 7.6 Hz, 1H), 7.51 (m, 2H), 7.57 (d, *J* = 1.9 Hz, 1H), 7.61 (d, *J* = 7.6 Hz, 1H), 7.84 (d, *J* = 7.6 Hz, 1H), 7.96 (dd, *J* = 7.6, 1.9 Hz, 1H); ¹³C NMR (100 MHz, CDCl₃) δ ppm 10.8 (CH₃), 16.2 (CH₃), 18.5 (CH₂), 23.6 (CH₂), 24.1 (CH₃), 31.8 (CH₂), 40.5 (CH), 47.3 (CH₂), 52.1 (CH), 52.5 (CH₃), 59.4 (CH₂), 59.6 (CH), 63.2 (CH), 65.1 (CH₂), 69.5 (CH₂), 70.1 (CH₂), 70.5 (2×CH₂), 70.6 (CH₂), 70.7 (CH₂), 82.1 (C), 90.8 (C), 118.4 (C), 120.1 (CH), 124.4 (CH), 125.9 (CH), 127.0 (C), 127.6 (C), 128.7 (CH), 130.1 (CH), 130.2 (CH), 132.0 (CH), 135.0 (CH), 140.6 (C), 144.1 (C), 146.5 (C), 166.0 (C), 169.0 (C), 169.7 (C), 170.2 (C), 171.3 (C); LRMS (ESI+) *m*/*z* (%) 807 (100) [*M*+Na]⁺, 785 (48) [*M*+H]⁺; HRMS (ESI+) *m*/*z* calc. for C₄₁H₄₉N₆O₁₀ [*M*+H]⁺ 785.3505, found 785.3502.

### Methyl-(2S,3R,4S)-1-acetyl-4-(4-((2-((3-(2-(2,6-dioxopiperidin-3-yl)-1-oxoisoindolin-4-yl)prop-2-yn-1-yl)oxy)ethoxy)methyl)-1H-1,2,3-triazol-1-yl)-2-ethyl-3-methyl-1,2,3,4-tetrahydroquinoline-6-carboxylate (YE)

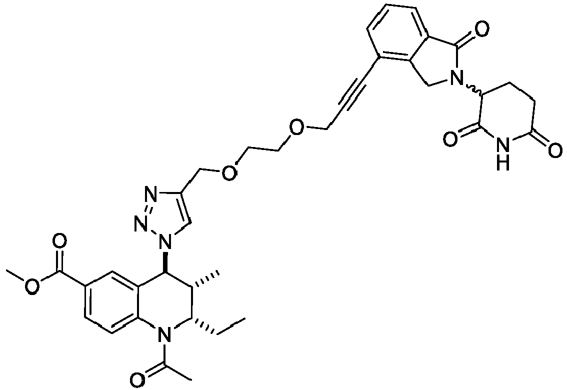

Using the same protocol as **YF,** 15.0 mg of compound **YE** was obtained (52%);
R*_{f}*= 0.30 (4% MeOH/CH₂Cl₂); ¹H NMR (400 MHz, CDCl₃) *δ* ppm 0.92 (t, *J* = 7.2 Hz, 3H), 1.04 (m, 3H), 1.22 (m, 1H), 1.72 (m, 1H), 2.23 (m, 1H), 2.37 (s, 3H), 2.48 (m, 1H), 2.57 (m, 1H), 2.85 (m, 1H), 2.95 (m, 1H), 3.65-3.79 (m, 4H), 3.83 (s, 3H), 4.41 (dd, *J* = 16.8, 3.4 Hz, 1H), 4.43 (s, 2H), 4.54 (dd, *J* = 16.8, 6.1 Hz, 1H), 4.67-4.72 (m, 3H), 5.24 (dt, *J* = 13.3, 5.3 Hz, 1 H), 5.59 (m, 1H), 7.44 (s, 1 H), 7.46 (t, *J* = 7.3 Hz, 1 H), 7.48 (m, 1H), 7.57 (s, 1 H), 7.61 (dd, *J* = 7.6, 1.0 Hz, 1H), 7.85 (d, *J* = 7.6 Hz, 1H), 7.96 (m, 1H) ); ¹³C NMR (100 MHz, CDCl₃) *δ* ppm 10.6 (CH₃), 15.9 (CH₃), 18.2 (CH₂), 23.4 (CH₂), 23.8 (CH₃), 31.6 (CH₂), 40.4 (CH), 47.1 (CH₂), 51.9 (CH), 52.3 (CH₃), 59.1 (CH₂), 59.3 (CH), 62.9 (CH), 64.9 (CH₂), 69.2 (CH₂), 69.7 (CH₂), 82.0 (C), 90.4 (C), 118.1 (C), 120.7 (CH), 124.7 (CH), 125.7 (CH), 126.8 (C), 127.4 (C), 128.4 (CH), 129.9 (CH), 130.0 (CH), 131.8 (CH), 134.8 (CH), 140.4 (C), 143.9 (C), 146.2 (C), 165.8 (C), 168.8 (C), 169.5 (C), 170.0 (C), 171.1 (C); LRMS (ESI+) *m*/*z* (%) 719 (100) [*M*+Na]⁺, 697 (90) [*M*+H]⁺; HRMS (ESI+) *m*/*z* calc. for C₃₇H₄₁N₆O₈ [*M*+H]⁺ 697.2980, found 697.2978.

### Methyl-(2S,3R,4S)-1-acetyl-4-(4-(20-(2-(2,6-dioxopiperidin-3-yl)-1-oxoisoindo)in-4-y))-2,5,8,11,14,17-hexaoxaicos-19-yn-1-y))-1H-1,2,3-triazol-1-yl)-2-ethyl-3-methyl-1,2,3,4-tetrahydroquinoline-6-carboxylate (YG)

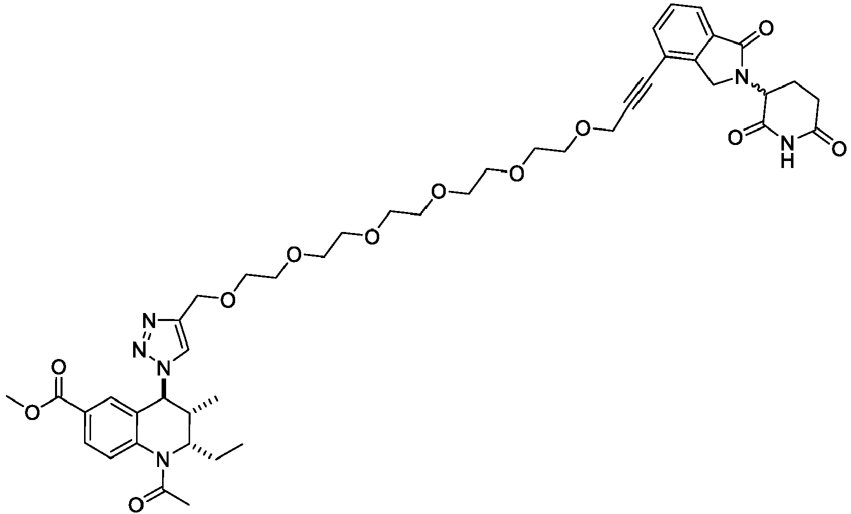

Using the same protocol as **YF,** 15.0 mg of compound **Y** was obtained (59%);R*_{f}* = 0.28 (4% MeOH/CH₂Cl₂); ¹H NMR (400 MHz, CDCl₃) *δ* ppm 0.92 (t, *J* = 7.2 Hz, 3H), 1.04 (d ; *J* = 6.8 Hz, 3H), 1.22 (m, 1H), 1.73 (m, 1H), 2.23 (m, 1H), 2.38 (s, 3H), 2.48 (m, 1H), 2.65 (m, 1H), 2.84 (m, 1H), 2.92 (m, 1H), 3.59-3.80 (m, 20H), 3.84 (s, 3H), 4.44 (m, 1H), 4.48 (s, 2H), 4.55 (dd, *J* = 16.9, 2.5 Hz, 1H), 4.71 (bs, 3H), 5.22 (m, 1H), 5.60 (d, *J* = 11.2 Hz, 1H), 7.46 (t, *J* = 7.7 Hz, 1H), 7.51 (bs, 2H), 7.57 (d, *J* = 1.1 Hz, 1H), 7.62 (d, *J* = 7.7 Hz, 1H), 7.84 (d, *J* = 7.7, 1.1 Hz, 1H), 7.96 (d, *J* = 7.7 Hz, 1H); ¹³C NMR (100 MHz, CDCl₃) *δ* ppm 10.8 (CH₃), 16.2 (CH₃), 18.5 (CH₂), 23.6 (CH₂), 24.1 (CH₃), 31.8 (CH₂), 40.4 (CH), 47.5 (CH₂), 52.2 (CH), 52.5 (CH₃), 59.4 (CH₂), 59.6 (CH), 63.1 (CH), 64.9 (CH₂), 69.3 (CH₂), 69.8 (CH₂), 70.3 (6×CH₂), 70.5 (2×CH₂), 82.2 (C), 90.7 (C), 118.3 (C), 121.3 (CH), 124.4 (CH), 125.9 (CH), 127.0 (C), 127.5 (C), 128.6 (CH), 130.1 (CH), 130.2 (CH), 132.0 (CH), 134.8 (CH), 140.7 (C), 144.1 (C), 146.2 (C), 166.0 (C), 169.0 (C), 169.8 (C), 170.2 (C), 171.4 (C); LRMS (ESI+) *m*/*z* (%) 895 (100) [*M*+Na]⁺, 873 (48) [*M*+H]⁺; HRMS (ESI+) *m*/*z* calc. for C₄₅H₅₇N₆O₁₂ [*M*+H]⁺ 873.4029, found 873.4029.

### 2S,3R,4S)-4-azido-2-ethyl-3-methyl-6-(trifluoromethyl)-1,2,3,4-tetrahydroquinoline (YA)

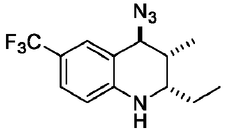

To a solution of 4(trifluoromethyl)aniline (506.7 mg, 3.145 mmol) and propionaldehyde (41.52g, 77.58 mmol) in CH₃CN (31 mL) at -20°C was added D-proline (109.4 mg, 0.955 mmol). The reaction mixture was stirred for 20 min at -20°C and kept at -20°C for two days. TMSN₃ (0.58 mL, 4.027 mmol) and BF₃Et₂O (0.28 mL, 2.201 mmol) were added at rt and the reaction mixture was stirred at this temperature for 30 min. After the reaction was complete, it was quenched by NaOH (1 N) until neutralization, extracted with EtOAc, dried by MgSO₄, filtered off and concentrated under vacuum. After flash chromatoghraphy (silica gel, 1% EtOAc/5% DCM/ 86% cyclohexane), the product **YA** was isolated (710.9 mg, 80%); R*_{f}* = 0.32 (2% EtOAc/cyclohexane); [α]_{D}²⁰ = -207.1 (c=0.78, CHCl₃); IR vₘₐₓ (thin film, CH₂Cl₂) 2970, 2090, 1622, 1520, 1330, 1109 cm⁻¹; ¹H NMR (400 MHz, CDCl₃) *δ* ppm 0.75 (d, *J* = 7.1 Hz, 3H), 1.02 (t, *J* = 7.5 Hz, 3H), 1.53-1.60 (m, 2H), 1.97 (m, 1H), 3.38 (td, *J* = 7.2, 2.8 Hz, 1H), 4.20 (s, 1H), 4.24 (d, *J* = 2.8 Hz, 1H), 6.56 (d, *J* = 8.1 Hz, 1H), 7.30-7.35 (m, 2H); ¹³C NMR (100 MHz, CDCl₃) *δ* ppm 10.4 (CH₃), 10.5 (CH₃), 25.3 (CH₂), 33.2 (CH), 51.0 (CH), 63.7 (CH), 113.9 (CH), 115.0 (C), 118.4 (C, q, *J* = 32.8 Hz), 124.9 (C, q, *J* = 270.5 Hz), 126.9 (C, q, *J* = 3.5 Hz), 128.6 (C, q, *J =* 7.5 Hz), 146.8 (C); LRMS (ESI+) *m*/*z* (%) 304 (100), 278 (10) [*M*+Na]⁺; HRMS (ESI-) *m*/*z* calc. for C₁₃H₁₅ F₃N₄Cl [*M*+Cl]-319.0942, found 319.0929.

### 1-((2S,3R,4S)-4-azido-2-ethyl-3-methyl-6-(trifluoromethyl)-3,4-dihydroquinolin-1(2H)-yl)ethan-1-one (YB)

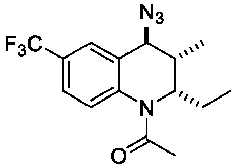

To a solution of **YA** (638.6 mg, 2.21 mmol) and pyridine (0.53 mL, 6.63 mmol) in CH₂Cl₂ (5 mL) was added acetyl chloride (0.32 mL, 4.42 mmol) at 0°C. The reaction mixture was stirred at 0°C to rt for 2h30 and then stirred at rt overnight. The reaction was concentrated under vacuum. After flash chromatography (silica gel, 20% EtOAc/cyclohexane), the product **YB** was isolated (350.1 mg, 48.5%); R*_{f}* = 0.12 (10% EtOAc/cyclohexane); [α]_{D}²⁰= +145.6 (c=1, CHCl₃); IR vₘₐₓ (thin film, CH₂Cl₂): 3478, 3055, 2969, 2486, 2097, 1666, 1331, 1169, 737 cm⁻¹; ¹H NMR (400 MHz, CDCl₃) *δ* ppm 0.88 (t, *J* = 7.2 Hz, 3H), 1.09 (m, 1H), 1.25 (d, *J* = 6.9 Hz, 3H), 1.64 (m, 1H), 2.30 (s, 3H), 2.34 (m, 1H), 4.11 (d, *J* = 10.4 Hz, 1H), 4.62 (bs, 1H), 7.49 (m, 1H), 7.56 (dd, *J* = 8.5, 2.1 Hz, 1H), 7.72 (bs, 1H); ¹³C NMR (100 MHz, CDCl₃) *δ* ppm 10.6 (CH₃), 16.7 (CH₃), 18.3 (CH₃), 23.6 (CH₂), 38.8 (CH), 59.1 (CH), 63.3 (CH), 119.7 (CH), 122.4 (C), 125.1 (C), 125.4 (C, q, *J =* 125.4 Hz), 125.9 (C), 126.2 (C, q, *J* = 126.21 Hz), 127.3 (C, q, *J* = 127.5 Hz), 128.6 (C), 139.8 (C), 169.9 (C); LRMS (ESI+) *m*/*z* (%) 242 (100) 327 (40) [*M*+H]⁺; HRMS (ESI+) *m*/*z* calc. for C₁₅H₁₈N₄OF₃ 327.1433 found 327.1425.

### 1-((2S,3R,4S)-2-Ethyl-3-methyl-4-(4-phenyl-1H-1,2,3-triazol-1-yl)-6-(trifluoromethyl)-3,4-dihydroquinolin-1(2H)-yl)ethan-1-one (YC)

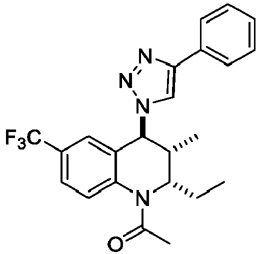

From **YB** (55.0 mg, 0.17 mmol), phenylacetylene (37 µL, 0.34 mmol), Amberlyst Cul.Et₃N (20.0 mg), CH₂Cl₂ (0.5 mL), and using the method **B** with stirring for 72 h, the product was obtained after flash chromatography (silica, 20% to 40% EtOAc/cyclohexane) to give **YC** (67.7 mg, 93.5%); R*_{f}* = 0.26 (20% EtOAc/cyclohexane); ¹H NMR (400 MHz, CDCl₃) *δ* ppm 0.97 (t, *J* = 7.3 Hz, 3H), 1.09 (d, *J* = 6.9 Hz, 3H), 1.26 (m, 1H), 1.78 (m, 1H), 2.40 (s, 3H), 2.67 (m, 1H), 4.65 (m, 1H), 5.66 (d, *J* = 11.5 Hz, 1H), 7.22 (s, 1H), 7.35 (d, *J* = 7.5 Hz, 1H), 7.39-7.45 (m, 3H), 7.56-7.60 (m, 2H), 7.83 (d, *J* = 7.5 Hz, 2H);; LRMS (ESI+) *m*/*z* (%) 429 (100) [*M*+H]⁺; HRMS (ESI+) *m*/*z* calc. for C₂₃H₂₄N₄OF₃ 429.1902, found 429.1898.

### 1-((2S,3R,4S)-2-ethyl-3-methyl-4-(4-pentyl-1H-1,2,3-triazol-1-yl)-6-(trifluoromethyl)-3,4-dihydroquinolin-1(2H)-yl)ethan-1-one (YD)

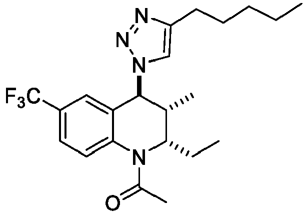

From **YB** (100.0 mg, 0.31 mmol), 1-heptyne (59 mg, 0.61 mmol), Amberlyst CuI.Et₃N (40.0 mg), CH₂Cl₂ (1 mL), and using the method **B** with stirring for 48 h, the product was obtained after flash chromatography (silica, 20% to 30% EtOAc/cyclohexane) to give **YD** (107.5 mg, 83%); R*_{f}* = 0.38 (30% EtOAc/cyclohexane); ¹H NMR (400 MHz, CDCl₃) *δ* ppm 0.87 (t, *J* = 6.8 Hz, 3H), 0.95 (t, *J* = 7.2 Hz, 3H), 1.04 (d, *J* = 6.8 Hz, 3H), 1.2-1.38 (m, 5H), 1.61-1.71 (m, 3H), 1.75 (m, 1H), 2.38 (s, 3H), 2.59 (m, 1H), 2.71 (t *J* = 7.6 Hz, 2H), 4.63 (bs, 1H), 5.57 (d, *J* = 11.3 Hz, 1H), 7.12 (m, 1H), 7.15 (m, 1H), 7.56 (d, *J* = 8.0 Hz, 1H);; LRMS (ESI+) *m*/*z* (%) 423 (100) [*M*+H]⁺; HRMS (ESI+) *m*/*z* calc. for C₂₂H₃₀N₄OF₃ 423.2372, found 423.2374.

### N-(4-((2S,3R,4S)-1-acetyl-4-azido-2-ethyl-3-methyl-1,2,3,4-tetrahydroquinolin-6-yl)phenyl)-N-hydroxyoctanediamide (YH)

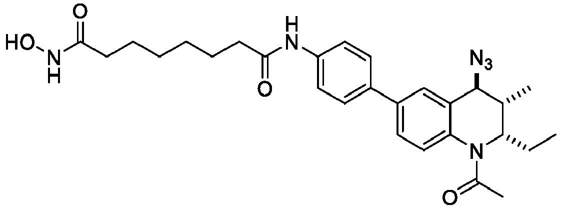

R*_{f}* = 0.22 (5% MeOH/CH₂Cl₂); ¹H NMR (400 MHz, CDCl₃) *δ* ppm 0.85 (d, *J* = 7.3 Hz, 3H), 1.11 (m, 1H), 1.21 (d, *J* = 6.9 Hz, 3H), 1.27-1.46 (m, 5H), 1.64 (m, 2H), 1.71 (m, 2H), 2.09 (t, *J* = 7.4 Hz, 2H), 2.29 (s, 3H), 2.31 (m, 1H), 2.39 (t, *J* = 7.4 Hz, 2H), 4.32 (d, *J* = 9.5 Hz, 1H), 4.64 (m, 1H), 7.23 (d, *J* = 7.9 Hz, 1H), 7.44 (m, 1H), 7.60 (d, *J* = 8.2 Hz, 2H), 7.66 (m, 1H), 7.70 (d, *J* = 8.2 Hz, 2H); ¹³C NMR (100 MHz, CD₃OD) δ ppm 11.0 (CH₃), 16.9 (CH₃), 19.5 (CH), 21.4 (CH₃), 26.7 (CH₂), 26.8 (CH₂), 29.9 (CH₂), 30.0 (CH₂), 34.8 (CH₂), 38.0 (CH₂), 40.9 (CH), 60.4 (CH), 65.0 (CH), 121.7 (CH), 127.1 (2xCH), 127.7 (CH), 128.2 (CH), 129.9 (2xCH), 130.6 (C), 136.7 (C), 139.8 (C), 141.9 (C), 143.6 (C), 172.8 (C), 173.1 (C), 174.8 (C); LRMS (ESI+) *m*/*z* (%) 521 (30) [*M*+H]⁺, 543 (100) [*M*+Na]⁺; HRMS (ESI+) *m*/*z* calc. for C₂₈H₃₇N₆O₄ 521.2863, found 521.2863.

### Methyl-(2S,3R,4S)-1-acetyl-2-ethyl-4-(6-(hydroxymethyl)-5,5a,6,6a,7,8-hexahydrocyclopropa[5,6]cycloocta[1,2-d][1,2,3]triazol-1(4H)-yl)-3-methyl-1,2,3,4-tetrahydroquinoline-6-carboxylate (YI)

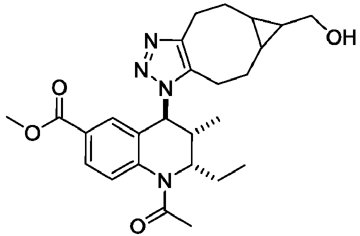

Compound **26** (21 mg, 0.06 mmol) and bicyclo[6.1.0]non-4-yn-9-ylmethanol (BCN, 10 mg, 0.06 mmol) were dissolved in CHCl₃ (1 mL) and the reaction was stirred for 2 h at room temperature. The solvent was removed *in vacuo* and the product was obtained after flash chromatography (silica, 4% MeOH/CH₂Cl₂) to give **YI** (13 mg, 42%);
Rf= 0.33 (4% MeOH/CH₂Cl₂); ¹H NMR (400 MHz, CDCl₃) *δ* ppm 0.93 (d, *J=* 7.2 Hz, 3H), 0.96-1.34 (m, 9H), 1.44-1.59 (m, 2H), 1.67-1.95 (m, 3H), 2.26 (m, 1H), 2.36 (s, 3H), 2.48-2.79 (m, 2H), 2.92 (m, 1H), 3.11 (m, 1H), 3.61 (m, 1H), 3.72 (m, 1H), 3.82 (s, 3H), 4.81 (m, 1H), 5.59 (m, 1H), 7.42 (m, 1H), 7.48 (m, 1H), 7.96 (m, 1H);); LRMS (ESI+) *m*/*z* (%) 489 (88) [*M*+Na]⁺, 467 (100) [*M*+H]⁺; HRMS (ESI+) *m*/*z* calc. for C₂₆H₃₅N₄O₄ [*M*+H]+ 467.2653, found 467.2646.

### References

[1] Hatano, M.; Yamashita, K.; Ishihara, K. Org. Lett. 2015, 17, 2412-2415
[2] Allais, F.; Ducrot, P.-H. Synthesis 2010, 1649-1653
[3] Girard, C.; Onen, E.; Aufort, M.; Beauvière, S.; Samson, E.; Herscovici, J. Org. Lett. 2006, 8, 1689-1692.
[4] Chong, Q.; Hu, Y. Zhou, B.; Fernandez-Salas, E.; Yang, C.-Y.; Liu, L.; McEachern, D. et al. J. Med. Chem. 2018, 61,6685-6704.

### In vitro experimentations

### Evaluation of cytotoxicity and cytokine expression after iBET/LPS treatment

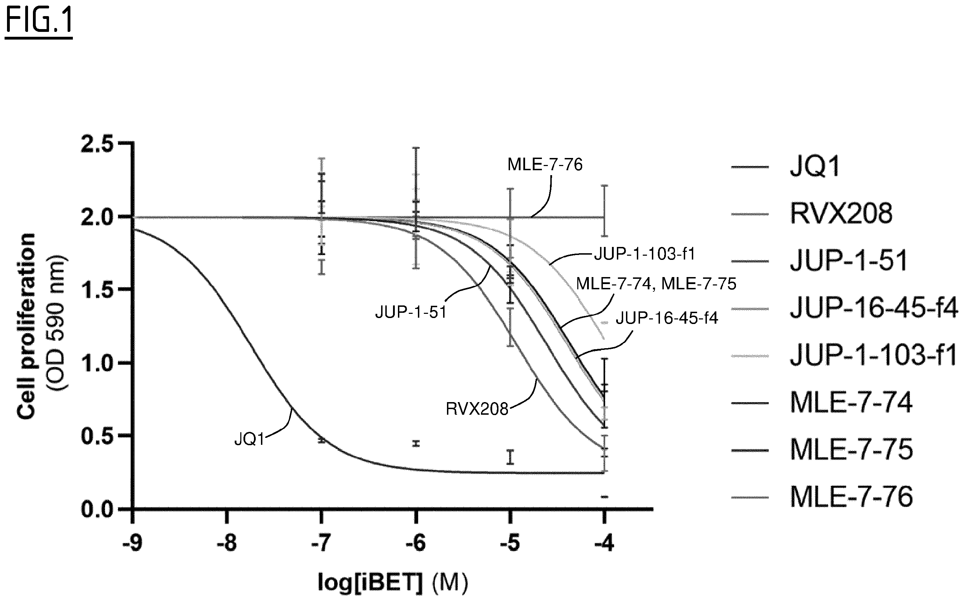

J774 mouse macrophages were cultured in DMEM 10% Fetal Calf Serum (FCS) 1% penicillin and streptomycin at 37°C 5% CO2. 250,000 cells/ ml. Cells were then seeded at 250,000 cells/ml and treated with 1 µM, 10 µM and 100 µM for each iBET and, then, after 30 min, with 100µg/ml LPS (lipopolysaccharides, Cell Signaling Technology ref: 14011S) to induce inflammatory response. After 24h, cytotoxicity was evaluated using CytoTox-ONE^{™} Homogeneous Membrane Integrity Assay and/or CellTiter 96 Non-radioactive Cell Proliferation Assay (Promega) according to manufacturer's instructions. IL6 and TNFα secretion were assessed using respectively
IL-6 Mouse ELISA assay and TNFα Mouse ELISA assay (Invitrogen) according to manufacturer's instructions.
Results are given on figures 1 (J774) and 2 to 5 (IL-6 and TNFα).

### In vitro evaluation of bromodomain inhibitors by Homogeneous Time Resolved Fluorescence

HTRF reagents and buffers were purchased from Cisbio Bioassays. The assay used a terbium (III) cryptate donor reagent conjugated to an anti-GST antibody (MAb anti-GST-Tb; GSTTLA), a streptavidin-conjugated acceptor reagent (streptavidin-d2) and Cisbio proprietary buffers (EPIgeneous Binding Domain Diluent and Detection buffer, respectively). GST-tagged bromodomains (BDs) were expressed in *E. coli* and purified using standard procedures. Incubation of GST-tagged BDs with biotinylated acetylated H4 peptide (H4K5acK8acK12acK16ac, here named H4ac4) brings the donor and acceptor into close proximity and allows for a FRET reaction. GST-tagged proteins in 25 mM Hepes pH 7.5, 150 mM NaCl, 0.5 mM DTT were assayed at a final concentration of 5 nM. Biotinylated H4ac4 peptides were used at a final concentration of 50, 600 nM in assays involving Brd4 BD1, Brd4 BD2, respectively. The antibody-conjugated donor was used at 0.5 nM and the streptavidin-conjugated acceptor was used at 1/8 of the H4ac4 peptide concentration. Inhibitors were tested by performing an eleven-point dilution series with a maximal final concentration of 20 mM. These concentrations allowed a fixed DMSO concentration at 0.2 %, critical for a Z' factor ≥ 0.8. Components were incubated at 4 °C for 4 h (BD1) or for 24 h (BD2). Experiments were performed in 384-well white plates (Greiner ref. 781080) and analyzed in a ClarioStar plate reader (BMG LABTECH, excitation at 330 nm and emission at 620 and 665 nm, corresponding to the donor and acceptor emission peaks, respectively; the 665/620 ratio is used to calculate the specific HTRF signal) with an integration delay of 60 µs and an integration time of 400 µs.

### HTRF Results (IC50 sur BD1 et BD2)

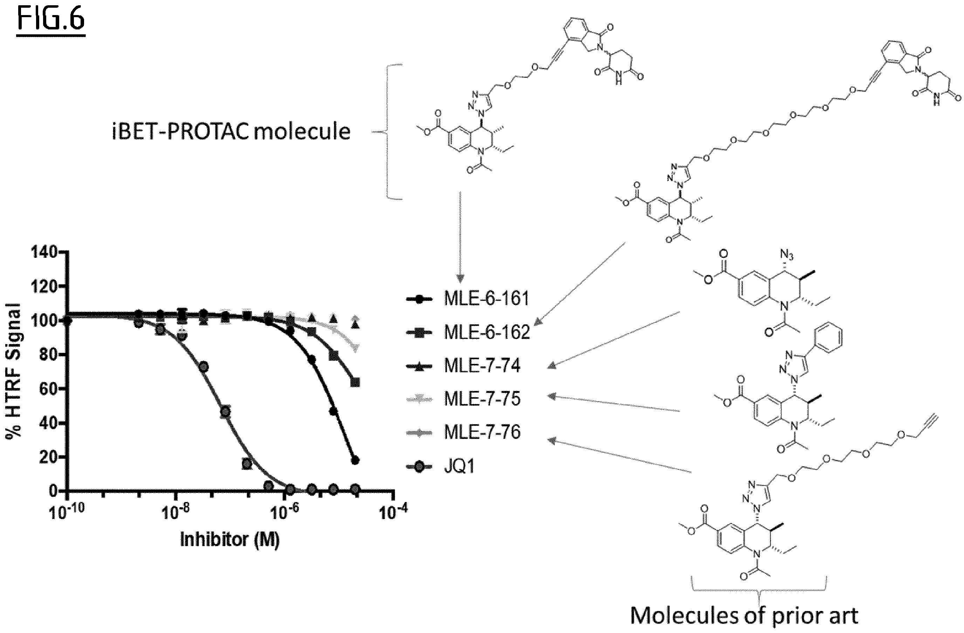

Some of the HTRF results are illustrated on Figure 6.

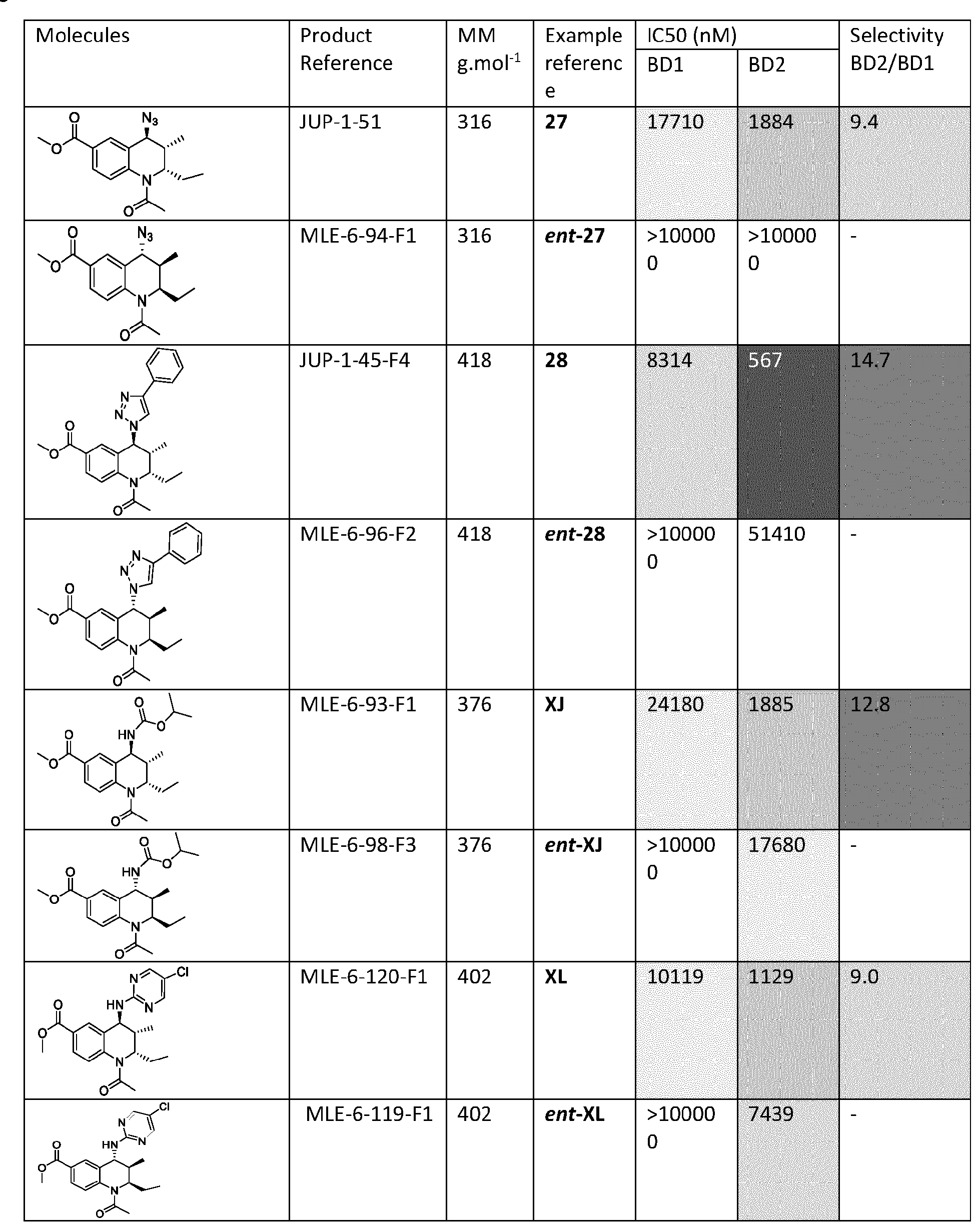

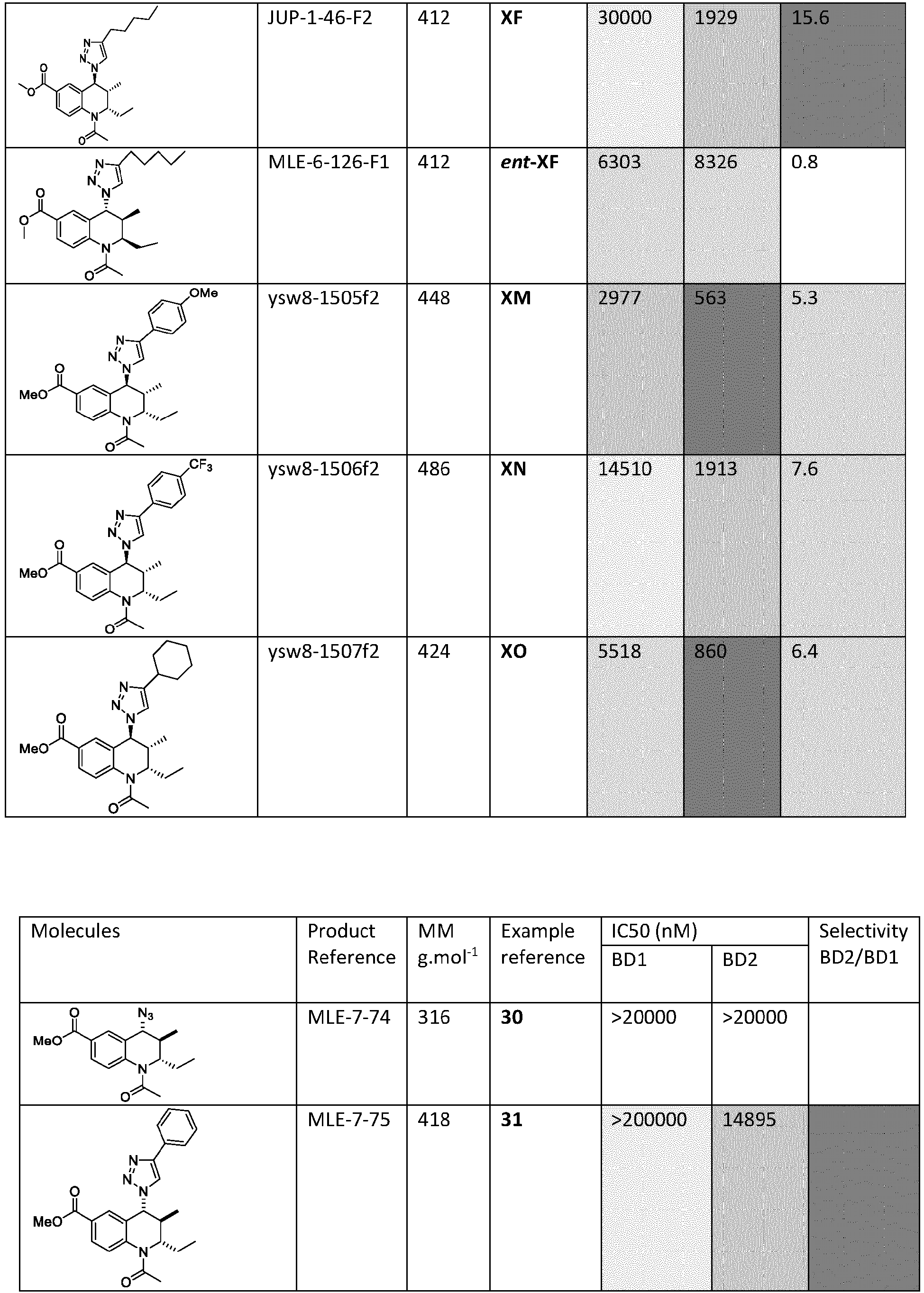

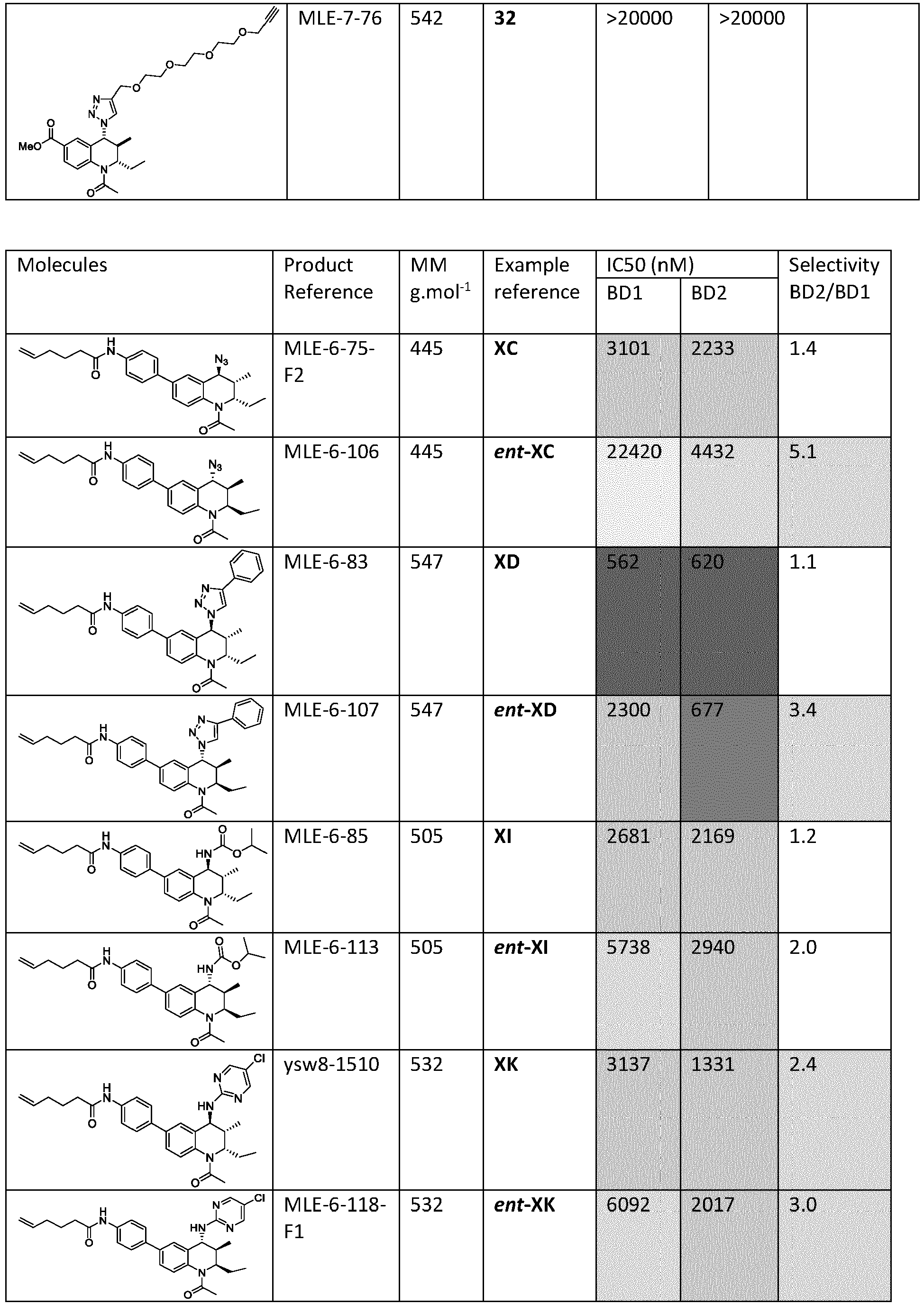

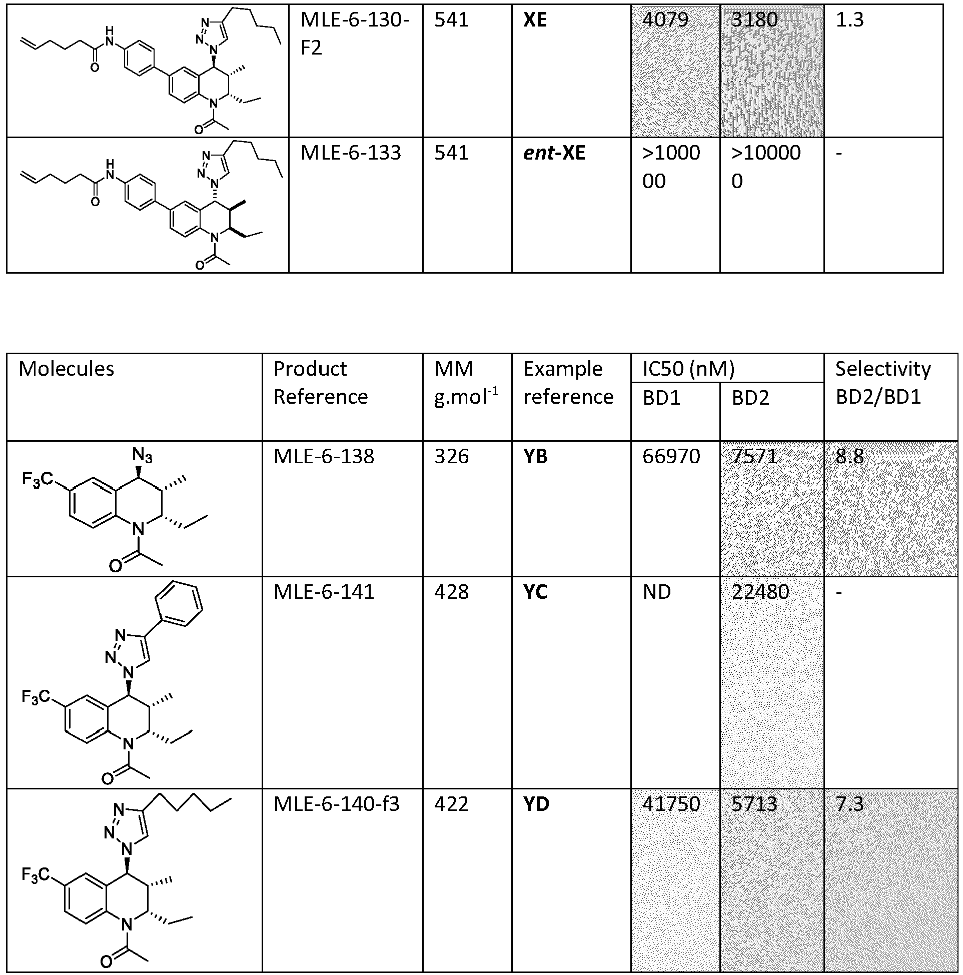

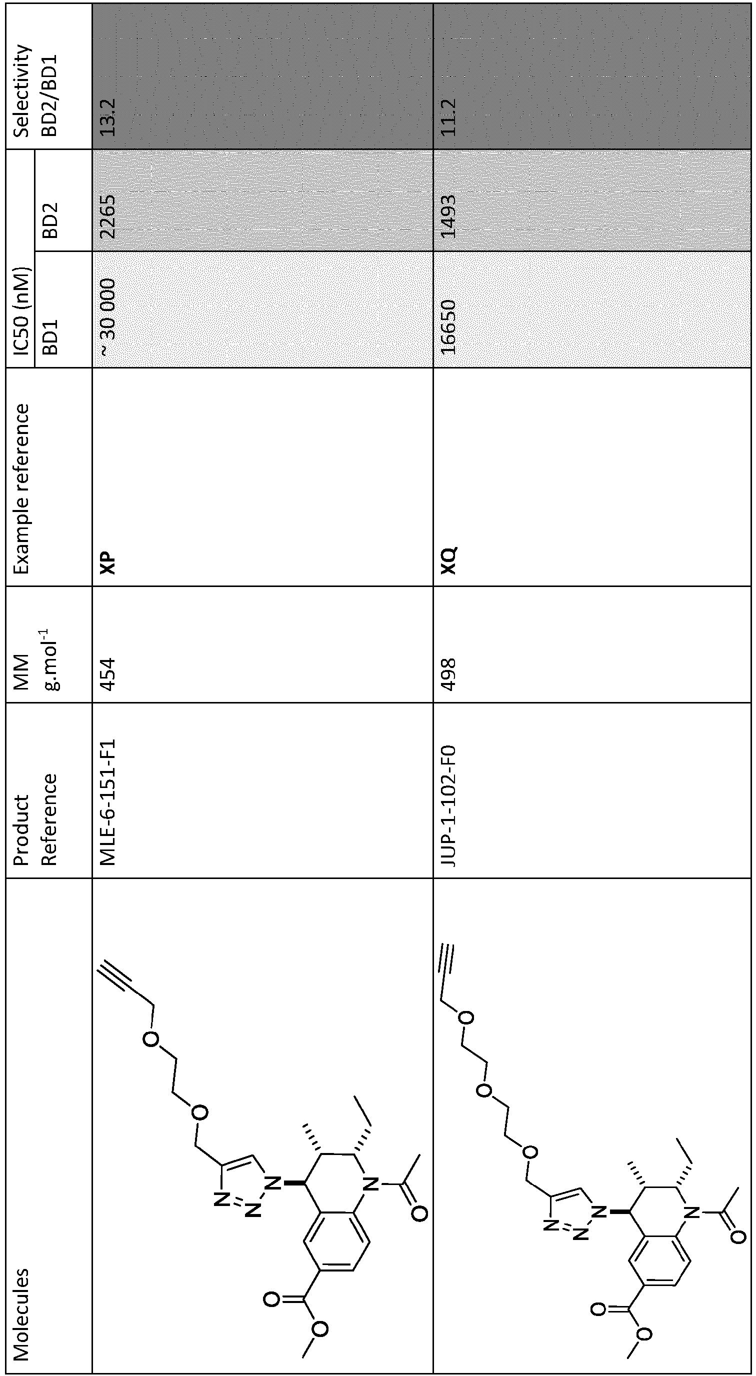

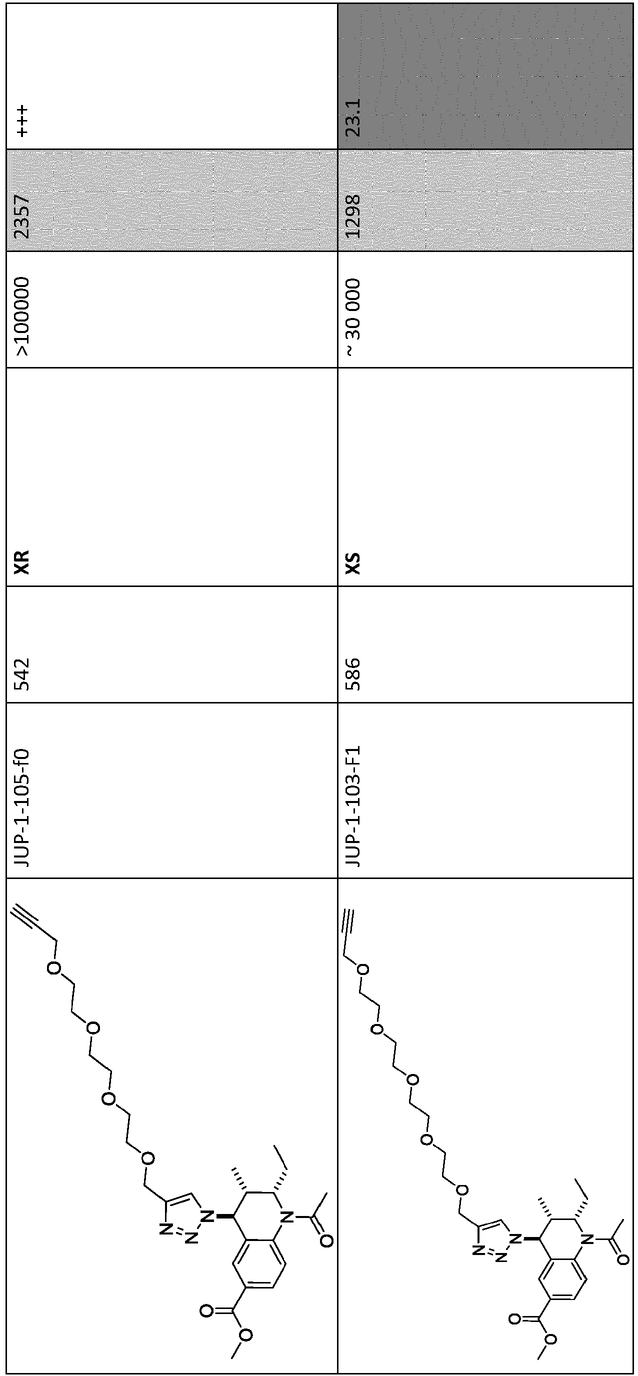

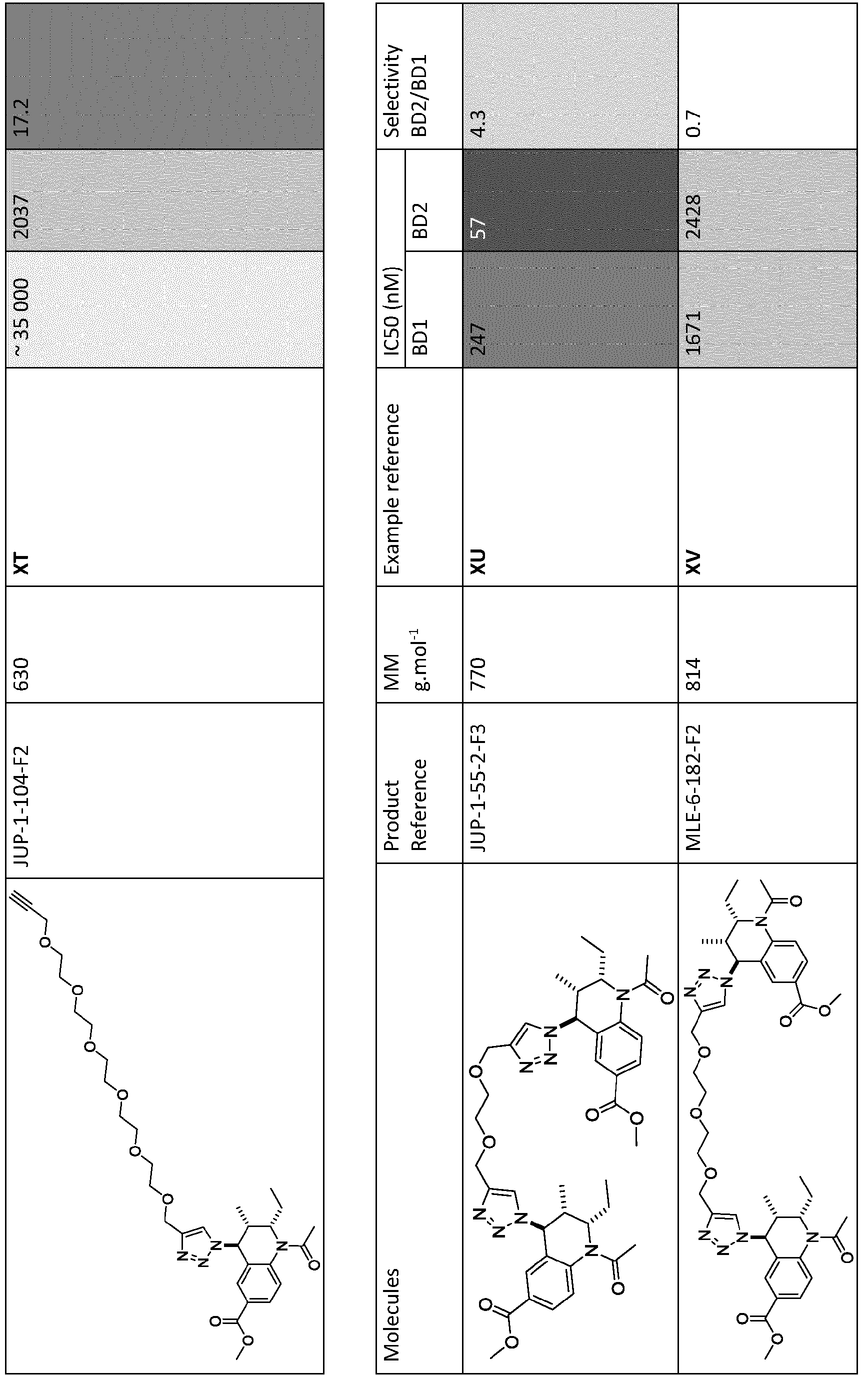

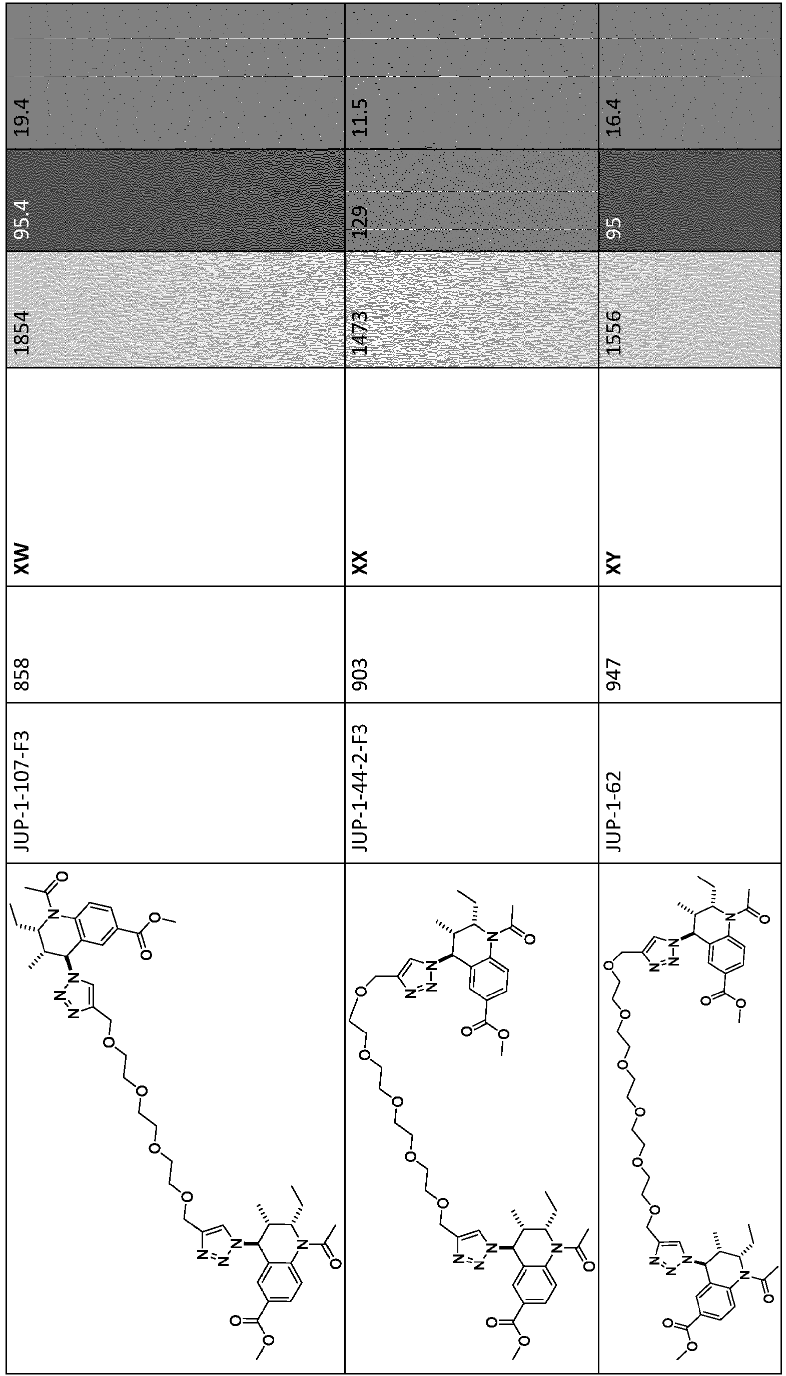

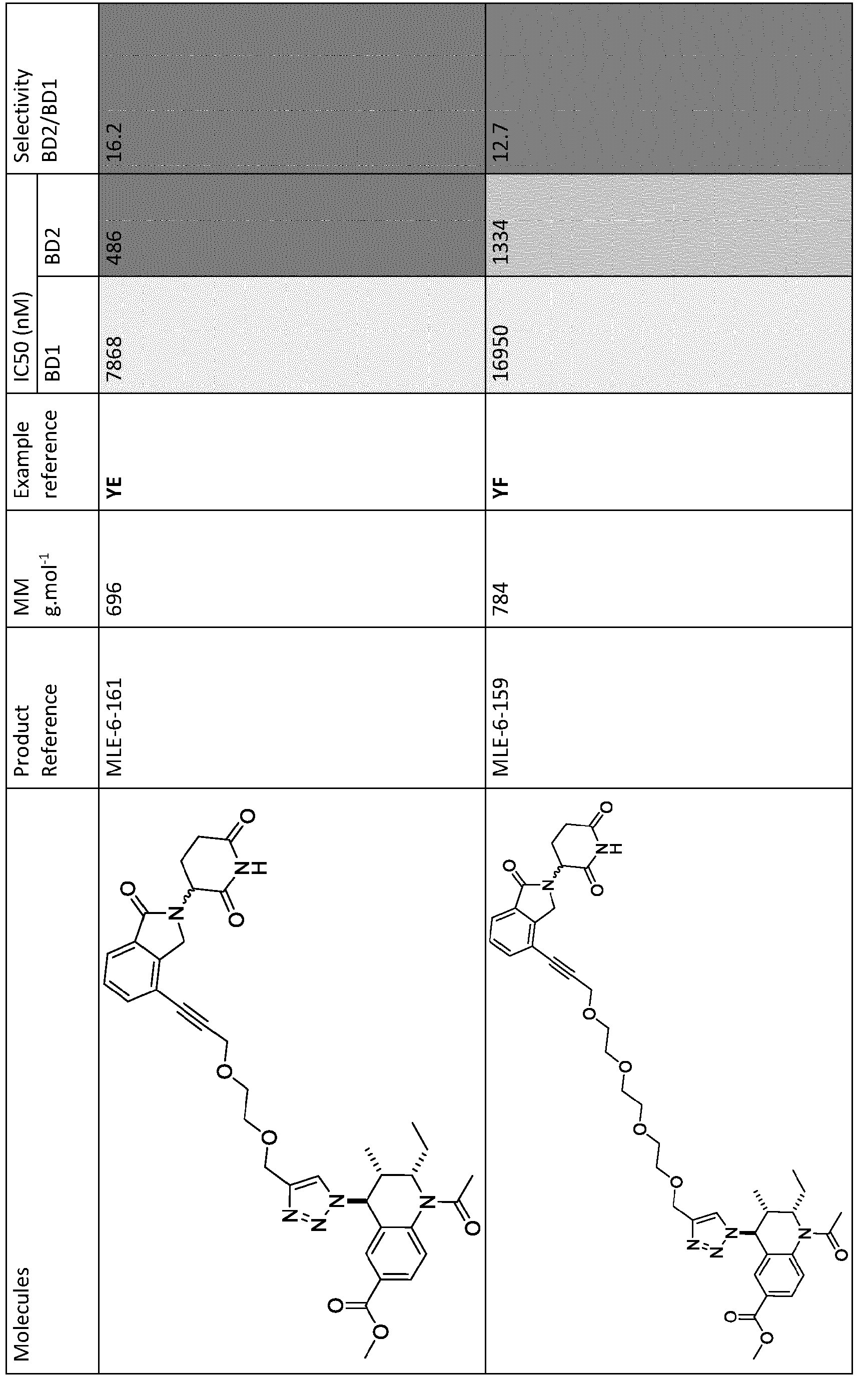

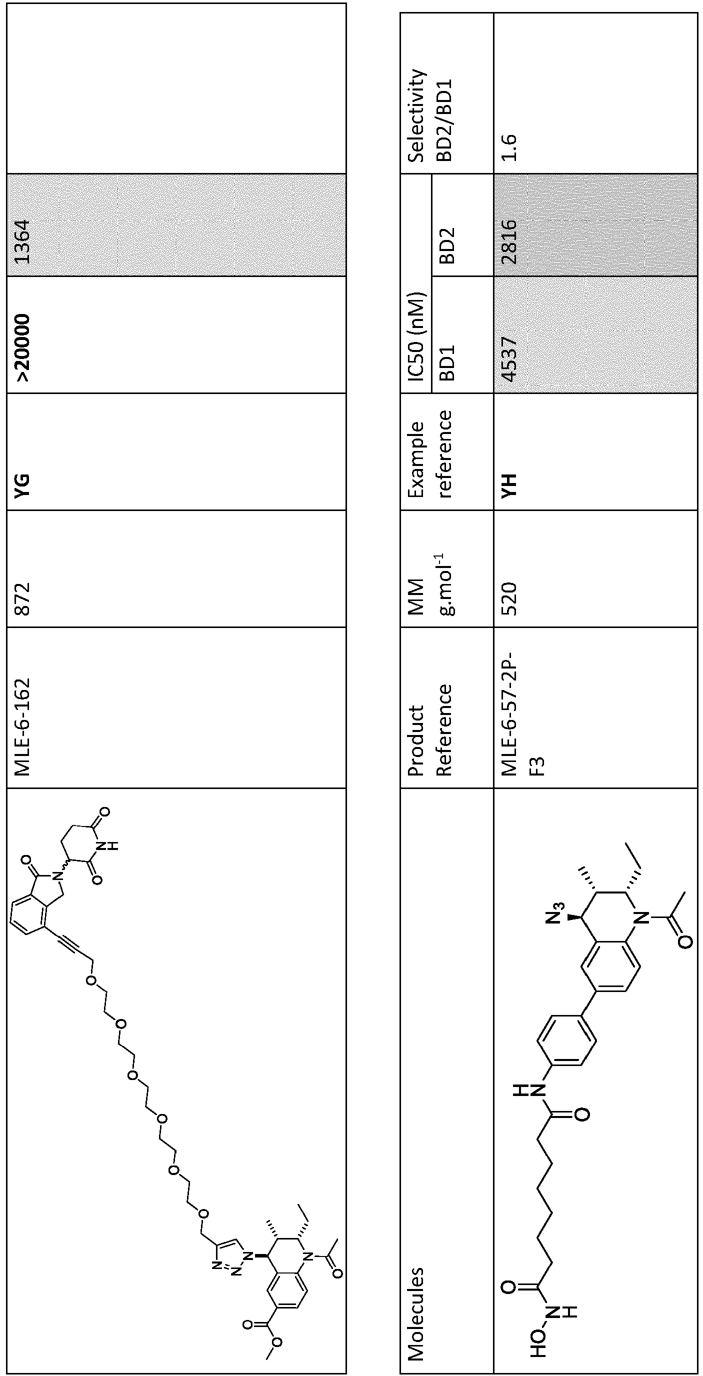

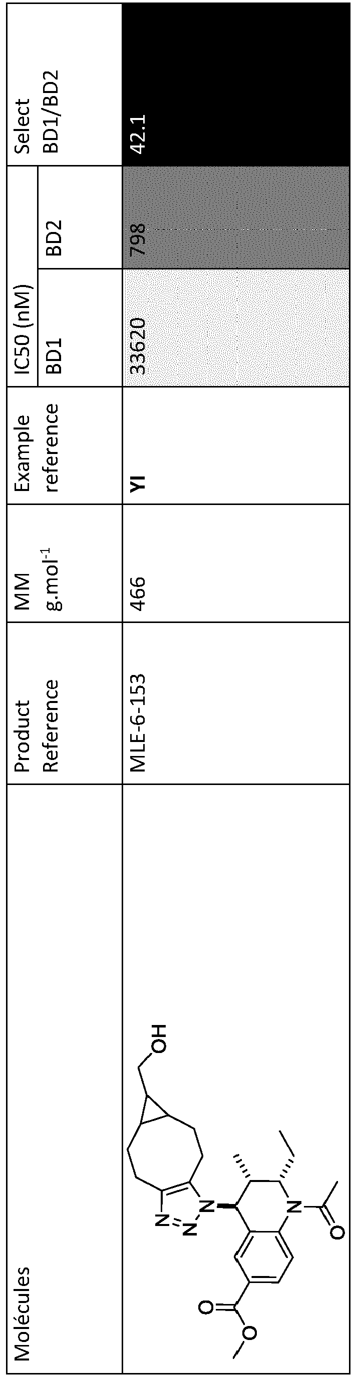

## Claims

1. A compound having the following structure : 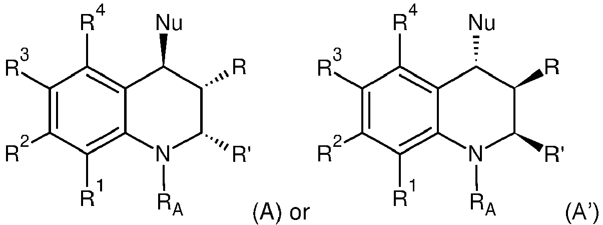 wherein R, R', R_{A}, Nu, R1, R2, R3, and R4 are each independently an atom or a chemical group of atoms.

2. The compound of claim 1, wherein said compound has the following structure : 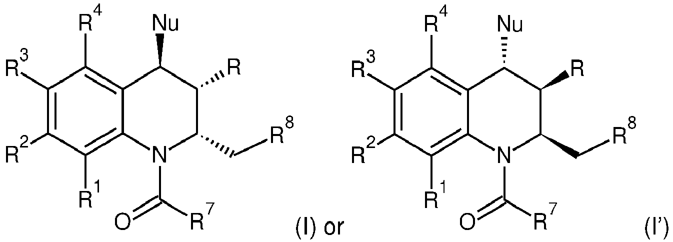 or 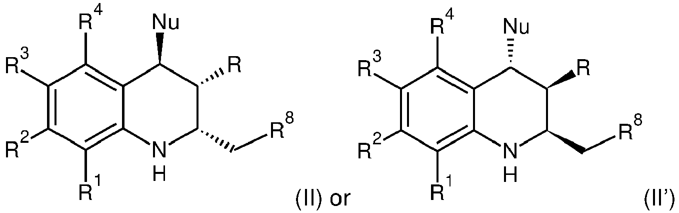
wherein R represents an alkyl group,
wherein R1, R2, R3, R4, R7 and R8 are each independently an atom or a chemical group of atoms,
wherein -Nu represents a group of atoms, preferably selected from the group consisting of -N₃, -OR₅, -SR₅, -SeR₅ and 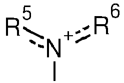 wherein -N is linked to the heterocycle and wherein the covalent bonds between N and R5, and N and R6 represent single or double bonds with R5 or R6, and wherein R5 and R6 represent each independently an atom or a chemical group of atoms and may form together a cycle or heterocycle, optionally substituted, or -Nu together with R form an heterocyle, for example thereby forming a hexahydropyrroloquinoline.

3. The compound of claim 1 or 2, wherein R1, R2 and R4 are hydrogen atoms.

4. The compound of any one of claims 1 to 3, wherein R is CH₃.

5. The compound of any one of claims 2 to 4, wherein R8 is CH₃.

6. The compound of any one of claims 1 to 5, wherein R5 and R6 are each independently selected from the group consisting of H, C(O)OR⁹, an aryl or heteroaryl ring optionally substituted, wherein R9 is an alkyl group.

7. The compound of any one of claims 1 to 6, wherein R5 and R6 form together an heteroaryl of the following structure: 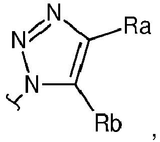 wherein Ra and Rb are each independently an atom or a chemical group of atoms, preferably selected from the group consisting of H, an halogen atom (Br, Cl, F), an alkyl group optionally comprising one or more heteroatoms (O, N, S), an optionally substituted aryl or heteroaryl, for example an optionally substituted phenyl ring, and wherein Ra and Rb may form together a cycle or heterocycle, optionally substituted.

8. The compound of claim 1, wherein said compound has the following structure: 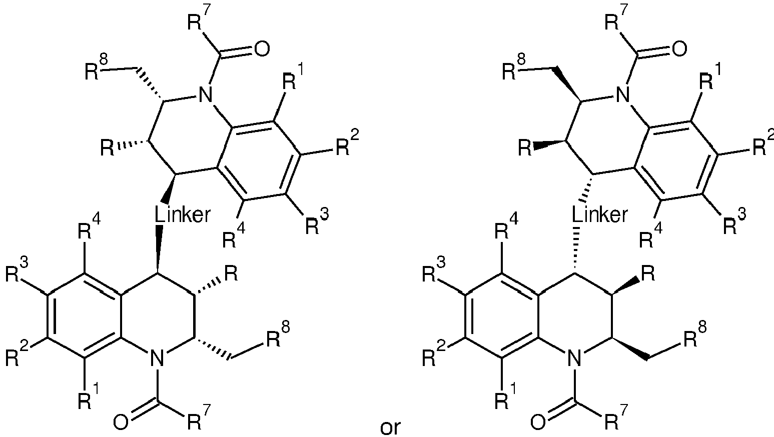 wherein « linker » represents a group of atoms; or 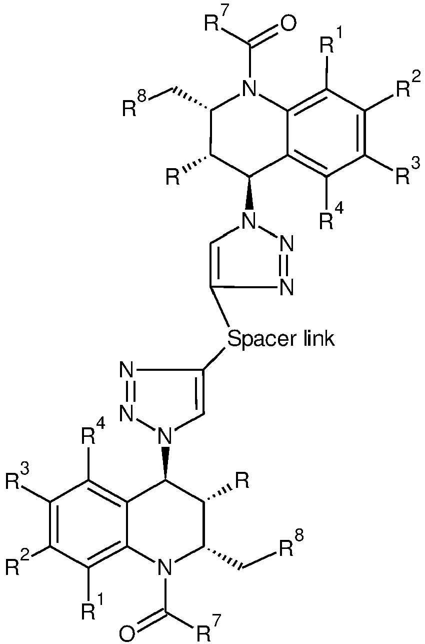 or a corresponding *cis*-2,3 and *trans-3,4* enantiomer thereof: 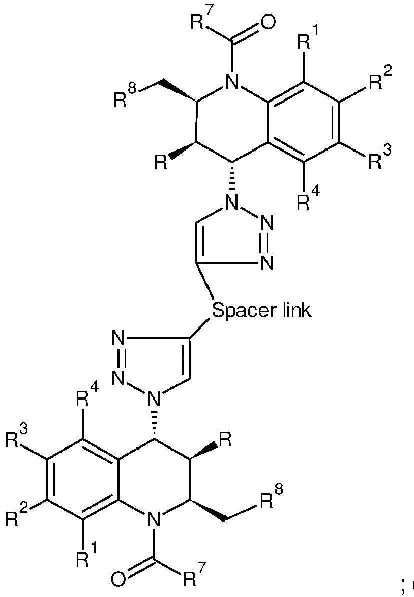 or 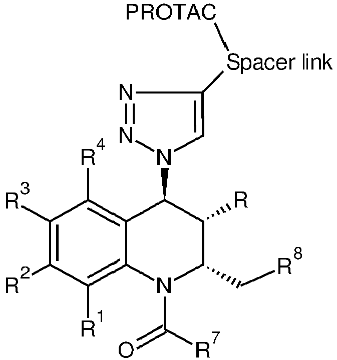 or the corresponding *cis*-2,3 and *trans-3,4* enantiomer thereof: 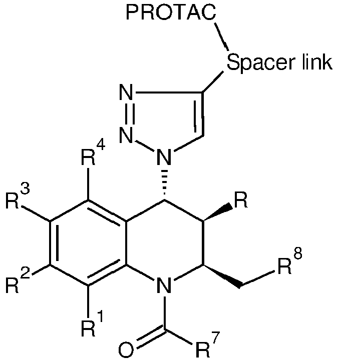 wherein the spacer link is a covalent link comprising several atoms.

9. The compound of claim 1, wherein said compound is selected from the group consisting of the following compounds: 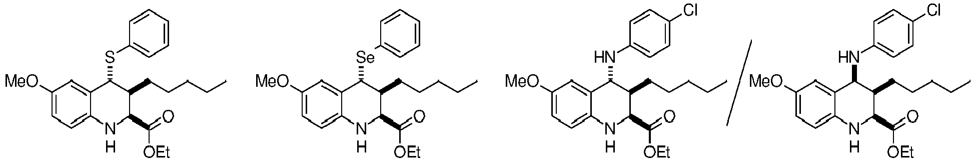 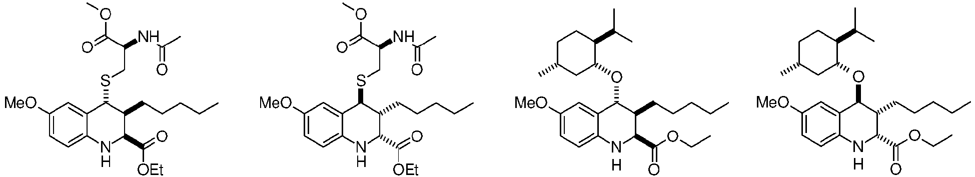 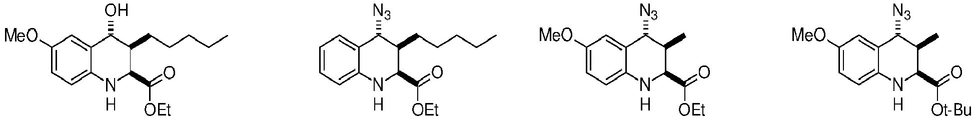 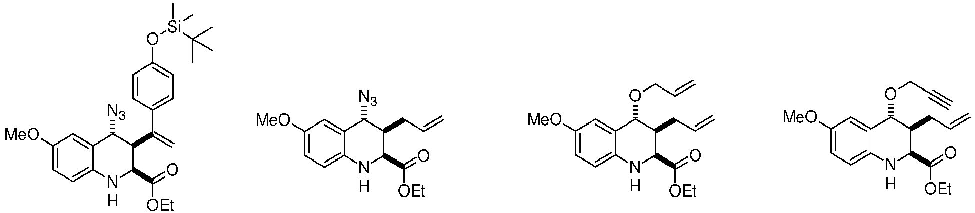 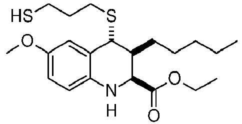 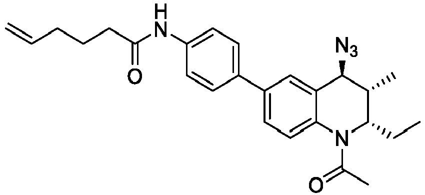 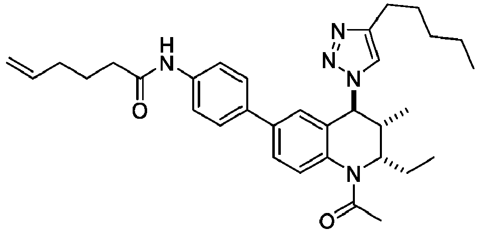 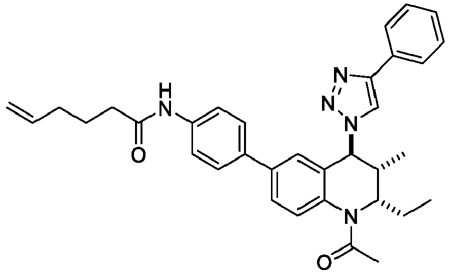 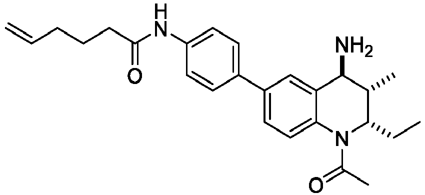 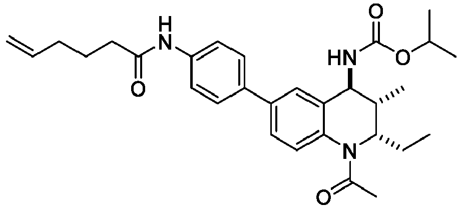 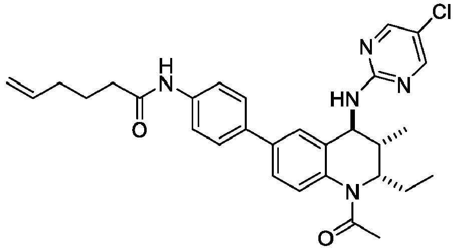 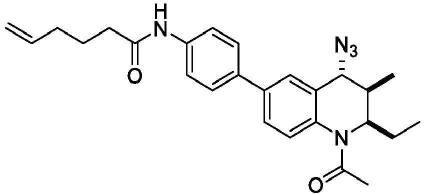 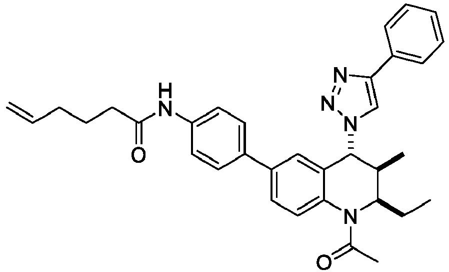 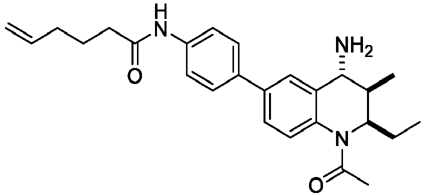 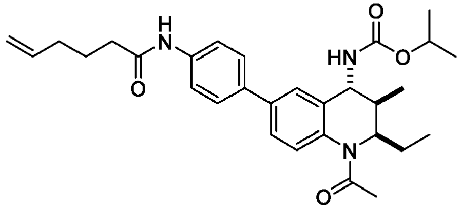 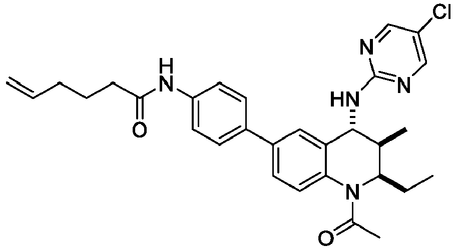 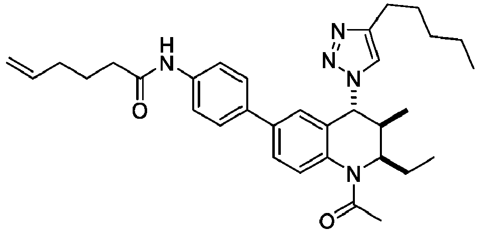 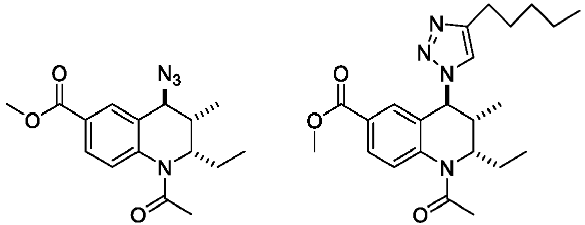 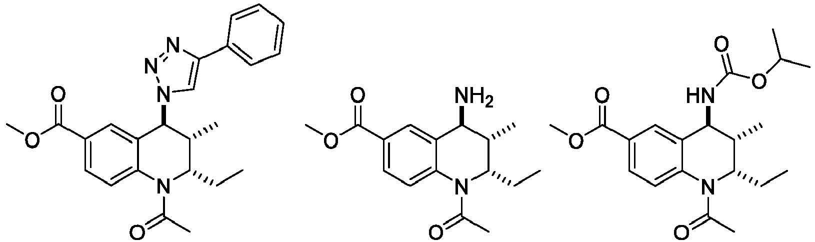 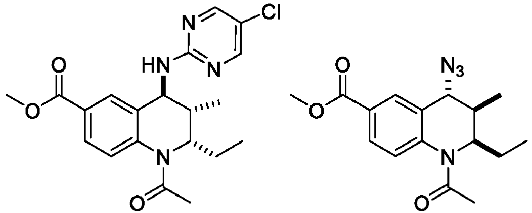 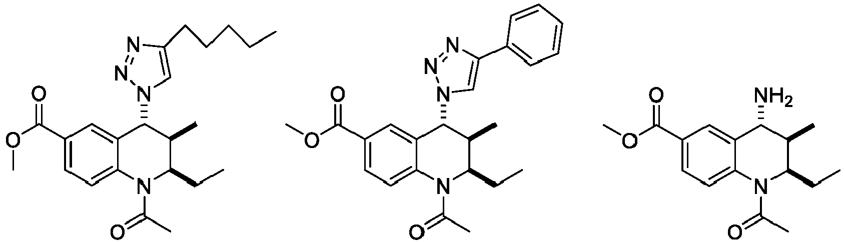 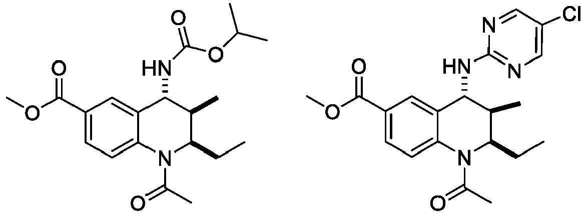 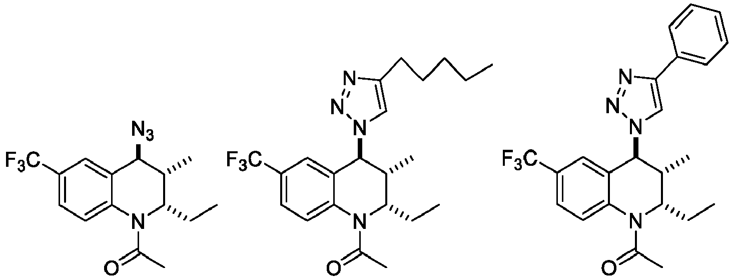 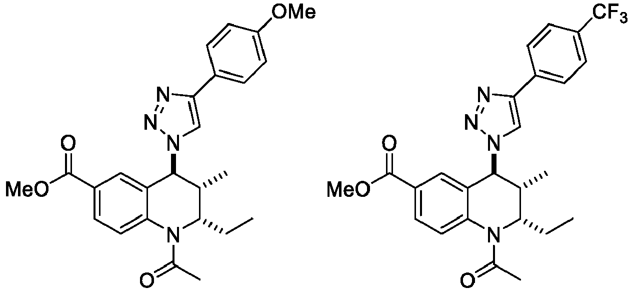 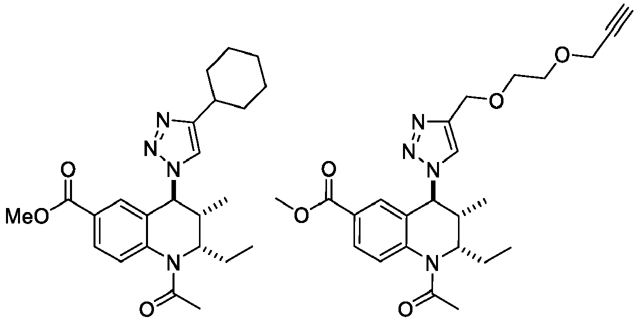 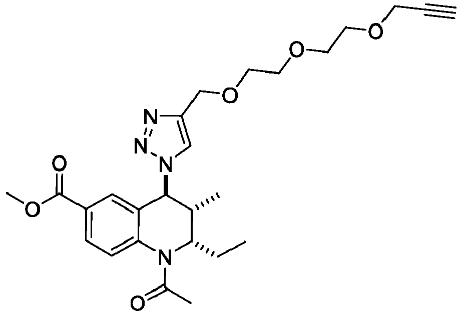 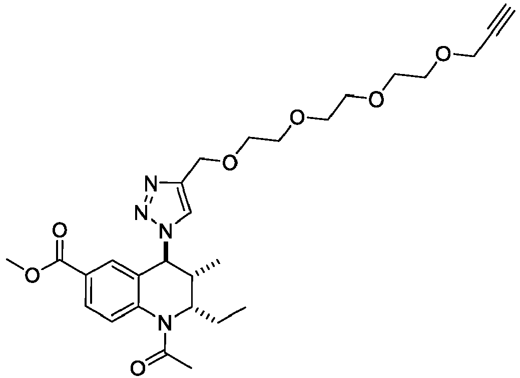 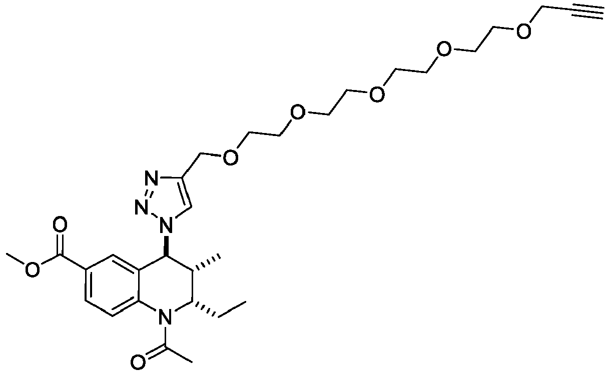 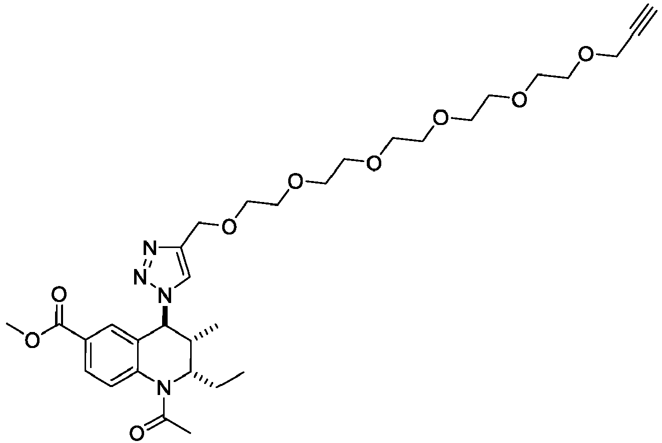 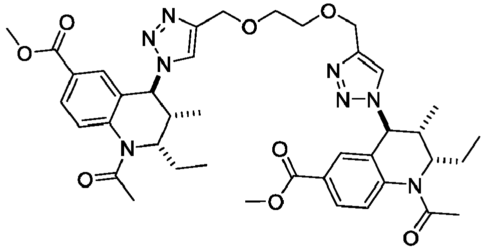 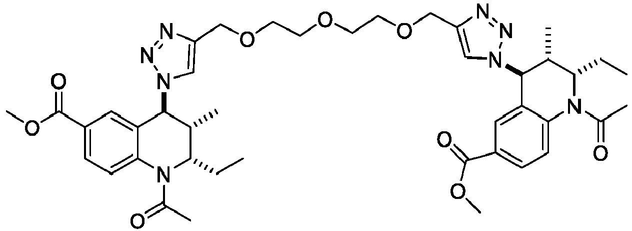 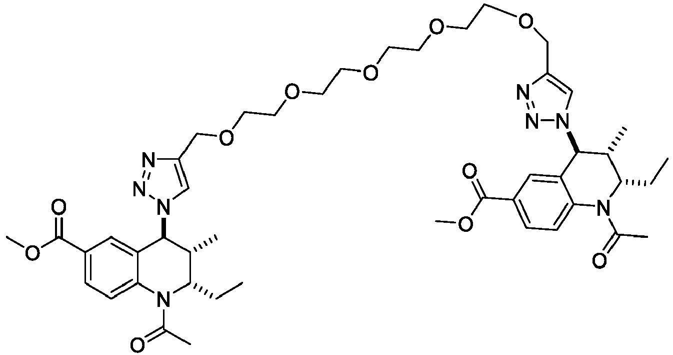 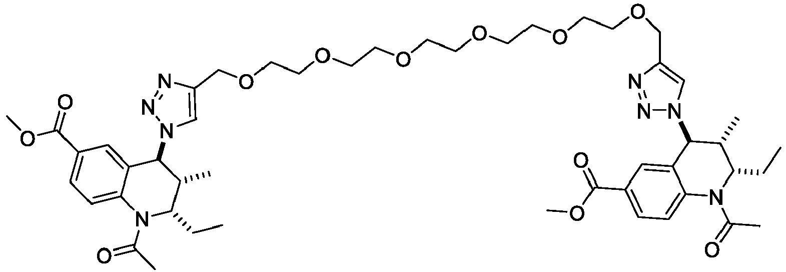 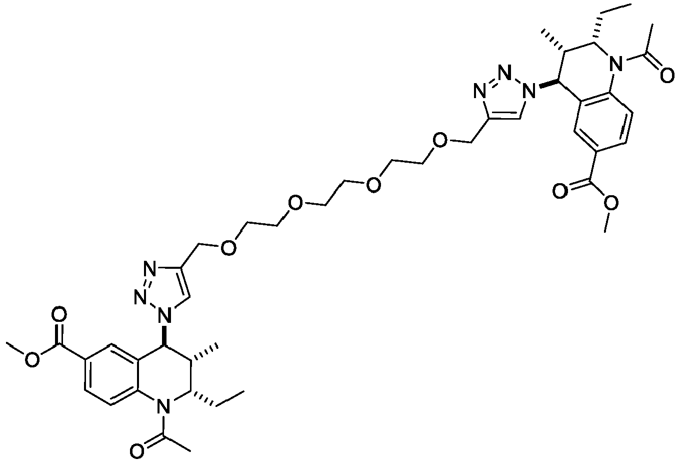 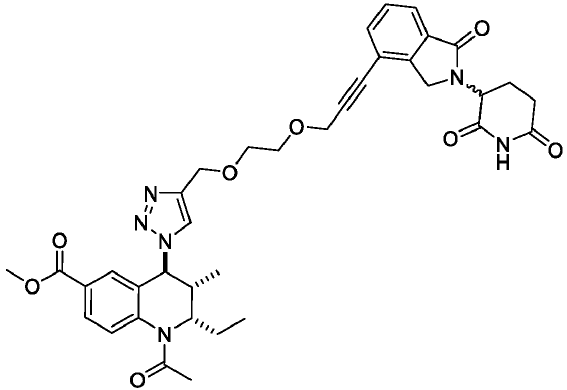 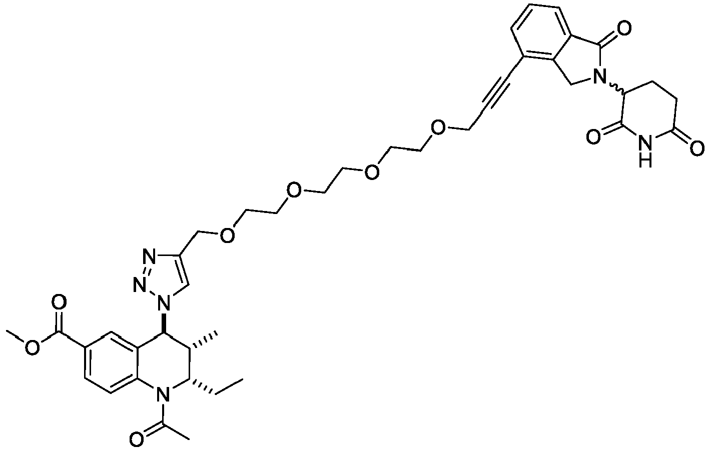 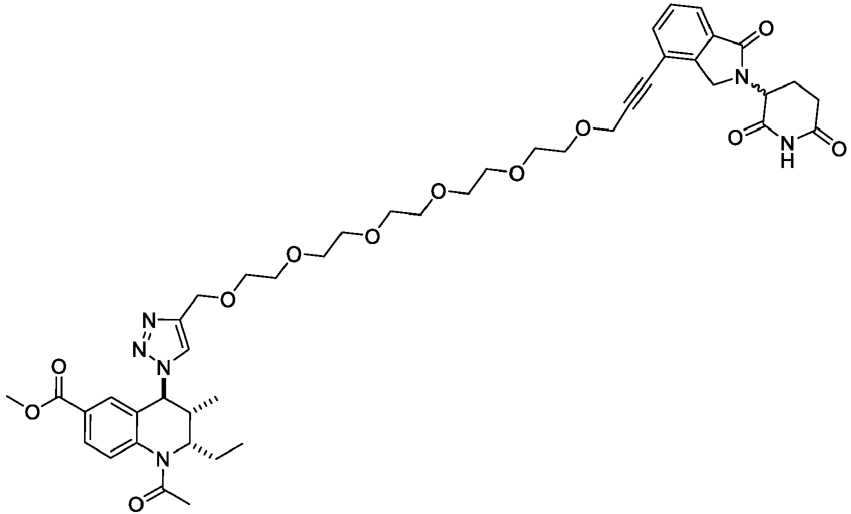 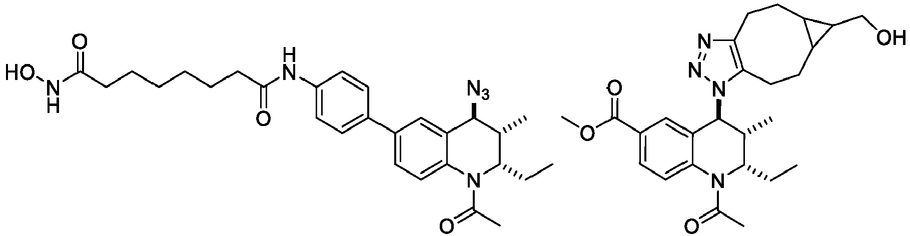 or the corresponding *cis*-2,3 and *trans-*3,4 enantiomer thereof.

10. A method for selective production of tetrahydroquinone, said method comprising reacting reactive compounds in the presence of (i) L-Proline and/or D-Proline and/or (S) silyl prolinol and/or (R) silyl prolinol and (ii) a Lewis acid to provide a tetrahydroquinone compound.

11. The method of claim 8 wherein said method comprises the following reaction:

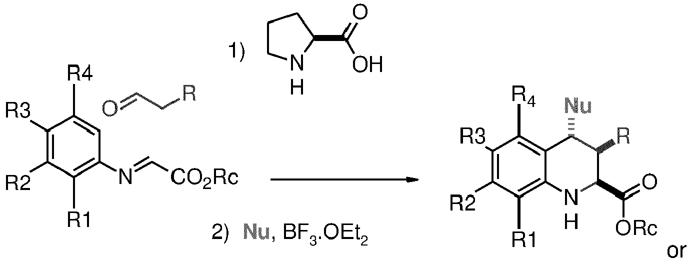

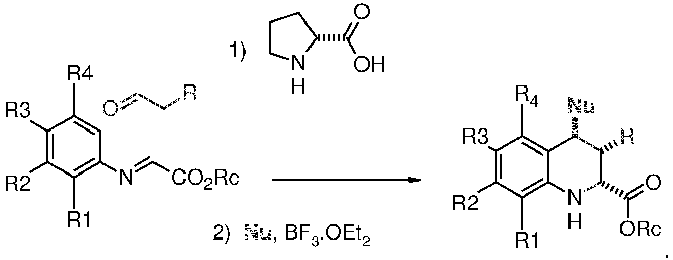

12. The method of claim 8, wherein said method comprises the following reaction: 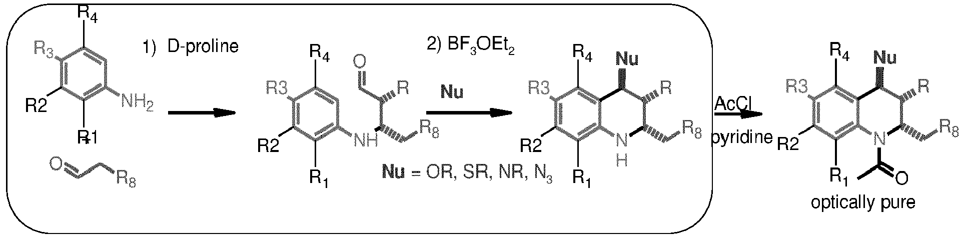 or

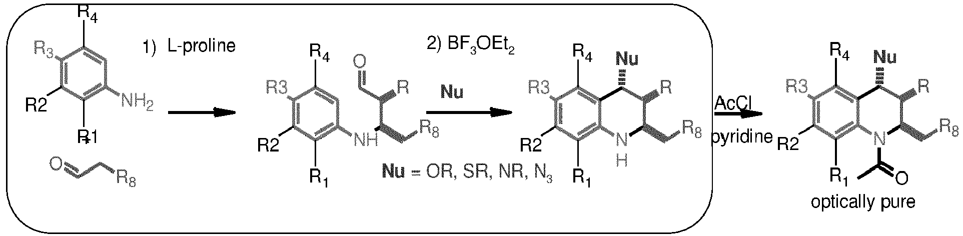

13. The method of any one of claims 8 to 10, wherein said Lewis acid is BF₃OEt₂.

14. A compound as claimed in any one of claims 1 to 7, for use as a selective BET inhibitor.

15. A composition comprising a compound as claimed in any one of claims 1 to 7.

16. A pharmaceutical composition comprising a compound as claimed in any one of claims 1 to 7.

17. A compound as claimed in any one of claims 1 to 7 or composition as claimed in claim 13 or 14, for use in a method for treating an inflammation or a cancer, preferably a cancer involving BET over-expression.
